# EUROPEAN PATENT APPLICATION

(11) **EP 4 843 489 A1**
(43) Date of publication of application: **25.11.2026**
(21) Application number: 26180648.3
(22) Date of filing: 22.05.2026
(51) Int. Cl.: A61B 5/024, A61B 5/026

(54) **METHODS, DEVICES, AND SYSTEMS FOR NON-CONTACT HEART RATE MEASUREMENT**

(30) Priority: 22.05.2025 US 202563810664 P; 23.07.2025 US 202563849439 P; 27.02.2026 US 202663993063 P; 28.04.2026 US 202619661420
(71) Applicant: Meta Platforms Technologies, LLC, Menlo Park, CA 94025 (US)
(72) Inventor: HEYWOOD, Daniel, Menlo Park, 94025 (US); KAPLAN, Kirsten, Menlo Park, 94025 (US); SANKHALA, Devangsingh, Menlo Park, 94025 (US); TAYLOR, Travis, Menlo Park, 94025 (US); TWISS, Peter, Menlo Park, 94025 (US)
(74) Representative: Murgitroyd & Company

(57) **Abstract**

An example method includes projecting an outputted optical signal to a user's body, where the light emitter operates in a pulsed mode with laser pulses separated by off periods. The method includes capturing a return optical signal, sampling it via an analog-to-digital converter to generate ADC samples, and processing a block of samples by filtering to isolate a frequency band of interest and performing energy quantification. The method includes generating an output sample at a rate corresponding to a physiological signal of interest and determining heart information. Such techniques enable non-contact biometric monitoring including heart rate measurement, don/doff detection, liveness detection, respiration rate estimation, and motion artifact compensation.

## Description

### RELATED APPLICATIONS

This application claims priority to U.S. Provisional Application Serial No. 63/810,664, filed May 22, 2025, entitled "Methods, Devices, And Systems For Non-Contact Heart Rate Measurement," U.S. Provisional Application Serial No. 63/849,439, filed July 23, 2025, entitled "Methods, Devices, And Systems For Non-Contact Heart Rate Measurement," and U.S. Provisional Application Serial No. 63/993,063, filed February 27, 2026, entitled "Methods, Devices, And Systems For Non-Contact Heart Rate Measurement".

### TECHNICAL FIELD

This relates generally to using laser Doppler flow techniques for biometric data monitoring, including but not limited to methods, devices, and systems for using pulsed-mode laser Doppler flow techniques.

### BACKGROUND

Various physiological metrics, such as heart rate and blood pressure, are useful for a person to identify and monitor health conditions. Non-invasive techniques such as a photoplethysmogram (PPG) can be used to determine heart information by detecting biometric data such as blood volume changes. However, accurate PPG often requires consistent, direct contact with a well-perfused area of the user's body. Additionally, PPG systems may require a user to remain motionless during PPG measurement. These drawbacks limit the usefulness of such techniques.

### SUMMARY

The present invention provides a method, a wearable device and a non-transitory computer readable storage medium as defined in the appended claims.

In accordance with a first aspect, there is provided a method comprising:
at a wearable device comprising a light emitter, a photodetector, and processing circuitry:
projecting, from the light emitter, an outputted optical signal to a portion of a body of a user, wherein the light emitter operates in a pulsed mode comprising a plurality of laser pulses separated by off periods;
capturing, by the photodetector, a return optical signal corresponding to the outputted optical signal;
sampling, by an analog-to-digital converter (ADC), the return optical signal at an ADC sampling rate to generate a plurality of ADC samples;
processing a block of the plurality of ADC samples to generate a processed sample, wherein processing the block of ADC samples comprises filtering the block of ADC samples to isolate a frequency band of interest and performing energy quantification on the filtered ADC samples;
generating an output sample based on one or more processed samples, wherein the output sample is generated at an output sample rate corresponding to a physiological signal of interest; and
determining, based on the output sample, heart information of the user.

Each laser pulse of the plurality of laser pulses may comprise multiple ADC samples captured during the laser pulse.

Each laser pulse of the plurality of laser pulses may correspond to a single ADC sample.

Each ADC sample may correspond to multiple laser pulses of the plurality of laser pulses.

A duty cycle of the pulsed mode may be controlled to adjust an average optical power of the outputted optical signal.

The duty cycle may be variable within a range from 1.25% to 80%.

The duty cycle may have a minimum value of 10%.

A pulse duration of each laser pulse may be on an order of 1 millisecond.

The ADC sampling rate may be 20 kHz or higher.

The output sample rate may be between 10 Hz and 100 Hz for capturing heartbeat information.

Performing energy quantification may comprise computing an average rectified value (ARV) of the filtered ADC samples.

In accordance with a second aspect, there is provided a wearable device comprising:
a light emitter configured to project an outputted optical signal to a portion of a body of a user, wherein the light emitter is configured to operate in a pulsed mode comprising a plurality of laser pulses separated by off periods;
a photodetector configured to capture a return optical signal corresponding to the outputted optical signal;
an analog-to-digital converter (ADC) configured to sample the return optical signal to generate ADC samples; and
processing circuitry configured to:
   process a block of the ADC samples to generate a processed sample, wherein processing the block of ADC samples comprises filtering the block of ADC samples to isolate a frequency band of interest and performing energy quantification on the filtered ADC samples;
   generate an output sample based on one or more processed samples, wherein the output sample is generated at an output sample rate corresponding to a physiological signal of interest; and
   determine, based on the output sample, heart information of the user.

Each laser pulse of the plurality of laser pulses may comprise multiple ADC samples captured during the laser pulse.

Each laser pulse of the plurality of laser pulses may correspond to a single ADC sample.

Each ADC sample may correspond to multiple laser pulses of the plurality of laser pulses.

A duty cycle of the pulsed mode may be controlled to adjust an average optical power of the outputted optical signal.

In accordance with a third aspect, there is provided a non-transitory computer-readable storage medium storing instructions that, when executed by one or more processors of a wearable device comprising a light emitter and a photodetector, cause the wearable device to perform operations comprising:
projecting, from the light emitter, an outputted optical signal to a portion of a body of a user, wherein the light emitter operates in a pulsed mode comprising a plurality of laser pulses separated by off periods;
capturing, by the photodetector, a return optical signal corresponding to the outputted optical signal;
sampling the return optical signal at an analog-to-digital (ADC) sampling rate to generate a plurality of ADC samples;
processing a block of the plurality of ADC samples to generate a processed sample, wherein processing the block of ADC samples comprises filtering the block of ADC samples to isolate a frequency band of interest and performing energy quantification on the filtered ADC samples;
generating an output sample based on one or more processed samples, wherein the output sample is generated at an output sample rate corresponding to a physiological signal of interest; and
determining, based on the output sample, heart information of the user.

Each laser pulse of the plurality of laser pulses may comprise multiple ADC samples captured during the laser pulse.

Each laser pulse of the plurality of laser pulses may correspond to a single ADC sample.

Each ADC sample may correspond to multiple laser pulses of the plurality of laser pulses.

The present disclosure describes, amongst other things, methods, devices, and systems for laser Doppler flowmetry (LDF) in wearable devices. LDF is an optical sensing technique that measures blood flow by detecting Doppler shifts in light scattered from moving blood cells. Unlike PPG, which measures vessel distension due to pressure changes, LDF measures actual blood flow velocity, providing complementary physiological information. The present disclosure describes several aspects of LDF implementation and signal processing.

In accordance with some aspects, the present disclosure describes pulsed-mode LDF operation in which a light emitter operates in a pulsed-mode comprising a plurality of laser pulses separated by off periods. A hierarchical sampling structure may be employed, including ADC samples captured at a high rate, processed samples generated by filtering and energy quantification, and output samples generated at a rate corresponding to a physiological signal of interest such as heartbeat. Pulsed-mode operation provides several benefits, including relaxed laser tolerance requirements (enabling use of commodity-grade VCSELs with wide manufacturing tolerances), a broader range of average optical power control through duty cycle variation, optical safety benefits from operating farther from the lasing threshold, and improved coexistence with other optical systems through time multiplexing. Additionally, pulsed-mode operation enables ambient light cancellation by sampling during off periods to measure ambient light levels and adjusting a digital-to-analog converter to null the ambient signal.

In accordance with some aspects, the present disclosure describes don/doff and liveness detection using LDF. A flow spectrum technique may quantify power of a return optical signal within a plurality of frequency bands to detect a spectral distribution characteristic of perfused skin, such as a 1/f frequency dependence. Blood flow results in a broad power spectrum in the LDF signal, in contrast to reflections from other materials which have power concentrated in a narrower band near DC. This enables the wearable device to determine a wear state indicating whether the device is donned or doffed, with improved accuracy compared to simple proximity sensing. Additionally, heartbeat detection may be used for liveness detection and security applications, where the presence of a heartbeat signal provides evidence that the device is targeting skin of a live person. A liveness indication may be used to extend periods between authentication events by verifying that an authenticated user has continuous custody of the device. A hybrid approach may combine both flow spectrum and heartbeat techniques using a time-frequency transform to capture both spectral power distribution arising from blood flow and temporal pulsatility arising from heartbeat.

In accordance with some aspects, the present disclosure describes respiration rate estimation using LDF. A time series flow signal may be collected and filtered to isolate a respiration frequency band, and a fundamental frequency within this band may be identified corresponding to the respiration rate. LDF may exhibit a more modest Mayer wave component compared to PPG signals, enabling improved resolution of the respiration rate. Respiratory modulation may appear in the LDF signal as a modulation of baseline flow, a modulation of flow pulsatility amplitude, or a modulation of interbeat interval. Sensor fusion may combine the LDF signal with signals from additional sensors such as an inertial measurement unit (IMU) measuring motion or a microphone capturing breath sounds, with complementarity between signals under different conditions. This enables respiration sensing on form factors not well suited to PPG, such as smartglasses.

In accordance with some aspects, the present disclosure describes self-compensation of motion artifacts in LDF signals. A low frequency component of the return optical signal encodes motion information corresponding to motion of the light emitter relative to the body, while higher frequency components encode blood flow information. Multiple independent time series may be extracted from the same optical signal by probing different portions of the frequency spectrum. The low frequency component may be used to compensate for motion artifacts in the higher frequency components using an adaptive filter. This self-compensation approach may achieve better accuracy than compensation using an external accelerometer because the signals share the same optical components. Additionally, this approach simplifies system architecture by eliminating the need for a separate accelerometer and enables embedding the heart rate algorithm on the LDF module itself.

The methods, devices, and systems described herein enable a wearable device (e.g., a headset, a wrist-wearable device etc.) to perform non-contact measurements to determine/monitor biometric data, e.g., heart information including heart rate. The methods described herein include methods of using LDF in an analog domain, e.g., using a measurement system that includes light emitter(s) (e.g., laser(s)), photodetector(s) (e.g., photodiode(s)), and an analog integrated circuit (IC). Such an LDF-based measurement system in the analog domain provides several advantages, including (i) non-contact operation (e.g., no direct skin contact required) with inherent resistance to motion artifacts, (ii) robustness to variations in head shape and slippage during motion, (iii) reduced design complexity (e.g., minimized customization of silicon chip(s)), (iv) a high-compact form factor with a smaller module footprint), and (v) lower power consumption, which can be particularly important for battery-powered devices.

Additionally, the methods, devices, and systems described herein enable a wearable device (e.g., a headset, a wrist-wearable device etc.) to correct positional disparity during an LDF-based measurement induced by motion artifacts. For example, motion artifacts may significantly degrade the accuracy of heart rate estimates, particularly during movement by a user (e.g., when the user is performing a physical activity). For example, the user's motion may introduce signals that are distinct from the true heartbeat signal, making it difficult to capture accurate heart rate estimates. This may lead to a poor user experience, either due to inaccurate readings or estimates with low confidence. In LDF-based measurements, a mechanism by which motion artifacts arise is self-mixing interferometry (SMI), e.g., also referred to as the self-mixing effect. This occurs when a portion of emitted light (e.g., from an optical cavity of a light source) is back-scattered and re-couples into the optical cavity and interferences with the emitted light. Such interference may introduce fluctuations in the output power of the emitted light, which is inadvertently detected and misinterpreted as biometric data such as blood flow. By reducing motion artifacts, particularly those arising from the SMI, the accuracy of the LDF-based measurement can be improved, thereby enhancing user experience and perception.

As an example, a user wearing an extended-reality (XR) headset may wish to monitor their heart rate while engaging in an activity such as playing a video game or performing an exercise or fitness routine. In this example, the XR headset includes one or more inward-facing light emitters and corresponding photodetector(s). As described herein, the inward-facing light emitters and corresponding photodetector(s) of the virtual-reality headset can be used to monitor blood volume and/or flow rate changes in the user's facial tissue while the user engages in the activities. The blood volume and/or flow rate changes can be used to determine a heart rate of the user. In this way the user can monitor their heart rate without having to remain stationary and consecutive measurements of a region of the user's face can be obtained and compared.

An example wearable device includes a light emitter, a photodetector, and an analog IC comprising a high-pass filter, a rectifier, and a low-pass filter. In this example, the light emitter is configured to project an outputted optical signal to a portion of a body of a user, and the photodetector is configured to capture a return optical signal corresponding to the outputted optical signal. The analog IC in this example is configured to process the return optical signal in analog domain, including generating a filtered optical signal by applying the high-pass filter to remove non-heart information from the return optical signal, generating a rectified optical signal by applying the rectifier to rectify the filtered optical signal, and generating a time-averaged optical signal by applying the low-pass filter to the rectified optical signal. The wearable device is configured to determine (e.g., via a processor or other control circuitry) heart information of the user based on the time-averaged optical signal, and cause the heart information to be presented to the user (e.g., via a display of the device).

Another example wearable device includes a light emitter and a photodetector. The light emitter is configured to project an outputted optical signal to a portion of a body of a user. The photodetector is configured to capture a modulated optical signal corresponding to the outputted optical signal. The wearable device is configured to determine, based on the modulated optical signal, a positional disparity between the light emitter and the portion of the body. In accordance with a determination that the positional disparity satisfies disparity correction criteria, the wearable device is configured to adjust a driving current of the light emitter to compensate variations in the outputted optical signal caused by the positional disparity.

In accordance with some embodiments, a method of presenting heart information comprises: (i) projecting, from a light emitter, an outputted optical signal to a portion of a body of a user; (ii) capturing, by a photodetector, a return optical signal corresponding to the outputted optical signal; (iii) processing the return optical signal in analog domain, including: (a) generating a filtered optical signal by applying a high-pass filter to remove non-heart information from the return optical signal; (b) generating a rectified optical signal by applying a rectifier to rectify the filtered optical signal; and (c) generating a time-averaged optical signal by applying a low-pass filter to the rectified optical signal; (iv) determining, based on the time-averaged optical signal, heart information of the user; and (v) causing the heart information to be presented to the user

In accordance with some embodiments, a method of correcting positional disparity at a wearable device comprises: (i) projecting, from a light emitter, an outputted optical signal to a portion of a body of a user; (ii) capturing, by a photodetector, a modulated optical signal corresponding to the outputted optical signal; (iii) determining, based on the modulated optical signal, a positional disparity between the light emitter and the portion of the body; and (v) in accordance with a determination that the positional disparity satisfies disparity correction criteria, adjusting a driving current of the light emitter to compensate variations in the outputted optical signal caused by the positional disparity.

Instructions that cause performance of the methods and operations described herein can be stored on a non-transitory computer readable storage medium. The non-transitory computer-readable storage medium can be included on a single electronic device or spread across multiple electronic devices of a system (computing system). A non-exhaustive of list of electronic devices that can either alone or in combination (e.g., a system) perform the method and operations described herein include XR headset/glasses (e.g., a mixed-reality (MR) headset or a pair of augmented-reality (AR) glasses as two examples), a wrist-wearable device, an intermediary processing device, a smart textile-based garment, etc. For instance, the instructions can be stored on a pair of AR glasses or can be stored on a combination of a pair of AR glasses and an associated input device (e.g., a wrist-wearable device) such that instructions for causing detection of input operations can be performed at the input device and instructions for causing changes to a displayed user interface in response to those input operations can be performed at the pair of AR glasses. The devices and systems described herein can be configured to be used in conjunction with methods and operations for providing an XR experience. The methods and operations for providing an XR experience can be stored on a non-transitory computer-readable storage medium.

The devices and/or systems described herein can be configured to include instructions that cause the performance of methods and operations associated with the presentation and/or interaction with an XR headset. These methods and operations can be stored on a non-transitory computer-readable storage medium of a device or a system. It is also noted that the devices and systems described herein can be part of a larger, overarching system that includes multiple devices. A non-exhaustive of list of electronic devices that can, either alone or in combination (e.g., a system), include instructions that cause the performance of methods and operations associated with the presentation and/or interaction with an XR experience include an extended-reality headset (e.g., an MR headset or a pair of AR glasses as two examples), a wrist-wearable device, an intermediary processing device, a smart textile-based garment, etc. For example, when an XR headset is described, it is understood that the XR headset can be in communication with one or more other devices (e.g., a wrist-wearable device, a server, intermediary processing device) which together can include instructions for performing methods and operations associated with the presentation and/or interaction with an extended-reality system (i.e., the XR headset would be part of a system that includes one or more additional devices). Multiple combinations with different related devices are envisioned, but not recited for brevity.

The features and advantages described in the specification are not necessarily all inclusive and, in particular, certain additional features and advantages will be apparent to one of ordinary skill in the art in view of the drawings, specification, and claims. Moreover, it should be noted that the language used in the specification has been principally selected for readability and instructional purposes.

It will be appreciated that any features described herein as being suitable for incorporation into one or more aspects are intended to be generalizable across any and all aspects described herein. Other aspects can be understood by those skilled in the art in light of the description, the claims and the drawings. The foregoing general description and the following detailed description are exemplary and explanatory only and are not restrictive of the claims.

Having summarized the above example aspects, a brief description of the drawings will now be presented.

### BRIEF DESCRIPTION OF THE DRAWINGS

For a better understanding of the various described embodiments, reference should be made to the Detailed Description below, in conjunction with the following drawings in which like reference numerals refer to corresponding parts throughout the figures.
Figure 1A illustrates an example non-contact measurement system of monitoring biometric data using a wearable device, in accordance with some embodiments.
Figure 1B illustrates example configurations for biometric data monitoring implemented in a wearable device, in accordance with some embodiments.
Figure 2 illustrates a block diagram of a sensing unit of a wearable device, in accordance with some embodiments.
Figures 3A and 3B illustrate analog circuitry corresponding to an analog integrated circuit, in accordance with some embodiments.
Figures 4A and 4B illustrate aspects of analog circuitry corresponding to an integrated circuit, in accordance with some embodiments.
Figure 4C illustrates an example noise analysis corresponding to an integrated circuit, in accordance with some embodiments.
Figure 5A illustrates an example processing flow for using LDF for don/doff condition detection in accordance with some embodiments.
Figure 5B illustrates example receiver operating characteristic (ROC) curves in accordance with some embodiments.
Figures 5C and 5D illustrate example ROC curves based on varying characteristics in accordance with some embodiments.
Figure 5E illustrates example detector curves in accordance with some embodiments.
Figure 5F illustrates example reflection strength curves in accordance with some embodiments.
Figure 5G illustrates example skin reflectance spectrums in accordance with some embodiments.
Figure 5H illustrates example flow spectrums in accordance with some embodiments.
Figure 5I illustrates example heartbeat measurements in accordance with some embodiments.
Figure 6A illustrates a circuit diagram of an example driving circuit for a light emitter, in accordance with some embodiments.
Figure 6B illustrates example timing diagrams for pulsed-mode laser Doppler flow in accordance with some embodiments.
Figure 6C illustrates an example sample processing scheme in accordance with some embodiments.
Figure 6D illustrates example laser functionalities in accordance with some embodiments.
Figure 6E illustrates example time-series outputs in accordance with some embodiments.
Figure 6F illustrates an example wrist-based PPG signal in accordance with some embodiments.
Figure 6G illustrates an example forehead-based LDF signal in accordance with some embodiments.
Figure 6H illustrates example motion artifacts in a PPG signal in accordance with some embodiments.
Figure 6I illustrates example motion artifacts in an LDF signal in accordance with some embodiments.
Figure 6J illustrates another example motion artifact in an LDF signal in accordance with some embodiments.
Figure 6K illustrates an example method of extracting a pulse signal from an LDF signal in accordance with some embodiments.
Figure 6L illustrates an example compensation approach for LDF signals in accordance with some embodiments.
Figure 6M illustrates example compensation in LDF signals in accordance with some embodiments.
Figure 7 illustrates a flow diagram of a method of operating a wearable device for biometric data monitoring, in accordance with some embodiments.
Figures 8A, 8B, 8C-1, and 8C-2 illustrate example extended reality systems that include augmented-reality system and mixed-reality systems, in accordance with some embodiments.
Figures 9A and 9B illustrate example configurations for a light emitter of a wearable device, in accordance with some embodiments.
Figure 10 illustrates another example configuration for a light emitter of a wearable device, in accordance with some embodiments.
Figure 11 illustrates a flow diagram of a method of correcting positional disparity at a wearable device, in accordance with some embodiments.

In accordance with common practice, the various features illustrated in the drawings may not be drawn to scale. Accordingly, the dimensions of the various features may be arbitrarily expanded or reduced for clarity. In addition, some of the drawings may not depict all of the components of a given system, method, or device. Finally, like reference numerals may be used to denote like features throughout the specification and figures.

### DETAILED DESCRIPTION

The present disclosure describes, amongst other things, using LDF for a variety of applications, including heart rate monitoring, don/doff detection, liveness detection for security applications, and respiration rate estimation. As an illustrative example, a user wearing an XR headset may wish to monitor their heart rate while engaging in an activity such as playing a video game or performing an exercise routine. In this scenario, pulsed-mode LDF operation enables the XR headset to capture heart rate information while coexisting with other optical systems such as eye tracking. The pulsed-mode approach provides benefits including relaxed laser tolerance requirements, enabling use of commodity-grade VCSELs, and optical safety benefits from operating farther from the lasing threshold. Additionally, by varying the duty cycle of the laser pulses, the system can control average optical power over a broad range while maintaining consistent instantaneous power.

In another example, a user may place their smartglasses on top of a hat or hold them by the temple arm, which can cause false don events with conventional proximity sensing. The LDF-based don/doff detection described herein addresses this challenge by using a flow spectrum technique that quantifies power across multiple frequency bands to detect a spectral distribution characteristic of perfused skin. Blood flow results in a broad power spectrum in the LDF signal, in contrast to reflections from foam, fabric, or other materials which have power concentrated in a narrower band near DC. This enables the wearable device to distinguish between skin and other materials, reducing false don events and improving user experience.

In yet another example, a user running or performing other high-impact activities may experience motion artifacts that degrade heart rate measurement accuracy. The self-compensation techniques described herein address this by extracting a low frequency component from the LDF signal that encodes motion information, and using this component to compensate for artifacts in the higher frequency components that encode blood flow. Because both signals are derived from the same optical elements, this approach may achieve better accuracy than compensation using an external accelerometer, while also simplifying system architecture by eliminating the need for a separate motion sensor.

In a further example, a user may wish to monitor their respiration rate during daily activities. The LDF signal may exhibit a more modest Mayer wave component compared to PPG signals, enabling improved resolution of the respiration rate. Respiratory modulation may appear in the LDF signal as a modulation of baseline flow or flow pulsatility amplitude. Sensor fusion may combine the LDF signal with signals from additional sensors such as an inertial measurement unit measuring head motion or a microphone capturing breath sounds, with complementarity between signals under different conditions. This enables respiration sensing on form factors such as smartglasses that are not well suited to PPG-based approaches.

As an illustrative example, a wearable device (e.g., a head-wearable device, a wrist-wearable device, or other type of wearable device) may be configured to determine heart information for a user by capturing return optical signals from a light emitter. The wearable device may use analog circuitry to filter and time-average the return optical signals. The heart information may be determined from the filtered and time-averaged return optical signals. At least some of the heart information may be presented to the user (e.g., via a display or microphone). Calculating the heart information in this manner does not require skin contact and may be performed while the user is moving. Additionally, using analog circuitry to process the return optical signals can reduce the power consumption, area, and cost of the wearable device as compared to wearable devices that use digital circuitry.

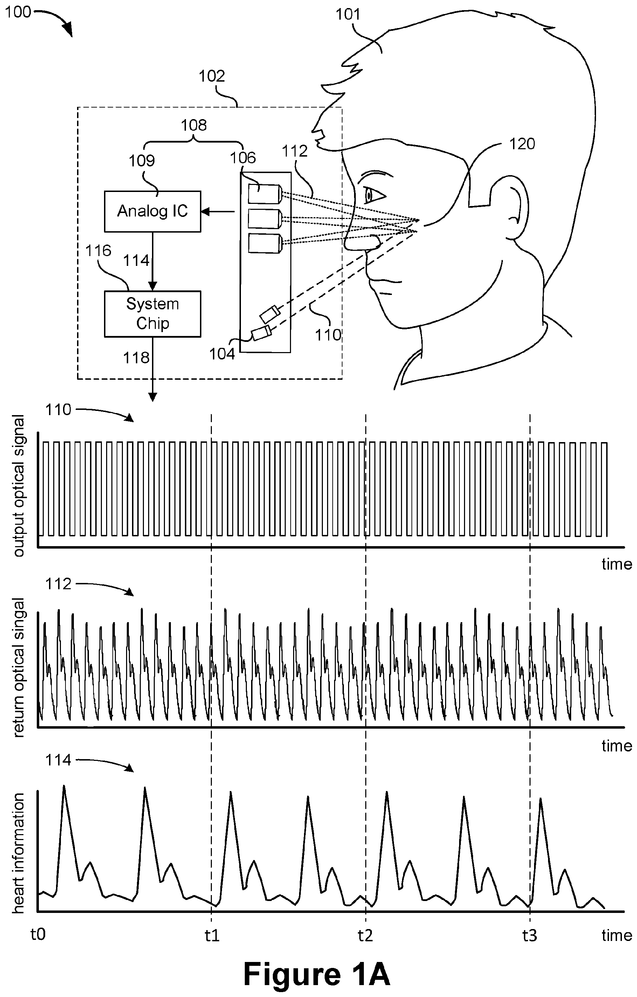

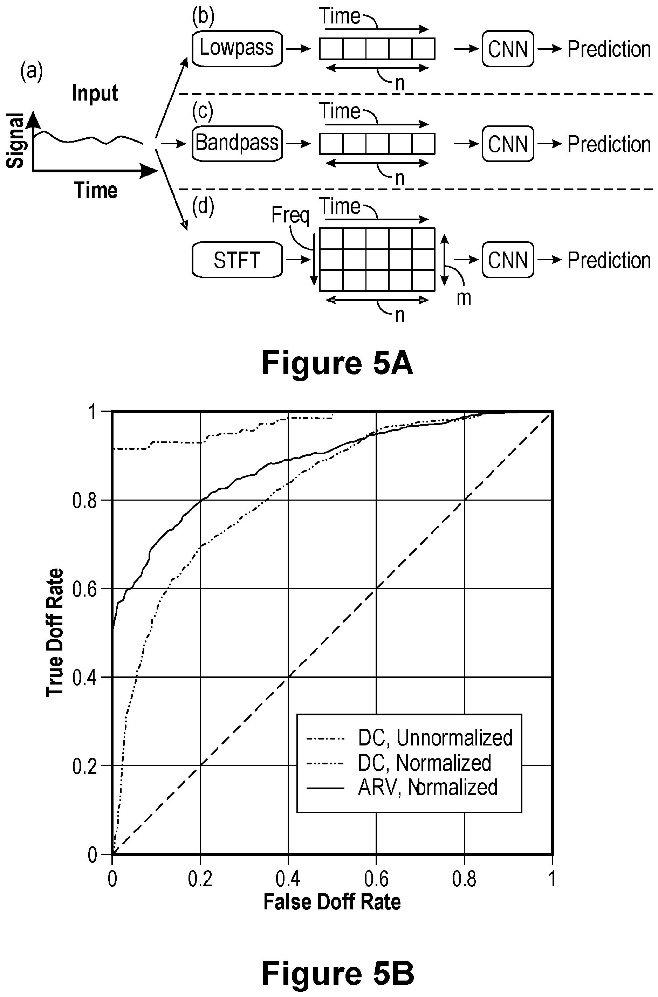

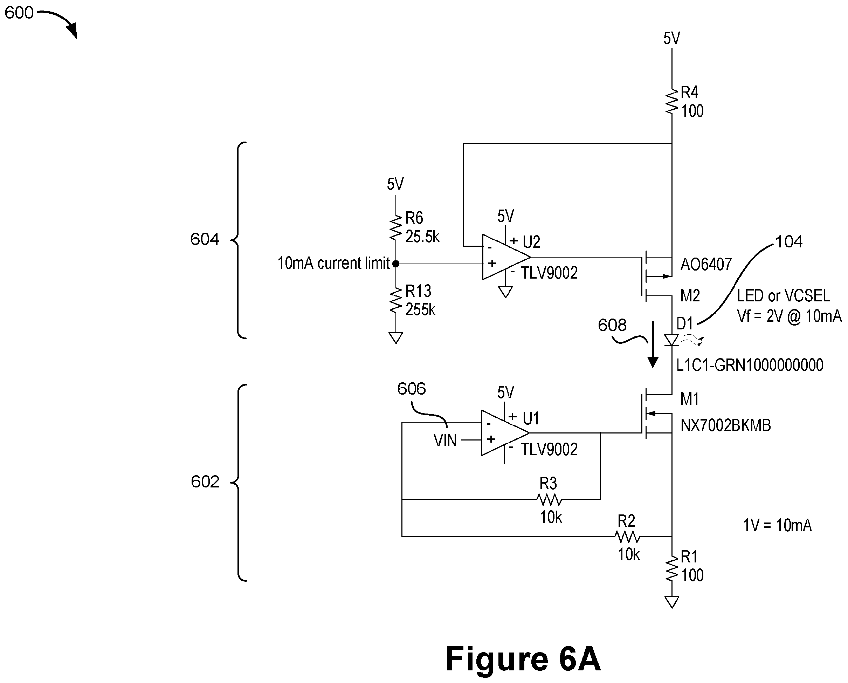

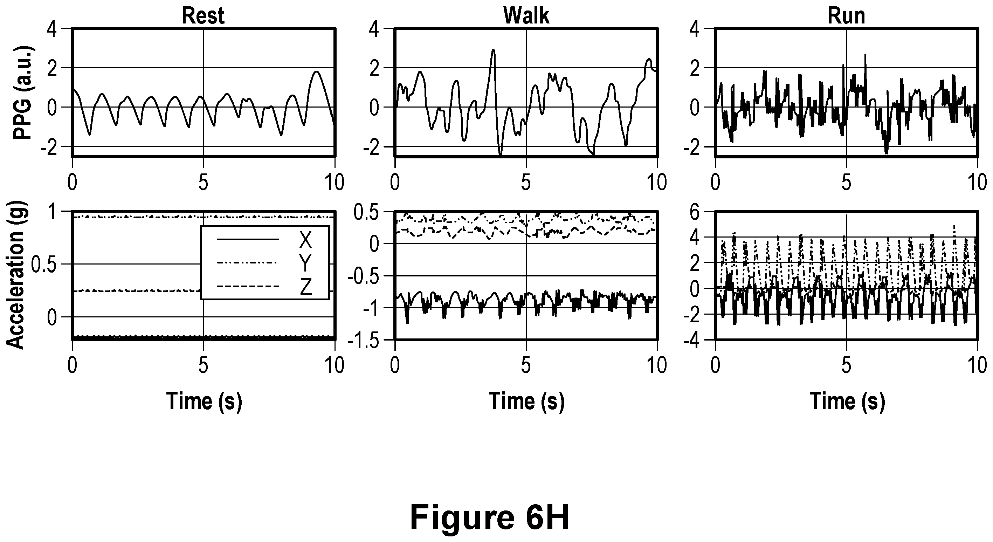

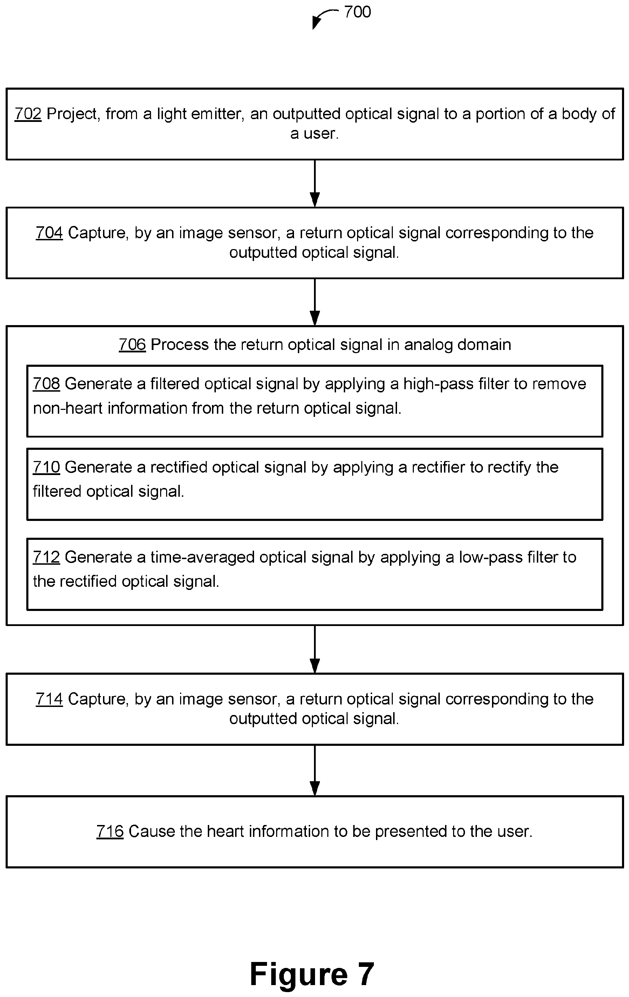

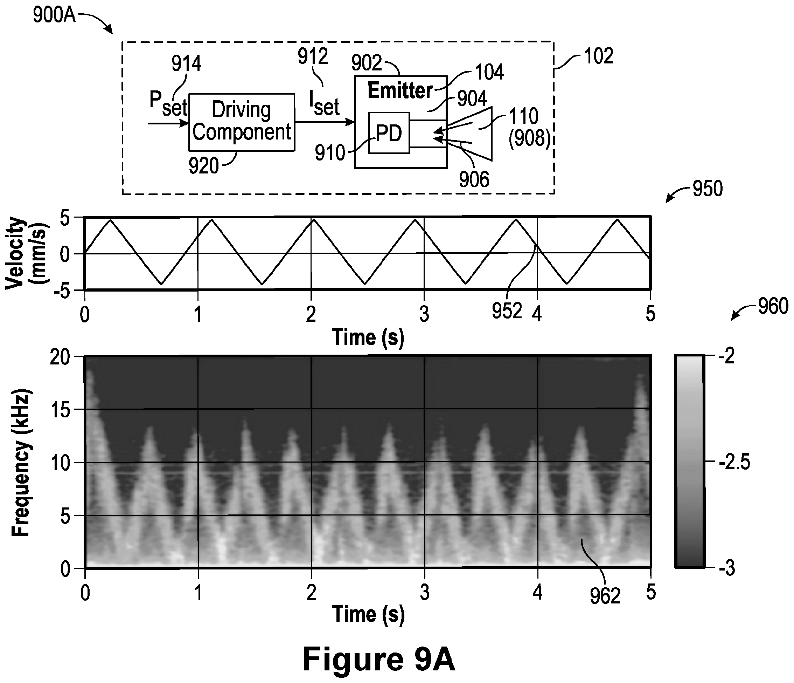

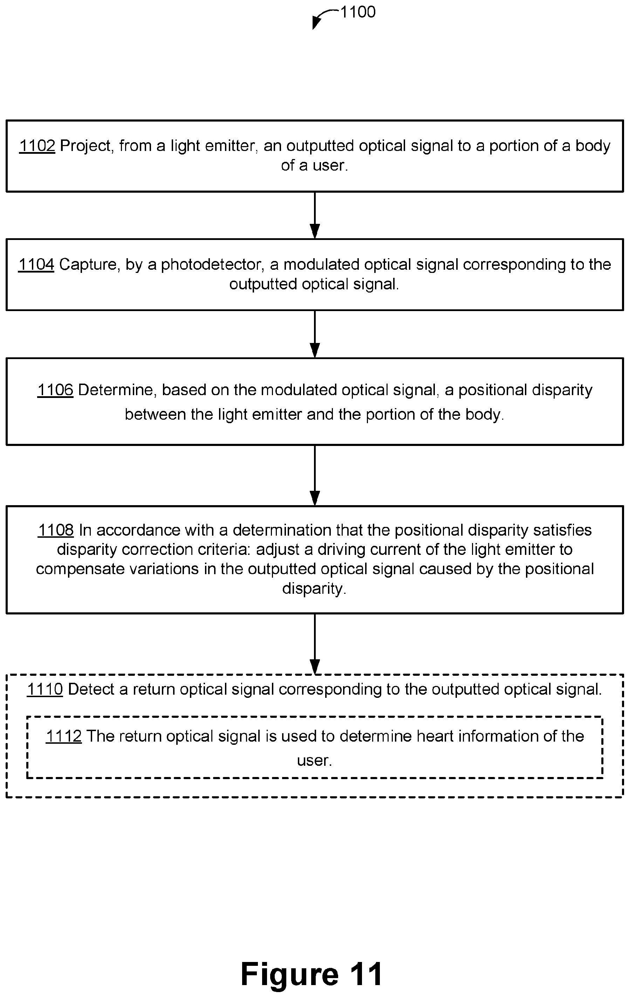

The following detailed description begins with an Overview of Extended-Reality Systems, describing various XR systems including AR glasses and MR headsets, as well as wrist-wearable devices and other electronic devices that may implement the LDF techniques described herein. Biometric Data Monitoring is then described with reference to Figures 1A-1B, 2, 3A-3B, and 4A-4C, covering LDF sensing unit configurations, analog signal processing circuitry, and methods for determining heart information of a user. Pulsed-mode LDF operation is addressed with reference to Figures 6A-6M, including driving circuits, timing diagrams for various pulsing schemes, hierarchical sample processing, and ambient light cancellation techniques. Don/doff and liveness detection are described with reference to Figures 5A-5I, including processing flows for detecting spectral distributions characteristic of perfused skin and receiver operating characteristic curves demonstrating detection performance. Motion artifact compensation is further addressed with reference to Figures 6H-6M, including examples of motion artifacts in PPG and LDF signals, self-mixing interferometry mechanisms, and self-compensation approaches using different frequency components of the LDF signal. Positional disparity correction is described with reference to Figures 9A-9B, 10, and 11, including configurations for compensating self-mixing interferometry effects through feedback control of the light emitter. Method flow diagrams are provided with reference to Figures 7 and 11, describing methods of operating a wearable device for biometric data monitoring and correcting positional disparity. The XR systems and wearable devices described in the overview may implement the LDF techniques described in the subsequent sections to provide non-contact biometric monitoring including heart rate measurement, don/doff detection, liveness detection, respiration rate estimation, and motion artifact compensation.

Numerous details are described herein to provide a thorough understanding of the example embodiments illustrated in the accompanying drawings. However, some embodiments may be practiced without many of the specific details, and the scope of the claims is only limited by those features and aspects specifically recited in the claims. Furthermore, well-known processes, components, and materials have not necessarily been described in exhaustive detail so as to avoid obscuring pertinent aspects of the embodiments described herein.

### Overview of Extended-Reality Systems

Embodiments of this disclosure can include or be implemented in conjunction with various types of XRs such as MR and AR systems. MRs and ARs, as described herein, are any superimposed functionality and/or sensory-detectable presentation provided by MR and AR systems within a user's physical surroundings. Such MRs can include and/or represent virtual realities (VRs) and VRs in which at least some aspects of the surrounding environment are reconstructed within the virtual environment (e.g., displaying virtual reconstructions of physical objects in a physical environment to avoid the user colliding with the physical objects in a surrounding physical environment). In the case of MRs, the surrounding environment that is presented through a display is captured via one or more sensors configured to capture the surrounding environment (e.g., a camera sensor, time-of-flight (ToF) sensor). While a wearer of an MR headset can see the surrounding environment in full detail, they are seeing a reconstruction of the environment reproduced using data from the one or more sensors (i.e., the physical objects are not directly viewed by the user). An MR headset can also forgo displaying reconstructions of objects in the physical environment, thereby providing a user with an entirely VR experience. An AR system, on the other hand, provides an experience in which information is provided, e.g., through the use of a waveguide, in conjunction with the direct viewing of at least some of the surrounding environment through a transparent or semitransparent waveguide(s) and/or lens(es) of the AR glasses. Throughout this application, the term "extended reality (XR)" is used as a catchall term to cover both ARs and MRs. In addition, this application also uses, at times, a head-wearable device or headset device as a catchall term that covers XR headsets such as AR glasses and MR headsets.

As alluded to above, an MR environment, as described herein, can include, but is not limited to, non-immersive, semi-immersive, and fully immersive VR environments. As also alluded to above, AR environments can include marker-based AR environments, markerless AR environments, location-based AR environments, and projection-based AR environments. The above descriptions are not exhaustive and any other environment that allows for intentional environmental lighting to pass through to the user would fall within the scope of an AR, and any other environment that does not allow for intentional environmental lighting to pass through to the user would fall within the scope of an MR.

The AR and MR content can include video, audio, haptic events, sensory events, or some combination thereof, any of which can be presented in a single channel or in multiple channels (such as stereo video that produces a three-dimensional effect to a viewer). Additionally, AR and MR can also be associated with applications, products, accessories, services, or some combination thereof, which are used, for example, to create content in an AR or MR environment and/or are otherwise used in (e.g., to perform activities in) AR and MR environments.

Interacting with these AR and MR environments described herein can occur using multiple different modalities and the resulting outputs can also occur across multiple different modalities. In one example AR or MR system, a user can perform a swiping in-air hand gesture to cause a song to be skipped by a song-providing application programming interface (API) providing playback at, for example, a home speaker.

A hand gesture, as described herein, can include an in-air gesture, a surface-contact gesture, and or other gestures that can be detected and determined based on movements of a single hand (e.g., a one-handed gesture performed with a user's hand that is detected by one or more sensors of a wearable device (e.g., electromyography (EMG) and/or inertial measurement units (IMUs) of a wrist-wearable device, and/or one or more sensors included in a smart textile wearable device) and/or detected via image data captured by an imaging device of a wearable device (e.g., a camera of a head-wearable device, an external tracking camera setup in the surrounding environment)). "In-air" generally includes gestures in which the user's hand does not contact a surface, object, or portion of an electronic device (e.g., a head-wearable device or other communicatively coupled device, such as the wrist-wearable device), in other words the gesture is performed in open air in 3D space and without contacting a surface, an object, or an electronic device. Surface-contact gestures (contacts at a surface, object, body part of the user, or electronic device) more generally are also contemplated in which a contact (or an intention to contact) is detected at a surface (e.g., a single- or double-finger tap on a table, on a user's hand or another finger, on the user's leg, a couch, a steering wheel). The different hand gestures disclosed herein can be detected using image data and/or sensor data (e.g., neuromuscular signals sensed by one or more biopotential sensors (e.g., EMG sensors) or other types of data from other sensors, such as proximity sensors, ToF sensors, sensors of an IMU, capacitive sensors, strain sensors) detected by a wearable device worn by the user and/or other electronic devices in the user's possession (e.g., smartphones, laptops, imaging devices, intermediary devices, and/or other devices described herein).

The input modalities as alluded to above can be varied and are dependent on a user's experience. For example, in an interaction in which a wrist-wearable device is used, a user can provide inputs using in-air or surface-contact gestures that are detected using neuromuscular signal sensors of the wrist-wearable device. In the event that a wrist-wearable device is not used, alternative and entirely interchangeable input modalities can be used instead, such as camera(s) located on the headset/glasses or elsewhere to detect in-air or surface-contact gestures or inputs at an intermediary processing device (e.g., through physical input components (e.g., buttons and trackpads)). These different input modalities can be interchanged based on both desired user experiences, portability, and/or a feature set of the product (e.g., a low-cost product may not include hand-tracking cameras).

While the inputs are varied, the resulting outputs stemming from the inputs are also varied. For example, an in-air gesture input detected by a camera of a head-wearable device can cause an output to occur at a head-wearable device or control another electronic device different from the head-wearable device. In another example, an input detected using data from a neuromuscular signal sensor can also cause an output to occur at a head-wearable device or control another electronic device different from the head-wearable device. While only a couple examples are described above, one skilled in the art would understand that different input modalities are interchangeable along with different output modalities in response to the inputs.

Specific operations described above may occur as a result of specific hardware. The devices described are not limiting and features on these devices can be removed or additional features can be added to these devices. The different devices can include one or more analogous hardware components. For brevity, analogous devices and components are described herein. Any differences in the devices and components are described below in their respective sections.

As described herein, a processor (e.g., a central processing unit (CPU) or microcontroller unit (MCU)), is an electronic component that is responsible for executing instructions and controlling the operation of an electronic device (e.g., a wrist-wearable device, a head-wearable device, a handheld intermediary processing device (HIPD), a smart textile-based garment, or other computer system). There are various types of processors that may be used interchangeably or specifically required by embodiments described herein. For example, a processor may be (i) a general processor designed to perform a wide range of tasks, such as running software applications, managing operating systems, and performing arithmetic and logical operations; (ii) a microcontroller designed for specific tasks such as controlling electronic devices, sensors, and motors; (iii) a graphics processing unit (GPU) designed to accelerate the creation and rendering of images, videos, and animations (e.g., VR animations, such as three-dimensional modeling); (iv) a field-programmable gate array (FPGA) that can be programmed and reconfigured after manufacturing and/or customized to perform specific tasks, such as signal processing, cryptography, and machine learning; or (v) a digital signal processor (DSP) designed to perform mathematical operations on signals such as audio, video, and radio waves. One of skill in the art will understand that one or more processors of one or more electronic devices may be used in various embodiments described herein.

As described herein, controllers are electronic components that manage and coordinate the operation of other components within an electronic device (e.g., controlling inputs, processing data, and/or generating outputs). Examples of controllers can include (i) microcontrollers, including small, low-power controllers that are commonly used in embedded systems and Internet of Things (IoT) devices; (ii) programmable logic controllers (PLCs) that may be configured to be used in industrial automation systems to control and monitor manufacturing processes; (iii) system-on-a-chip (SoC) controllers that integrate multiple components such as processors, memory, I/O interfaces, and other peripherals into a single chip; and/or (iv) DSPs. As described herein, a graphics module is a component or software module that is designed to handle graphical operations and/or processes and can include a hardware module and/or a software module.

As described herein, memory refers to electronic components in a computer or electronic device that store data and instructions for the processor to access and manipulate. The devices described herein can include volatile and non-volatile memory. Examples of memory can include (i) random access memory (RAM), such as DRAM, SRAM, DDR RAM or other random access solid state memory devices, configured to store data and instructions temporarily; (ii) read-only memory (ROM) configured to store data and instructions permanently (e.g., one or more portions of system firmware and/or boot loaders); (iii) flash memory, magnetic disk storage devices, optical disk storage devices, other non-volatile solid state storage devices, which can be configured to store data in electronic devices (e.g., universal serial bus (USB) drives, memory cards, and/or solid-state drives (SSDs)); and (iv) cache memory configured to temporarily store frequently accessed data and instructions. Memory, as described herein, can include structured data (e.g., SQL databases, MongoDB databases, GraphQL data, or JSON data). Other examples of memory can include (i) profile data, including user account data, user settings, and/or other user data stored by the user; (ii) sensor data detected and/or otherwise obtained by one or more sensors; (iii) media content data including stored image data, audio data, documents, and the like; (iv) application data, which can include data collected and/or otherwise obtained and stored during use of an application; and/or (v) any other types of data described herein.

As described herein, a power system of an electronic device is configured to convert incoming electrical power into a form that can be used to operate the device. A power system can include various components, including (i) a power source, which can be an alternating current (AC) adapter or a direct current (DC) adapter power supply; (ii) a charger input that can be configured to use a wired and/or wireless connection (which may be part of a peripheral interface, such as a USB, micro-USB interface, near-field magnetic coupling, magnetic inductive and magnetic resonance charging, and/or radio frequency (RF) charging); (iii) a power-management integrated circuit, configured to distribute power to various components of the device and ensure that the device operates within safe limits (e.g., regulating voltage, controlling current flow, and/or managing heat dissipation); and/or (iv) a battery configured to store power to provide usable power to components of one or more electronic devices.

As described herein, peripheral interfaces are electronic components (e.g., of electronic devices) that allow electronic devices to communicate with other devices or peripherals and can provide a means for input and output of data and signals. Examples of peripheral interfaces can include (i) USB and/or micro-USB interfaces configured for connecting devices to an electronic device; (ii) Bluetooth interfaces configured to allow devices to communicate with each other, including Bluetooth low energy (BLE); (iii) near-field communication (NFC) interfaces configured to be short-range wireless interfaces for operations such as access control; (iv) pogo pins, which may be small, spring-loaded pins configured to provide a charging interface; (v) wireless charging interfaces; (vi) global-positioning system (GPS) interfaces; (vii) Wi-Fi interfaces for providing a connection between a device and a wireless network; and (viii) sensor interfaces.

As described herein, sensors are electronic components (e.g., in and/or otherwise in electronic communication with electronic devices, such as wearable devices) configured to detect physical and environmental changes and generate electrical signals. Examples of sensors can include (i) imaging sensors for collecting imaging data (e.g., including one or more cameras disposed on a respective electronic device, such as a simultaneous localization and mapping (SLAM) camera); (ii) biopotential-signal sensors; (iii) IMUs for detecting, for example, angular rate, force, magnetic field, and/or changes in acceleration; (iv) heart rate sensors for measuring a user's heart rate; (v) peripheral oxygen saturation (SpO2) sensors for measuring blood oxygen saturation and/or other biometric data of a user; (vi) capacitive sensors for detecting changes in potential at a portion of a user's body (e.g., a sensor-skin interface) and/or the proximity of other devices or objects; (vii) sensors for detecting some inputs (e.g., capacitive and force sensors); and (viii) light sensors (e.g., ToF sensors, infrared light sensors, or visible light sensors), and/or sensors for sensing data from the user or the user's environment. As described herein biopotential-signal-sensing components are devices used to measure electrical activity within the body (e.g., biopotential-signal sensors). Some types of biopotential-signal sensors include (i) electroencephalography (EEG) sensors configured to measure electrical activity in the brain to diagnose neurological disorders; (ii) electrocardiography (ECG or EKG) sensors configured to measure electrical activity of the heart to diagnose heart problems; (iii) EMG sensors configured to measure the electrical activity of muscles and diagnose neuromuscular disorders; (iv) electrooculography (EOG) sensors configured to measure the electrical activity of eye muscles to detect eye movement and diagnose eye disorders.

As described herein, an application stored in memory of an electronic device (e.g., software) includes instructions stored in the memory. Examples of such applications include (i) games; (ii) word processors; (iii) messaging applications; (iv) media-streaming applications; (v) financial applications; (vi) calendars; (vii) clocks; (viii) web browsers; (ix) social media applications; (x) camera applications; (xi) web-based applications; (xii) health applications; (xiii) AR and MR applications; and/or (xiv) any other applications that can be stored in memory. The applications can operate in conjunction with data and/or one or more components of a device or communicatively coupled devices to perform one or more operations and/or functions.

As described herein, communication interface modules can include hardware and/or software capable of data communications using any of a variety of custom or standard wireless protocols (e.g., IEEE 802.15.4, Wi-Fi, ZigBee, 6LoWPAN, Thread, Z-Wave, Bluetooth Smart, ISA100.11a, WirelessHART, or MiWi), custom or standard wired protocols (e.g., Ethernet or HomePlug), and/or any other suitable communication protocol, including communication protocols not yet developed as of the filing date of this document. A communication interface is a mechanism that enables different systems or devices to exchange information and data with each other, including hardware, software, or a combination of both hardware and software. For example, a communication interface can refer to a physical connector and/or port on a device that enables communication with other devices (e.g., USB, Ethernet, HDMI, or Bluetooth). A communication interface can refer to a software layer that enables different software programs to communicate with each other (e.g., APIs and protocols such as HTTP and TCP/IP).

As described herein, a graphics module is a component or software module that is designed to handle graphical operations and/or processes and can include a hardware module and/or a software module.

As described herein, non-transitory computer-readable storage media are physical devices or storage medium that can be used to store electronic data in a non-transitory form (e.g., such that the data is stored permanently until it is intentionally deleted and/or modified).

### Biometric Data Monitoring

Various embodiments of this application are directed to methods, devices, and systems described for performing non-contact measurements for monitoring biometric data, e.g., heart information. The systems and device may include one or more wearable devices (e.g., any of the wearable devices described herein). The non-contact measurement may be based on principles of LDF in analog domain.

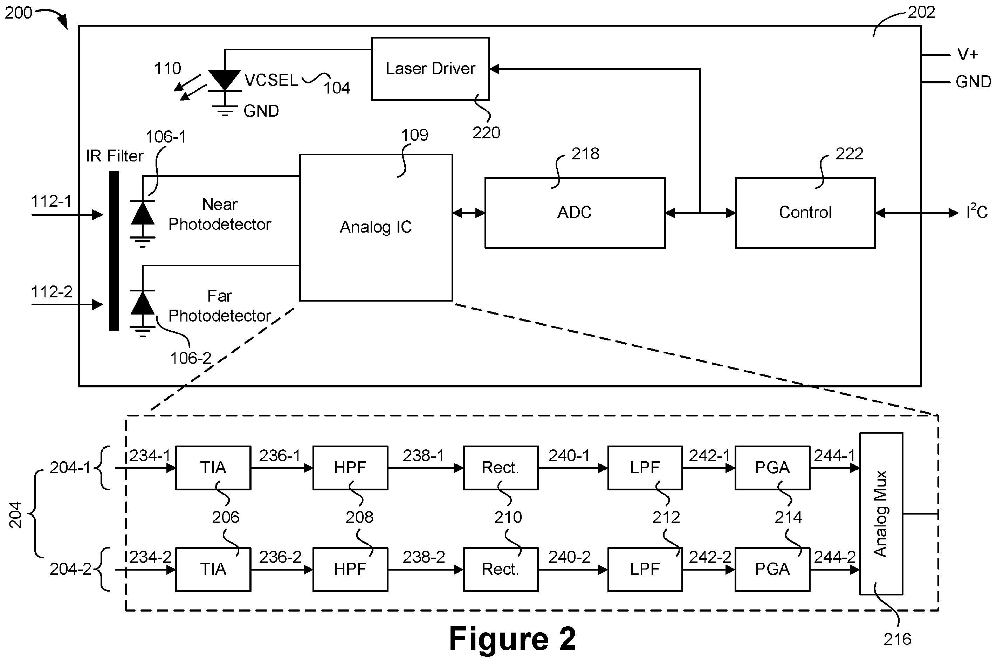

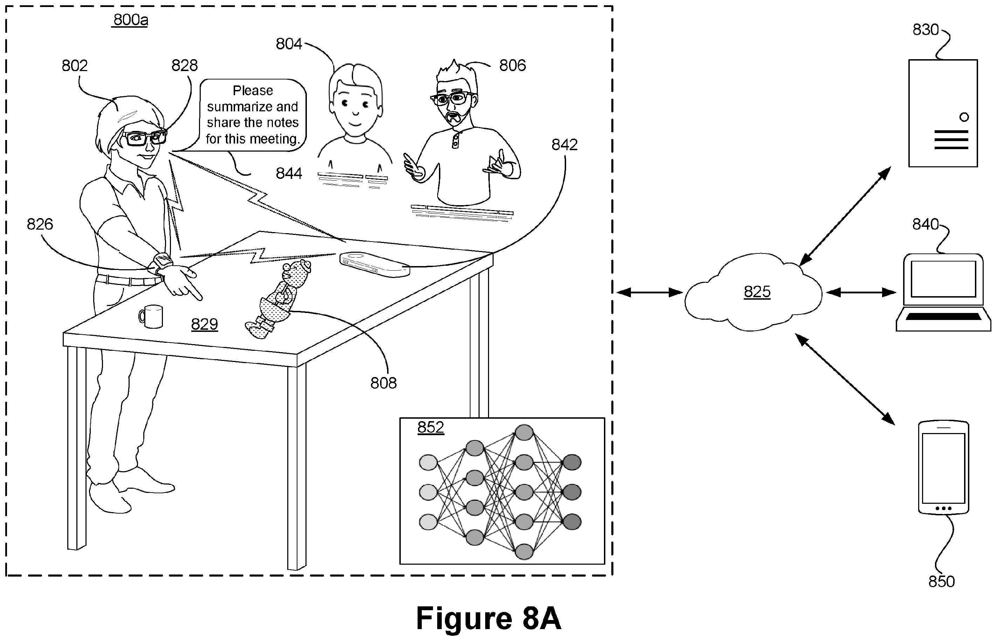

Figure 1A illustrates an example non-contact measurement system 100 of monitoring biometric data (e.g., heart information) using a wearable device 102, in accordance with some embodiments. In Figure 1A, a user 101 wears the wearable device 102 over a period time (e.g., from a starting time t0 to a first time t1, then to a second time t2, then to a third time t3, and so on). In some embodiments, the wearable device 102 comprises an extended-reality headset (e.g., a MR headset or a pair of AR glasses as two examples in reference to Figures 8A-8C). In some embodiments, the wearable device 102 comprises a wrist-wearable device (e.g., wrist-wearable device 826 in reference to Figures 8A-8C). In some embodiments, the wearable device 102 comprises a sensing unit 108 that includes one or more light emitters 104, one or more photodetectors 106, and an analog integrated circuit (IC) 109. In some embodiments, the one or more light emitters 104 comprise a laser source configured to project (e.g., emit) light. In some embodiments, the one or more photodetectors 106 comprise a photodiode configured to capture (e.g., detect) light. In some embodiments, the analog IC 109 is configured for analog signal processing, e.g., with customized analog signal processing functions. In some embodiments, the analog IC 109 comprises a high-pass filter, a rectifier, and a low-pass filter that are arranged on a printed circuit board (PCB) (e.g., discussed below with respect to Figure 2). In some embodiments, the wearable device 102 further includes a system chip 116 (e.g., a processing chip, a system-on-a-chip (SoC), etc.) configured to process an output signal from the analog IC 109, e.g., performing data analysis to extract heart information from the output signal. In some embodiments, the devices described herein include one or more photodetector(s). In some embodiments, the devices described herein include one or more image sensors (e.g., with each image sensor comprising one or more photodetectors).

In accordance with some embodiments, during biometric data monitoring, the wearable device 102 projects (e.g., emits), from the light emitter(s) 104 (e.g., laser source(s)), an outputted optical signal 110 (e.g., with beam(s) of coherent light) to a portion of a body 120 of the user 101. The wearable device 102 further captures, by the photodetector(s) 106 (e.g., photodiode(s)), a return optical signal 112 (e.g., scattered from the portion of the body 120) corresponding to the outputted optical signal 110. The wearable device 102 further processes the return optical signal 112 in an analog domain using the analog IC 109 to obtain a processed return optical signal 114. The wearable device 102 further determines heart information 118 of the user 101 based on the processed return optical signal 114, e.g., a period/frequency of heart rate. The wearable device 102 may cause the heart information 118 to be presented to the user 101, e.g., via a display of the wearable device 102 or a display communicatively coupled to the wearable device 102. In some embodiments, the wearable device 102 is configured to determine the heart information 118 of the user 101 using the system chip 116. In some embodiments, the portion of the body 120 comprises a face of the user 101 (e.g., when the heart information is monitored using an extended-reality headset). In some embodiments, the portion of the body 120 comprises a wrist of the user 101 (e.g., when the heart information is monitored using a wrist-wearable device). For example, the portion of the body 120 may comprise any portion of the body through which sufficient blood is flowing.

In some embodiments, the wearable device 102 is configured to perform operations for monitoring heart information of the user 101 in accordance with LDF techniques. In some embodiments, the LDF techniques involve collecting and processing an optical signal (e.g., return optical signal 112) at a sampling frequency (e.g., 10 kHz, 30 kHz, or 100 kHz) configured to capture heart information, e.g., greater than those frequencies conventionally employed in wearable biosensing applications (e.g., wearable biosensors). In some embodiments, during heart information monitoring, the outputted optical signal 110 projected (e.g., emitted) by the light emitter 104 is scattered from the portion of the body 120 of the user 101 (e.g., a target portion of a body such as skin(s), shallow soft tissue(s), blood-perfused region(s), etc.) due to motion of the blood in the body 120. Thus, the outputted optical signal 110 being scattered exhibits a blood motion-induced Doppler shift (e.g., a small shift in frequency).

In some embodiments, the return optical signal 112 comprises a Doppler-shifted optical signal and an unshifted optical signal (e.g., not exhibiting a blood motion-induced Doppler shift). The Doppler-shifted optical signal interferes with the unshifted optical signal at the photodetector 106 to generate a beat frequency. The beat frequency comprises a difference between the frequencies of the Doppler-shifted optical signal and the unshifted optical signal. For example, the beat frequency may fall within a characteristic range of kHz that represents superficial blood flow velocities. In some embodiments, the return optical signal 112 comprises a plurality of flow-induced optical frequency shifts in a range from 1 kHz to 50 kHz. For example, the frequencies may be in a range from 4 kHz to 25 kHz or 8 kHz to 25 kHz. In some embodiments, a target frequency for heart rate detection is 25 kHz.

In some embodiments, the heart information comprises heart rate information, heartbeat (e.g., arrhythmia) information, and/or other types of heart-related information (e.g., blood pressure information). In some embodiments, the heart information comprises microvasculature information. In some embodiments, the microvasculature information comprises components at a beat frequency on the order of 10 kHz.

In some embodiments, the heart information is presented to the user 101 via a graphical user interface on the wearable device 102. In some embodiments, the graphical user interface corresponds to an application executing at the wearable device 102. In some embodiments, the heart information is presented to the user via a display that is exterior to and communicatively coupled to the wearable device 102. In some embodiments, the heart information is presented to the user via a display of the wearable device 102.

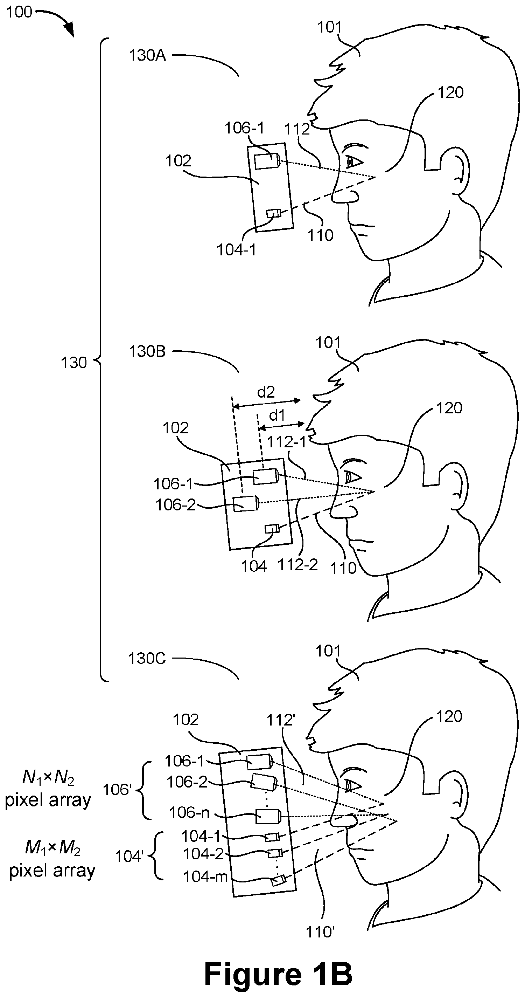

Figure 1B illustrates example configurations 130 (e.g., configurations 130A, 130B, and 130C) for biometric data monitoring implemented in the wearable device 102, in accordance with some embodiments. In some embodiments, the wearable device 102 comprises an extended-reality headset or a wrist-wearable device.

In some embodiments, the wearable device 102 implements a single-channel configuration 130A for biometric data monitoring. As an example, in the single-channel configuration 130A, the light emitter 104 may be a light emitter 104-1 and the photodetector 106 may be a photodetector 106-1, e.g., the light emitter 104-1 being paired with the photodetector 106-1. In some embodiments, the single-channel configuration 130A is implemented, e.g., to reduce hardware complexity (e.g., and simplify system assembly and integration), lower power consumption (e.g., enabling longer battery usage), and achieve high-compact form factor (e.g., reducing footprint), thereby facilitating integration of biometric data monitoring into existing designs for wearable devices.

In some embodiments, the wearable device 102 implements a dual-channel configuration 130B for biometric data monitoring. For example, in the dual-channel configuration 130B, the photodetector(s) 106 may comprise the photodetector 106-1 and a photodetector 106-2. The photodetector 106-1 (e.g., a near photodiode) and the photodetector 106-2 (e.g., a far photodiode) are arranged to be at different distances (e.g., d1 and d2, where d1 is less than d2) from the portion of the body 120 while the wearable device 102 is being worn. Moreover, the return optical signal 112 comprises a return optical signal 112-1 detected by the photodetector 106-1 and a return optical signal 112-2 detected by the photodetector 106-2. Accordingly, the return optical signal 112-1 may be processed in a channel of the analog IC 109 in the analog domain, and the return optical signal 112-2 may be processed in a second channel of the analog IC 109 in the analog domain (e.g., as discussed below with reference to Figure 2). In some embodiments, the light emitter 104 in the dual-channel configuration 130B comprises a first light emitter and a second light emitter, and each light emitter is paired with one of the photodetector 106-1 and the photodetector 106-2. In some embodiments, the photodetector 106-1 and the photodetector 106-2 are arranged to be at the same distance (e.g., d1 equal to d2) from the portion of the body 120.

In some embodiments, the wearable device 102 implements a multi-channel configuration 130C for biometric data monitoring. For example, in the multi-channel configuration 130C, the light emitter 104 may comprise a plurality of light emitters 104' (e.g., light emitter 104-1 to light emitter 104-m, where m is an integer greater than one) configured to project (e.g., emit) the plurality of outputted light signals 110', and the photodetector 106 may comprise a plurality of photodetectors 106' (e.g., photodetector 106-1 to photodetector 106-n, where n is an integer greater than one) configured to capture a plurality of return optical signals 112' corresponding to the plurality of outputted light signals 110'. In some embodiments, each light emitter of the plurality of light emitters 104' is paired with a respective photodetector (e.g., in a one-to-one fashion). For example, the light emitter 104-1 is paired with the photodetector 106-1, e.g., the photodetector 106-1 is configured to receive a respective return optical signal scattered from the portion of the body 120 that corresponds to an output optical signal from the light emitter 104-1. In some embodiments, each light emitter of the plurality of light emitters 104' is paired with one or more respective photodetectors (e.g., in a one-to-multiple fashion similar to the dual-channel configuration 130B). For example, the light emitter 104-1 is paired with the photodetector 106-1 and the photodetector 106-2, e.g., the photodetectors 106-1 and 106-2 is configured to receive a respective return optical signal scattered from the portion of the body 120 that corresponds to an output optical signal from the light emitter 104-1. In some embodiments, one or more respective light emitters are paired with a respective photodetector of the plurality of photodetectors 106' (e.g., in a multiple-to-one fashion). For example, the light emitter 104-1 and the light emitter 104-2 are paired with the photodetector 106-1, e.g., the photodetector 106-1 is configured to receive respective return optical signals scattered from the portion of the body 120 that correspond to output optical signals from the light emitters 104-1 and 104-2. In some embodiments, the plurality of light emitters 104' (e.g., light emitter 104-1 to light emitter 104-m) are arranged in an M1×M2 pixel array (e.g., M1 and M2 are integers greater than one). In some embodiments, the plurality of photodetectors 106' (e.g., photodetector 106-1 to photodetector 106-n) are arranged in an N1×N2 pixel array (e.g., N1 and N2 are integers greater than one). In some embodiments, the M1×M2 pixel array of the plurality of light emitters 104' and the N1×N2 pixel array of the plurality of photodetectors 106' are configured to monitor biometric data in accordance with any of the configurations 130 (e.g., single-channel configuration 130A, dual-channel configuration 130B, multi-channel configuration 130C) or their combination(s) thereof.

In some embodiments, each channel within a specific configuration (e.g., configurations 130A, 130B, or 130C) corresponds a distinct signal processing channel of the analog IC 109. For example, channels within the dual-channel configuration 130B correspond to a first signal processing channel and a second signal processing channel of the analog IC 109, respectively (e.g., discussed below with respect to Figure 2). In some embodiments, the wearable device 102 is configured to operate one signal processing channel (e.g., a first signal processing channel of the analog IC 109) while other signal processing channel(s) (e.g., second signal processing of the analog IC 109) are disabled (e.g., only a single channel is active at a time). In some embodiments, the signal processing channels of the analog IC 109 are substantially identical to one another, e.g., sharing similar IC circuities with the same electrical components (e.g., amplifiers, rectifiers, filters). In some embodiments, the wearable device 102 determines the heart information of the user 101 based on a subset of the return optical signal(s) (e.g., either of the return optical signals 112-1 and 112-2 in the dual-channel configuration 130B, a subset of the plurality of return optical signals 112' in the multi-channel configuration 130C, etc.). As an example, the wearable device 102 may determine a signal-to-noise ratio (SNR) for each return optical signal and select a respective return optical signal having the highest SNR for use with determining the heart information for a particular time period. For example, in the dual-channel configuration 130B, during a time period from t1 to t2, the wearable device 102 may be configured to select the return optical signal 112-1, captured by the photodetector 106-1, for determining the heart information; and, during a time period from t2 to t3, the wearable device 102 may be configured to select the return optical signal 112-2, captured by the photodetector 106-2, for determining the heart information.

In some embodiments, the light emitter(s) of the wearable device 102 are arranged to be an equal distance to the portion of the body 120 while the wearable device 102 is worn. For example, each light emitter (e.g., light emitter 104-1 to light emitter 104-m) of the plurality of light emitters 104' may be placed at an equal distance from the portion of the body 120 while the wearable device 102 is worn. In some embodiments, the photodetector(s) of the wearable device 102 are arranged to be an equal distance to the portion of the body 120 while the wearable device 102 is worn. For example, each photodetector (e.g., photodetector 106-1 to photodetector 106-n) of the plurality of photodetectors 106' may be placed at an equal distance from the portion of the body 120 while the wearable device 102 is worn. In some embodiments, at least a subset of the photodetector(s) of the wearable device 102 is arranged to be at different distances to the portion of the body 120 while the wearable device 102 is worn. For example, in the dual-channel configuration 130B, the photodetector 106-1 may be positioned to be proximal to the portion of the body 120 and the photodetector 106-2 may be positioned to be distal to the portion of the body 120 while the wearable device 102 is being worn.

In some embodiments, respective return optical signal(s) are captured from distinct angle(s), e.g., depending on contours of the portion of the body 120 and/or angles between respective photodetector(s) and the portion of the body 120. For example, as shown in Figure 1B, angles between the portion of the body 120 and at least a subset of the plurality of photodetectors 106' are distinct from one another. In some embodiments, respective output optical signal(s) are projected (e.g., emitted) from distinct angle(s), e.g., depending on contours of the portion of the body 120 and/or angles between respective light emitter(s) and the portion of the body 120. For example, as shown in Figure 1B, angles between the portion of the body 120 and at least a subset of the plurality of light emitters 104' are distinct from one another.

In some embodiments, the light emitter 104 and the photodetector 106 are arranged on a same circuit board. In some embodiments, the light emitter 104 and the photodetector 106 are positioned adjacent to one another on the same circuit board. In some embodiments, the light emitter 104, the photodetector 106, and the analog IC 109 comprising a high-pass filter, a rectifier, and a low-pass filter are assembled on a same circuit board. In some embodiments, the circuit board is implemented as a printed circuit board (PCB). In some embodiments, the circuit board is part of the sensing unit 108. In some embodiments, the light emitter 104 and the photodetector 106 are arranged on different circuit boards.

In some embodiments, the sensing unit 108 (or sensing unit 202) is implemented as an integrated sensing module that packages the light emitter 104 (e.g., a VCSEL), the photodetector 106 (e.g., one or more photodiodes), and data acquisition (DAQ) electronics into a single module package. In some embodiments, the DAQ electronics include the analog IC 109, the ADC 218, the driver 220, and the control component 222. In some embodiments, the integrated sensing module provides a compact, self-contained unit that can be readily integrated into various wearable device form factors, including extended-reality headsets, wrist-wearable devices, earbuds, smart rings, and other body-worn devices. In some embodiments, the integrated sensing module reduces system complexity by consolidating optical and electronic components into a single package with defined electrical and optical interfaces.

In some embodiments, the integrated sensing module includes one or more optical windows that are optically transparent at the operating wavelength of the light emitter 104 and provide environmental protection for the optical components within the module. In some embodiments, the one or more optical windows include a transmit window aligned with the light emitter 104 and a receive window aligned with the photodetector 106. In some embodiments, the one or more optical windows include a shared window that serves both the light emitter 104 and the photodetector 106. In some embodiments, the optical window comprises glass, sapphire, optical-grade polymer, or other optically transparent materials suitable for the operating wavelength (e.g., 850 nm or 940 nm). In some embodiments, the optical window includes one or more coatings, such as anti-reflection coatings to reduce optical losses, bandpass filter coatings to reject out-of-band light, or infrared-pass coatings to transmit near-infrared light while blocking visible light. In some embodiments, the optical window is bonded to the module housing using adhesive bonding, glass frit bonding, laser welding, or other sealing techniques. In some embodiments, the optical window provides a hermetic or near-hermetic seal to protect the optical components from moisture, dust, and contaminants.

In some embodiments, the light emitter 104 and the photodetector 106 are positioned on a common substrate or submount within the module housing. In some embodiments, the common substrate comprises silicon, ceramic (e.g., aluminum oxide, aluminum nitride), or other suitable substrate materials. In some embodiments, the DAQ electronics (e.g., the transimpedance amplifier 206, the high-pass filter 208, the rectifier 210, the low-pass filter 212, the programmable gain amplifier 214, the ADC 218, the driver 220, and the control component 222) are integrated on the same substrate as the light emitter 104 and the photodetector 106. In some embodiments, the DAQ electronics are implemented on a separate substrate or die within the module housing. In some embodiments, electrical interconnections between the light emitter 104, the photodetector 106, and the DAQ electronics are formed using wire bonding (e.g., gold wire bonding, aluminum wire bonding) or flip-chip bonding (e.g., solder bumps, copper pillars). In some embodiments, the substrates within the module are interconnected using wire bonds, ribbon bonds, or substrate-to-substrate interconnects.

In some embodiments, the module housing comprises ceramic (e.g., low-temperature co-fired ceramic (LTCC), high-temperature co-fired ceramic (HTCC)), molded plastic, metal (e.g., aluminum, copper, Kovar), or combinations thereof. In some embodiments, the module housing provides electromagnetic shielding to reduce electromagnetic interference (EMI) with other electronic components in the wearable device. In some embodiments, the module housing provides thermal management by conducting heat away from the light emitter 104 and the DAQ electronics. In some embodiments, the module housing includes a thermal interface material or heat spreader to facilitate heat dissipation. In some embodiments, the module housing provides optical isolation between the transmit path (associated with the light emitter 104) and the receive path (associated with the photodetector 106) to reduce optical crosstalk. In some embodiments, the module housing includes one or more optical barriers or baffles positioned between the light emitter 104 and the photodetector 106 to block direct optical paths and reduce stray light coupling. In some embodiments, the optical barriers comprise opaque materials such as black epoxy, metal partitions, or absorptive coatings.

In some embodiments, the one or more optical windows are aligned with apertures of the light emitter 104 and the photodetector 106 within the module housing. In some embodiments, the transmit window is positioned directly above the light emitter 104 such that the outputted optical signal 110 passes through the transmit window with minimal optical loss. In some embodiments, the receive window is positioned directly above the photodetector 106 such that the return optical signal 112 passes through the receive window to the photodetector 106. In some embodiments, the optical windows are recessed within the module housing or are flush with an outer surface of the module housing. In some embodiments, the optical windows include alignment features (e.g., fiducials, mechanical stops) to facilitate precise positioning during module assembly.

In some embodiments, the integrated sensing module is manufactured using semiconductor packaging techniques. In some embodiments, the module is manufactured using chip-on-board (COB) techniques in which bare dies (e.g., the light emitter 104, the photodetector 106, DAQ electronics dies) are attached directly to a substrate and wire bonded. In some embodiments, the module is manufactured using system-in-package (SiP) techniques in which multiple dies and passive components are integrated within a single package. In some embodiments, the module is manufactured using multi-chip module (MCM) techniques in which multiple dies are mounted on a common substrate with high-density interconnects. In some embodiments, the manufacturing process includes wafer-level packaging steps in which packaging structures are formed on the wafer prior to singulation. In some embodiments, the manufacturing process includes die attach steps in which individual dies are attached to the substrate using adhesive die attach, eutectic die attach, or solder die attach. In some embodiments, the manufacturing process includes wire bonding steps in which electrical connections are formed between bond pads on the dies and bond pads on the substrate. In some embodiments, the manufacturing process includes encapsulation steps in which the dies and wire bonds are protected by an encapsulant material (e.g., epoxy molding compound, glob-top encapsulant). In some embodiments, the manufacturing process includes singulation steps in which individual modules are separated from a panel or strip of modules.

In some embodiments, the integrated sensing module has defined external electrical interfaces for integration into the wearable device. In some embodiments, the external electrical interfaces include an I2C interface, a serial peripheral interface (SPI), a universal asynchronous receiver-transmitter (UART) interface, or other serial communication interfaces. In some embodiments, the external electrical interfaces include power supply connections (e.g., V+, GND) and control signal connections. In some embodiments, the integrated sensing module has defined optical interfaces comprising the one or more optical windows for transmitting and receiving optical signals. In some embodiments, the integrated sensing module has a compact form factor with dimensions suitable for integration into wearable devices, such as dimensions on the order of 5 mm × 5 mm × 3 mm or smaller. In some embodiments, the integrated sensing module is surface-mountable and can be attached to a host printed circuit board of the wearable device using surface-mount technology (SMT) processes such as solder reflow.

Although specific module configurations are described above, the present disclosure is not limited to these particular implementations. In some embodiments, the integrated sensing module includes different window configurations, such as a single shared window, separate transmit and receive windows, or multiple windows for multi-channel configurations. In some embodiments, the substrate comprises materials other than silicon or ceramic, such as glass, organic laminates, or flexible substrates. In some embodiments, the bonding techniques include techniques other than wire bonding or flip-chip bonding, such as through-silicon vias (TSVs), interposers, or embedded die technologies. In some embodiments, the module housing comprises materials other than ceramic, plastic, or metal, such as glass housings or composite materials. In some embodiments, the optical window coatings include coatings other than anti-reflection or bandpass coatings, such as hydrophobic coatings, oleophobic coatings, or scratch-resistant coatings. In some embodiments, the module includes additional components such as temperature sensors, reference photodetectors, or calibration elements. In some embodiments, the module includes multiple light emitters and/or multiple photodetectors arranged in arrays for multi-channel sensing configurations. Accordingly, the specific module configurations described above are provided as examples and should not be construed as limiting the scope of the present disclosure, the invention being defined in the appended claims.

In some embodiments, the light emitter 104 comprises a vertical-cavity surface-emitting laser (VCSEL). In some embodiments, the light emitter 104 includes VCSELs arranged in an M₁×M₂ pixel array.

In some embodiments, the light emitter 104 is configured to operate in a near-infrared band. In some embodiments, the light emitter 104 is configured to emit light at a wavelength (e.g., nominal emission wavelength) of approximately 850 nm (e.g., within 10%, 5%, 2%, or 1% of 850 nm). In some embodiments, the light emitter 104 is configured to emit light at a wavelength (e.g., nominal emission wavelength) of approximately 940 nm (e.g., within 10%, 5%, 2%, or 1% of 940 nm). In some embodiments, the wavelength of the light emitter 104 is selected to better ensure eye safety for the user.

In some embodiments, the light emitter 104 is positioned such that an emission axis of the light emitter 104 is normal (e.g., perpendicular) to a surface of the portion of the body 120 while the wearable device 102 is being worn by the user 101, e.g., the outputted optical signal 110 is incident normal to the surface. In some circumstances, this perpendicular alignment enhances efficiency of optical signal transmission through the surface (e.g., a skin) with minimized angular distortion and/or scattering effect. In some embodiments, implementing normal incidence simplifies optical calibration of the light emitter 104 across (i) different portions of a body of a single user and/or (ii) different users.

In some embodiments, the light emitter 104 comprises a laser component configured to emit light having a coherence length greater than a distance (e.g., distance d1, distance d2) between the photodetector 106 and the portion of the body 120. In some embodiments, the wearable device 102 is configured such that the distance between the photodetector 106 and the portion of the body 120 is 1 cm to 5 cm (e.g., a distance of 2 cm) while the wearable device 102 is worn by the user 101. In some embodiments, the light emitter 104 is configured to have a long coherent length and a corresponding narrow emission linewidth, thereby enhancing measurement precision in an interference-based or depth-resolving optical system (e.g., non-contact measurement system 100 based on LDF).

In some embodiments, the light emitter 104 is configured to operate without a feedback loop. In some embodiments, the driving circuit for the light emitter operates without requiring automatic pulse control (APC), e.g., APC can be disabled, which allows the non-contact measurement system 100 to operate with only minimal modifications to existing control logic. For example, APC may be used to modulate output power of the light emitter 104 (e.g., a laser source) by adjusting its drive current based on feedback. When APC is disabled, the wearable device 102 no longer requires real-time monitoring or adjustment of the light emitter 104, and thus a control logic can be simplified. Accordingly, the control circuitry or firmware for the light emitter 104 can be implemented with reduced complexity, e.g., fewer circuit components or less complex logic responsible for managing the light emitter 104. In some embodiments, the driving circuit for the light emitter operates with APC. In some embodiments, the light emitter 104 and its driving circuit for the light emitter 104 is part of the sensing unit 108.

In some embodiments, the photodetector 106 comprises a silicon (Si) photodiode. In some embodiments, the photodetector 106 comprises a gallium arsenide (GaAs) photodiode. In some embodiments, the photodetector 106 and its driving circuit are part of the sensing unit 108.

Although Figures 1A and 1B illustrate specific configurations for biometric data monitoring using a wearable device, the present disclosure is not limited to these particular implementations. In some embodiments, the wearable device 102 comprises a form factor other than extended-reality headsets, including wrist-wearable devices, earbuds, smart rings, smart patches, smart textile-based garments, or other body-worn devices. In some embodiments, the portion of the body targeted for sensing includes areas other than the face, such as the wrist, ear, neck, chest, temple, or other regions with sufficient blood perfusion. In some embodiments, the light emitter comprises light sources other than VCSELs, including edge-emitting lasers (EELs), superluminescent diodes (SLDs), light-emitting diodes (LEDs), and/or other coherent or partially coherent light sources. In some embodiments, the light emitter operates at wavelengths other than 850 nm or 940 nm, including other near-infrared wavelengths, visible wavelengths, or short-wave infrared (SWIR) wavelengths selected based on tissue penetration depth, eye safety considerations, or compatibility with other optical systems. In some embodiments, the photodetector comprises detector types other than silicon or gallium arsenide photodiodes, including avalanche photodiodes (APDs), single-photon avalanche diodes (SPADs), or other photosensitive elements. In some embodiments, other channel configurations are employed, such as configurations with varying numbers of light emitters and photodetectors arranged in different spatial patterns. In some embodiments, signal processing is performed in the digital domain rather than the analog domain, or using a combination of analog and digital processing. In some embodiments, signal processing is performed on-device, off-device at a communicatively coupled computing device, or distributed across multiple devices. In some embodiments, the LDF techniques described herein are applied to applications beyond heart rate monitoring, such as blood pressure estimation, oxygen saturation measurement, stress detection, hydration monitoring, and/or other physiological assessments. Accordingly, the specific configurations illustrated in Figures 1A and 1B are provided as examples and should not be construed as limiting the scope of the present disclosure, the invention being defined in the appended claims.

Figure 2 illustrates a block diagram 200 of a sensing unit 202 of the wearable device 102, in accordance with some embodiments. In some embodiments, the sensing unit 202 is an instance of the sensing unit 108 (e.g., illustrated in Figures 1A and 1B). In some embodiments, the sensing unit 202 comprises the light emitter 104 configured to project (e.g., emit) the outputted optical signal 110, the photodetector 106 configured to capture (e.g., detect) the return optical signal 112, and the analog IC 109 configured to process the return optical signal 112 for obtaining the heart information of the user 101. In some embodiments, the sensing unit 202 comprises a driver 220 (e.g., a laser driver) configured to drive the light emitter 104. In some embodiments, the sensing unit 202 further includes infrared filter(s) (e.g., infrared bandpass filter(s), infrared neutral density filters, etc.) for filtering the return optical signal 112 in accordance with optical sensing requirements/specifications, prior to the return optical signal 112 being captured by the photodetector 106. In this circumstance, the filtered return optical signal is processed by the analog IC 109 of the sensing unit 202. In some embodiments, the photodetector 106 is configured to generate, based on the return optical signal 112 or the filtered return optical signal (e.g., by infrared filter(s)), a detected optical signal (e.g., detected optical signal 234-1, detected optical signal 234-2) for subsequent signal processing by the analog IC 109 of the sensing unit 202. In some embodiments, the detected optical signal is a photocurrent (e.g., a low-level photocurrent).

In some embodiments, the sensing unit 202 of the wearable device 102 implements a dual-channel configuration 130B for biometric data monitoring. The photodetector 106 of the sensing unit 202 comprises the photodetector 106-1 (e.g., a near photodiode), which is configured to capture (e.g., detect) the return optical signal 112-1, and a photodetector 106-2 (e.g., a far photodiode), which is configured to capture (e.g., detect) the return optical signal 112-2. For example, the photodetector 106-1 is positioned to be proximal (e.g., at a distance d1) to the portion of the body 120 and the photodetector 106-2 is positioned to be distal (e.g., at a distance d2) to the portion of the body 120 while the wearable device 102 is being worn. The analog IC 109 of the sensing unit 202 comprises a signal processing channel 204-1 and a signal processing channel 204-2. The signal processing channels 204-1 and 204-2 are configured to process the return optical signals 112-1 and 112-2 in the analog domain, respectively. In some embodiments, each signal processing channel (e.g., signal processing channel 204-1, signal processing channel 204-2) of the analog IC 109 comprises a transimpedance amplifier 206, a high-pass filter 208, a rectifier 210, a low-pass filter 212, and a programmable gain amplifier 214. In some circumstances, each signal processing channel of the analog IC 109 includes a respective subset of these electrical components. For example, a signal processing channel may include the high-pass filter 208, the rectifier 210, and the low-pass filter 212, and may not include the transimpedance amplifier 206 and/or the programmable gain amplifier 214. In some embodiments, a processing channel may include one or more components not shown in Figure 2 (e.g., a capacitor, inductor, transistor, etc.). In some embodiments, the analog IC 109 includes an analog multiplexer 216 configured to couple the signal processing channels 204-1 and 204-2 to an analog-to-digital converter (ADC) 218 of the sensing unit 202. In some embodiments, the sensing unit 202 of the wearable device 102 implements a single-channel configuration 130A for biometric data monitoring. In some embodiments, the sensing unit 202 is communicatively coupled to an I²C interface through a control component 222 of the sensing unit 202. In some embodiments, the control component 222 is communicatively coupled to the ADC 218 and the driver 220.

In some embodiments, a respective signal processing channel (e.g., signal processing channel 204-1 or signal processing channel 204-2) of the analog IC 109 comprises the transimpedance amplifier 206 configured to generate a respective converted optical signal (e.g., converted optical signal 236-1 or converted optical signal 236-2) by amplifying a respective detected optical signal (e.g., detected optical signal 234-1 or detected optical signal 234-2) that corresponds to a respective return optical signal (e.g., return optical signal 112-1 or return optical signal 112-2). In some embodiments, the transimpedance amplifier 206 is electrically coupled to the photodetector 106 (e.g., an input of the transimpedance amplifier 206 is electrically coupled to an output of the photodetector 106). As an example, the transimpedance amplifier 206 may be configured to covert a low-level photocurrent (e.g., respective detected optical signal) generated by the photodetector 204 to a corresponding voltage suitable (e.g., respective converted optical signal) for further analog and/or digital processing. In some embodiments, the transimpedance amplifier 206 includes an operational amplifier (op-amp) with a feedback loop having resistor components and capacitor components. Values of these resistor components and capacitor components may be selected so as to maintain stability of the transimpedance amplifier 206. In some circumstances, the resistor components and capacitor components of the transimpedance amplifier 206 provide a low-pass filter characteristic for filtering the respective return optical signal and setting an operational bandwidth of the transimpedance amplifier 206. In some embodiments, the transimpedance amplifier 206 has a bandwidth of at least 5 kHz, 10 kHz, 25 kHz, or 50 kHz. For example, the transimpedance amplifier 206 may have a bandwidth in the range of 8 kHz to 40 kHz. In another example, the bandwidth (e.g., on the order of 25 kHz) of the transimpedance amplifier 206 may be selected to suppress amplification of noise component(s) outside of a frequency band of interest (e.g., microvasculature component on the order of 10 kHz) associated with the heart information of the user 101. In some embodiments, a gain of the transimpedance amplifier 206 is in a range of 1×10⁵ V/A to 1×10⁷ V/A. In some embodiments, the gain of the transimpedance amplifier 206 is configured to be 1×10⁶ V/A. In some embodiments, the gain of the transimpedance amplifier 206 is tunable.

In some embodiments, a respective signal processing channel (e.g., signal processing channel 204-1, signal processing channel 204-2) of the analog IC 109 comprises the high-pass filter 208 configured to generate a respective filtered optical signal (e.g., filtered optical signal 238-1 or filtered optical signal 238-2) by removing non-heart information from a respective return optical signal (e.g., return optical signal 112-1 or return optical signal 112-2). In some embodiments, the high-pass filter 208 is electrically coupled to the photodetector (e.g., an input of the high-pass filter 208 is electrically connected to an output of the photodetector 106). In some embodiments, the high-pass filter 208 is electrically coupled to the transimpedance amplifier 206 (e.g., an input of the high-pass filter 208 is electrically connected to an output of the transimpedance amplifier 206). In this circumstance, the high-pass filter 208 is configured to generate a respective filtered optical signal 238 by removing non-heart information from a converted optical signal 236 (e.g., converted optical signal 236-1, converted optical signal 236-2). In some embodiments, the high-pass filter 208 has a cutoff frequency in a range from 1 kHz to 10 kHz. In some embodiments, the cutoff frequency of the high-pass filter 208 is in the range of 1 kHz to 6 kHz (e.g., 5 kHz). In some embodiments, the high-pass filter 208 is configured to pass signals (e.g., a respective return optical signal or a respective converted optical signal) with frequency components above a predetermined cutoff frequency (e.g., 5 kHz). For example, in conjunction with the bandwidth (e.g., an upper bandwidth limit) set by the transimpedance amplifier 206, the cutoff frequency of the high-pass filter 208 may set a lower bound that defines an effective passband. In another example, the defined passband can be optimized to capture signals with a strong cardiac signature, while maintaining robustness against and reducing lower-frequency artifacts (e.g., motion-induced noise/signals). In some embodiments, the cutoff frequency for the high-pass filter 208 is selected based on the portion of the body of the user (e.g., based on a tissue type, a blood vessel size, and/or other characteristic of the portion of the body).

In some embodiments, the high-pass filter 208 is a second-order high-pass filter. For example, the high-pass filter 208 has an attenuation of 40 dB below a cutoff frequency. In some embodiments, the high-pass filter 208 has an order greater than two. In some embodiments, the high-pass filter 208 is a passive circuit comprising resistor components and capacitor components. In some embodiments, the high-pass filter is an active circuit comprising one or more amplifiers.

In some embodiments, a respective signal processing channel (e.g., signal processing channel 204-1 or signal processing channel 204-2) of the analog IC 109 comprises the rectifier 210 configured to generate a respective rectified optical signal (e.g., rectified optical signal 240-1 or rectified optical signal 240-2) by rectifying a respective filtered optical signal (e.g., filtered optical signal 238-1 or filtered optical signal 238-2). In some embodiments, the rectifier 210 is electrically coupled to the high-pass filter 208 (e.g., an input of the rectifier 210 is electrically connected to an output of the high-pass filter 208). In some embodiments, the rectifier 210 comprises a non-linear electrical component. In some embodiments, the non-linear electrical component of the rectifier 210 comprises a rectifier diode (e.g., the rectifier 210 is a diode-based rectifier). For example, the rectifier 210 may be configured to disrupt the symmetry of the respective filtered optical signal about a zero-voltage reference. In some embodiments, the rectifier 210 is a half-wave rectifier (e.g., configured to block a negative portion of the respective filtered optical signal). In some embodiments, the rectifier 210 is a full-wave rectifier (e.g., configured to maintain a positive portion of the respective filtered optical signal) configured to convert the negative portion of the filtered optical signal to be positive. For example, the rectifier 210 may be configured to rectify the respective filtered optical signal to produce an output signal (e.g., a respective rectified optical signal) varying between zero and a positive voltage level, such that the respective rectified optical signal encodes information representative of the time-varying magnitude or variance of the respective filtered optical signal (e.g., variance of the respective filtered optical signal over time). In some embodiments, the rectifier 210 has a bandwidth (e.g., 1 kHz to 25 kHz, 5 kHz to 25 kHz, or 10 kHz to 25 kHz) substantially similar to or greater than that of the respective filtered optical signal. As an example, the bandwidth of the respective filtered optical signal may include a passband determined by the transimpedance amplifier 206, the high-pass filter 208, or a combination thereof. In some embodiments, the rectifier 210 operates at a voltage that is no greater than 1 V.

In some embodiments, a respective signal processing channel (e.g., signal processing channel 204-1 or signal processing channel 204-2) of the analog IC 109 comprises the low-pass filter 212 configured to generate a respective time-averaged optical signal (e.g., time-averaged optical signal 242-1 or time-averaged optical signal 242-2) by removing high-frequency components of a respective rectified optical signal (e.g., rectified optical signal 240-1 or rectified optical signal 240-2). In some embodiments, the low-pass filter 212 is electrically coupled to the rectifier 210 (e.g., an input of the low-pass filter 212 is electrically connected to an output of the rectifier 210). In some embodiments, the low-pass filter 212 is configured to perform averaging in time domain by attenuating high-frequency (e.g., isolates power variations occurring over relatively long time scales associated with heartbeat). In some embodiments, the low-pass filter 212 has a cutoff frequency in the range of 7 Hz to 200 Hz (e.g., is 10 Hz). In some embodiments, the cutoff frequency of the low-pass filter 212 is determined to be no greater than 100 Hz or no greater than 200 Hz, which is sufficient to capture heart-induced vibrations (e.g., resulting from heartbeat).

In some embodiments, the low-pass filter 212 is a second-order low-pass filter. For example, the low-pass filter 212 may have an attenuation of 40 dB above a cutoff frequency. In some embodiments, the low-pass filter 212 has an order greater than two. In some embodiments, the low-pass filter 212 is a passive circuit comprising resistor components and capacitor components. In some embodiments, the low-pass filter 212 is an active circuit comprising one or more amplifiers.

In some embodiments, a respective signal processing channel (e.g., signal processing channel 204-1 or signal processing channel 204-2) of the analog IC 109 comprises the programmable gain amplifier 214 configured to generate a respective amplified optical signal (e.g., amplified optical signal 244-1 or amplified optical signal 244-2) by amplifying a respective time-averaged optical signal (e.g., time-averaged optical signal 242-1 or time-averaged optical signal 242-2). In some embodiments, the programmable gain amplifier 214 is electrically coupled to the low-pass filter 212 (e.g., an input of the programmable gain amplifier 214 is electrically connected to an output of the low-pass filter 212). In some embodiments, the programmable gain amplifier 214 is electrically coupled to the ADC 218 (e.g., an output of the programmable gain amplifier 214 is electrically connected to an input of the ADC 218). In some embodiments, the programmable gain amplifier 214 is configured such that the respective amplified optical signal is within an input voltage range of the ADC 218. For example, the programmable gain amplifier 214 may be configured to provide a gain for the respective amplified optical signal to match the input voltage range of the ADC 218. In some embodiments, the programmable gain amplifier 214 is electrically coupled to the ADC 218 via the analog multiplexer 216 (e.g., in a dual-channel configuration 130B, in a multi-channel configuration 130C, etc.).

In some embodiments, the sensing unit 202 includes the ADC 218 electrically coupled to the analog IC 109. In some embodiments, the ADC 218 is configured to digitize a respective amplified optical signal (e.g., amplified optical signal 244-1 or amplified optical signal 244-2) at a sampling rate (e.g., on the order of 100 Hz) exceeding the cutoff frequency of the low-pass filter 212. For example, given the bandwidth imposed by the low-pass filter 212, the ADC 218 may be required to support only a dynamic range and resolution that are sufficient to capture the pulsatility component of the respective amplified optical signal. In another example, a 10-bit resolution (e.g., a bit depth of 10 bits) may be sufficient to provide the required dynamic range and resolution. In some circumstances, design requirements (e.g., specifications) of the ADC 218 may be relaxed to allow a reduction in overall cost, size (e.g., form factor), and power consumption of the sensing unit 202.

In some embodiments, the respective amplified optical signal is a digital signal. In some embodiments, the sampling rate is at least 30 - 100 kHz in the ADC 218. In some embodiments, the sampling rate is 500 kHz - 1MHz in the ADC 218 so that the respective amplified optical signal is oversampled to reduce noise.

Although Figure 2 illustrates a specific dual-channel configuration for the sensing unit, the present disclosure is not limited to this particular implementation. In some embodiments, the sensing unit implements a single-channel configuration with one light emitter and one photodetector. In some embodiments, the sensing unit implements a multi-channel configuration with three or more photodetectors arranged at varying distances from the portion of the body. In some embodiments, the signal processing channels of the analog IC include components other than those illustrated, such as bandpass filters in place of separate high-pass and low-pass filters, full-wave rectifiers in place of half-wave rectifiers, or variable gain amplifiers with automatic gain control. In some embodiments, the high-pass filter has a cutoff frequency other than 5 kHz, such as cutoff frequencies in the range of 1 kHz to 10 kHz selected based on the target tissue type or blood vessel characteristics. In some embodiments, the low-pass filter has a cutoff frequency other than 10 Hz, such as cutoff frequencies in the range of 5 Hz to 200 Hz selected based on the physiological signal of interest. In some embodiments, the transimpedance amplifier has a bandwidth other than 25 kHz, such as bandwidths in the range of 10 kHz to 50 kHz. In some embodiments, the transimpedance amplifier has a gain other than 1×106 V/A, such as gains in the range of 1×105 V/A to 1×107 V/A. In some embodiments, the ADC has a sampling rate other than 100 Hz, such as sampling rates in the range of 50 Hz to 1 kHz. In some embodiments, the ADC has a bit depth other than 10 bits, such as bit depths of 8 bits, 12 bits, or 16 bits. In some embodiments, the analog multiplexer is omitted and each signal processing channel has a dedicated ADC. In some embodiments, the driver for the light emitter includes automatic power control (APC) circuitry. In some embodiments, the sensing unit components are implemented as discrete components on a printed circuit board rather than as an integrated circuit. In some embodiments, the control component communicates via interfaces other than I2C, such as SPI, UART, or other serial communication protocols. Accordingly, the specific configuration illustrated in Figure 2 is provided as an example and should not be construed as limiting the scope of the present disclosure, the invention being defined in the appended claims.

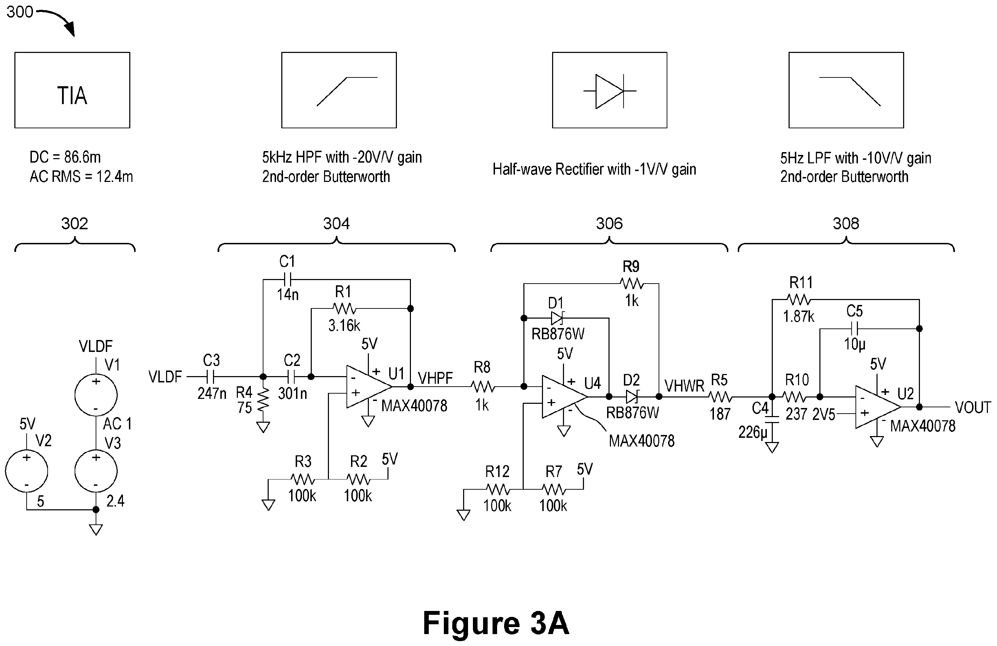

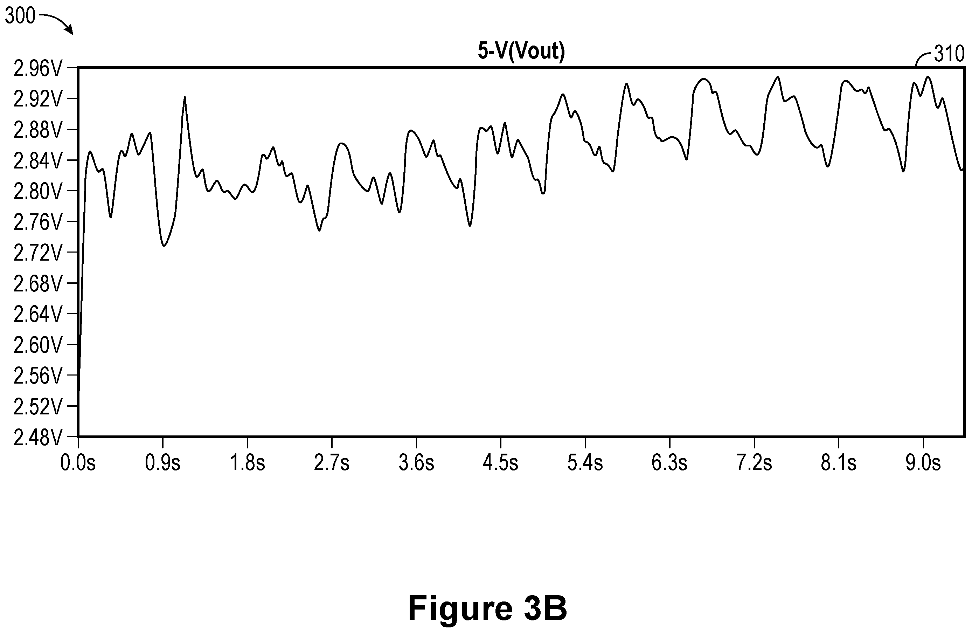

Figures 3A and 3B illustrate analog circuitry 300 corresponding to the analog IC 109, in accordance with some embodiments. In some embodiments, the analog circuitry 300 is arranged as a signal processing channel (e.g., signal processing channel 204-1 or signal processing channel 204-2 in Figure 2) of the analog IC 109. Turning to Figure 3A, the analog circuitry 300 comprises a transimpedance amplifier 302 (e.g., an instance of the transimpedance amplifier 206), a high-pass filter 304 (e.g., an instance of the high-pass filter 208), a rectifier 306 (e.g., an instance of the rectifier 210), and a low-pass filter 308 (e.g., an instance of the programmable gain amplifier 214). In some embodiments, the transimpedance amplifier 302, the high-pass filter 304, the rectifier 306, and the low-pass filter 308 are electrically connected in sequence (e.g., in a series connection). For example, the transimpedance amplifier 302 is configured to generate an output signal "VLDF," and the high-pass filter 304 is configured to generate, based on the output signal "VLDF," an output signal "VHPF" to the rectifier 306. In another example, the rectifier 306 is configured to generate, based on the output signal "VHPF," an output signal "VHWR" to the low-pass filter 308. In yet another example, the low-pass filter 308 is configured to generate, based on the output signal "VHWR," an output signal "VOUT" to subsequent circuitry (e.g., programmable gain amplifier, ADC, etc.). In some embodiments, the output signal "VLDF" corresponds to a converted optical signal (e.g., converted optical signal 236-1, converted optical signal 236-2). In some embodiments, the output signal "VHPF" corresponds to a filtered optical signal (e.g., filtered optical signal 238-1 or filtered optical signal 238-2). In some embodiments, the output signal "VHWR" corresponds to a rectified optical signal (e.g., rectified optical signal 240-1 or rectified optical signal 240-2). In some embodiments, the output signal "VOUT" corresponds to a time-averaged optical signal (e.g., time-averaged optical signal 242-1 or time-averaged optical signal 242-2).

In some embodiments, the transimpedance amplifier 302 is configured to generate the output signal "VLDF" with a direct current (DC) output voltage of 86.6 mV and an alternating current (AC) root-mean-square (RMS) voltage of 12.4 mV to the high-pass filter 304. In some embodiments, the DC output voltage of the output signal "VLDF" ranges from 50 mV to 4.5V. In some embodiments, the AC RMS voltage of the output signal "VLDF" ranges from 3 mV to 30 mV. In some embodiments, the high-pass filter 304 is a second-order Butterworth filter, e.g., with a cutoff frequency of 5 kHz and a gain of -20V/V. In some embodiments, the high-pass filter 304 is an active circuit comprising one or more amplifiers. In some embodiments, the rectifier 306 is a half-wave rectifier, e.g., with a gain of -1V/V (e.g., unity gain). In some embodiments, the rectifier 306 is an active circuit comprising one or more amplifiers. In some embodiments, the low-pass filter 308 is a second-order Butterworth filter, e.g., with a cutoff frequency of 5 Hz and a gain of -10V/V. In some embodiments, the low-pass filter 308 is an active circuit comprising one or more amplifiers.

Turning to Figure 3B, the analog circuitry 300 comprises an example waveform 310 of the output signal "VOUT" of the low-pass filter 308. For example, the waveform 310 of the output signal "VOUT" has a DC output voltage of about 2.85 V (e.g., in the range of 2.80 V to 2.88 V) and AC RMS voltage of about 1.2 V (e.g., in the range of 0.80 V to 1.8 V). In some embodiments, the output signal "VOUT" includes a heart rate signal for calculating heart rate. In some embodiments, a detected optical signal (e.g., detected optical signal 234-1 or detected optical signal 234-2) generated by the photodetector 106 is a photocurrent (e.g., a low-level photocurrent). For example, the photocurrent may range from 0.5 µA (e.g., corresponding to pure signal) to 4.5 µA (e.g., corresponding to pure signal and noise from ambient). In some embodiments, the photocurrent comprises a first component that includes useful biometric data (e.g., heart information) and a second component that includes uninformative data (e.g., non-heart information). For example, the first component within a photocurrent of 0.5 µA (e.g., pure signal without noise from ambient) may be in the range of 50 nA to 100 nA (e.g., 67 nA). In some embodiments, a converted optical signal (e.g., converted optical signal 236-1, converted optical signal 236-2, or output signal "VLDF") generated by the transimpedance amplifier 302 comprises a supply voltage component and a signal component. For example, the supply voltage component may range from 1 V to 5 V corresponding to an input voltage bias of 0.5 V, and the signal component may be in the range of 50 mV to 100 mV (e.g., 67 mV) corresponding to a transimpedance gain of 1 MΩ, e.g., 67 nA × 1 MΩ = 67 mV. In some embodiments, the high-pass filter 304 is configured to have a cutoff frequency of 5 kHz and a gain of -20V/V, yielding an output voltage (e.g., filtered optical signal 238-1, filtered optical signal 238-2, output signal "VHPF") in the range of 420 mV to 860 mV (e.g., is 570 mV) corresponding to an input voltage of 67 mV.

Although Figures 3A and 3B illustrate specific analog circuitry configurations for the analog IC, the present disclosure is not limited to these particular implementations. In some embodiments, the transimpedance amplifier has a DC output voltage other than 86.6 mV, such as DC output voltages in the range of 50 mV to 500 mV. In some embodiments, the transimpedance amplifier has an AC RMS voltage other than 12.4 mV, such as AC RMS voltages in the range of 3 mV to 50 mV. In some embodiments, the transimpedance amplifier comprises operational amplifiers other than the MAX40078, such as low-noise operational amplifiers, rail-to-rail operational amplifiers, or other amplifier types selected based on bandwidth, noise, or power consumption requirements. In some embodiments, the high-pass filter has an order other than second-order, such as first-order, third-order, or higher-order filters providing different roll-off characteristics. In some embodiments, the high-pass filter has a cutoff frequency other than 5 kHz, such as cutoff frequencies in the range of 1 kHz to 10 kHz. In some embodiments, the high-pass filter has a gain other than -20 V/V, such as gains in the range of -5 V/V to -50 V/V. In some embodiments, the high-pass filter comprises a topology other than Butterworth, such as Chebyshev, Bessel, or elliptic filter topologies. In some embodiments, the rectifier comprises a full-wave rectifier rather than a half-wave rectifier, providing improved signal utilization. In some embodiments, the rectifier has a gain other than -1 V/V, such as gains in the range of -0.5 V/V to -10 V/V. In some embodiments, the rectifier comprises precision rectifier configurations using operational amplifiers to reduce diode forward voltage drop effects. In some embodiments, the rectifier comprises diode types other than RB876W, such as Schottky diodes, silicon diodes, or other rectifier diodes selected based on forward voltage drop or switching speed. In some embodiments, the low-pass filter has a cutoff frequency other than 5 Hz, such as cutoff frequencies in the range of 1 Hz to 50 Hz. In some embodiments, the low-pass filter has a gain other than -10 V/V, such as gains in the range of -1 V/V to -100 V/V. In some embodiments, the resistor and capacitor values differ from those illustrated, with component values selected based on desired filter characteristics, noise performance, or power consumption. In some embodiments, the analog circuitry operates from supply voltages other than 5 V, such as 3.3 V, 1.8 V, or other supply voltages compatible with the wearable device power system. In some embodiments, the output voltage range differs from the illustrated range of 2.80 V to 2.88 V, with output ranges selected to match the input requirements of subsequent processing stages. In some embodiments, the signal processing chain includes additional stages such as notch filters for removing interference at specific frequencies or additional amplification stages. In some embodiments, the order of the signal processing stages differs from the illustrated sequence. Accordingly, the specific configurations illustrated in Figures 3A and 3B are provided as examples and should not be construed as limiting the scope of the present disclosure, the invention being defined in the appended claims.

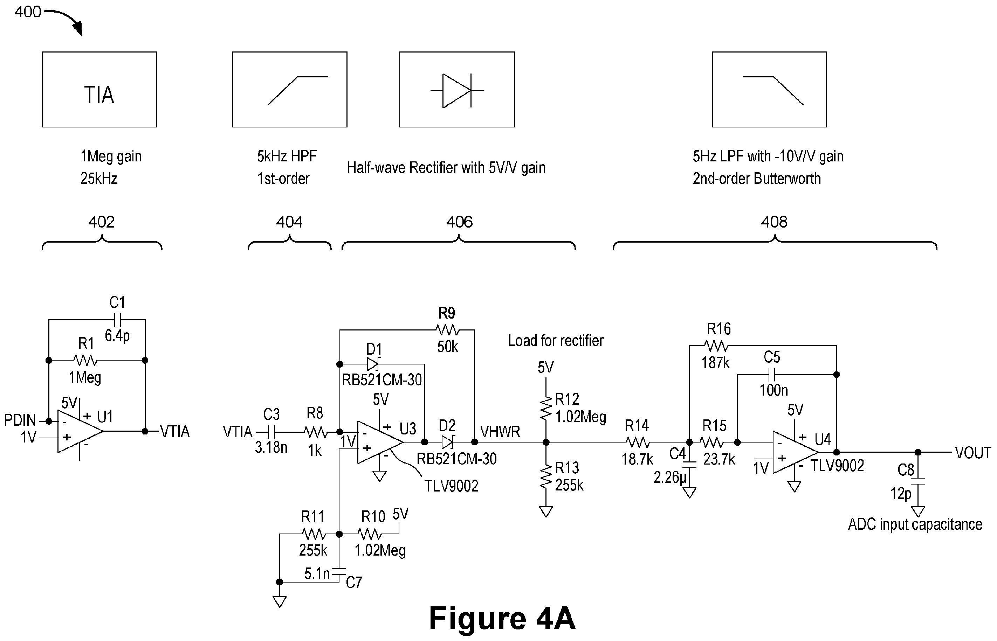

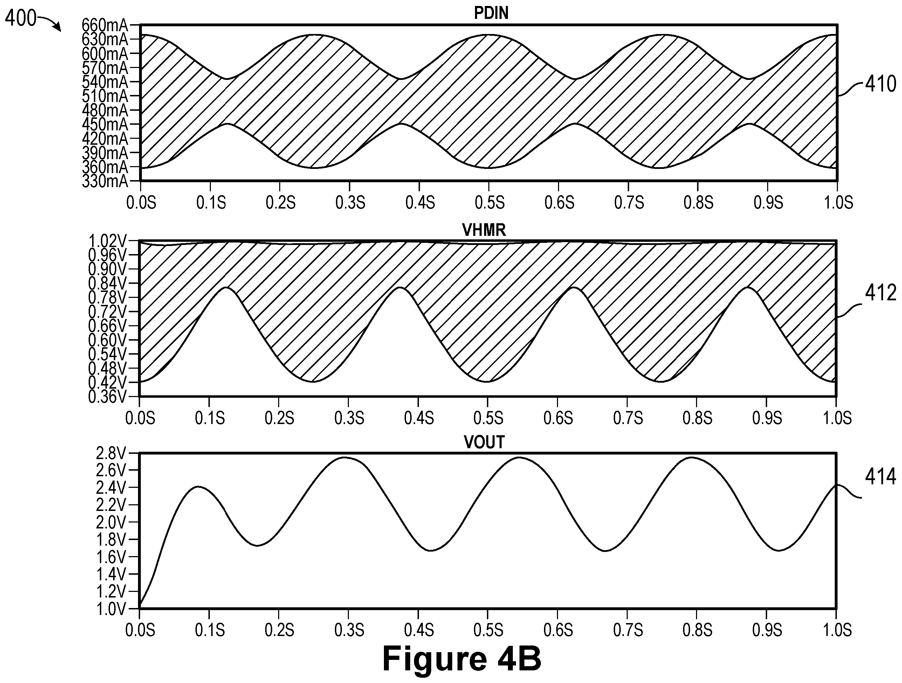

Figures 4A and 4B illustrate aspects of analog circuitry 400 corresponding to the analog IC 109, in accordance with some embodiments. In some embodiments, the analog circuitry 400 is configured as a signal processing channel (e.g., signal processing channel 204-1 or signal processing channel 204-2 in Figure 2) of the analog IC 109. In Figure 4A, the analog circuitry 400 comprises a transimpedance amplifier 402 (e.g., an instance of the transimpedance amplifier 206), a high-pass filter 404 (e.g., an instance of the high-pass filter 208), a rectifier 406 (e.g., an instance of the rectifier 210), and a low-pass filter 408 (e.g., an instance of the programmable gain amplifier 214). In some embodiments, the transimpedance amplifier 402, the high-pass filter 404, the rectifier 406, and the low-pass filter 408 are electrically connected in sequence. For example, the transimpedance amplifier 402 is configured to generate, based on an input signal "PDIN," an output signal "VTIA," and the high-pass filter 404 and the rectifier 406 are configured to generate, based on the output signal "VTIA," an output signal "VHWR" to the low-pass filter 408. In another example, the low-pass filter 408 is configured to generate, based on the output signal "VHWR," an output signal "VOUT" to subsequent circuitry (e.g., programmable gain amplifier, ADC, etc.). In some embodiments, the output signal "VTIA" corresponds to a converted optical signal (e.g., converted optical signal 236-1 or converted optical signal 236-2). In some embodiments, the output signal "VHWR" corresponds to a rectified optical signal (e.g., rectified optical signal 240-1 or rectified optical signal 240-2). In some embodiments, the output signal "VOUT" corresponds to a time-averaged optical signal (e.g., time-averaged optical signal 242-1 or time-averaged optical signal 242-2).

In some embodiments, the transimpedance amplifier 402 has a transimpedance gain of 1 MΩ and a bandwidth of 25 kHz. In some embodiments, the high-pass filter 404 is a first-order filter, e.g., with a cutoff frequency of 5 kHz. In some embodiments, in conjunction with the bandwidth (e.g., an upper bandwidth limit of 25 kHz) set by the transimpedance amplifier 402, the cutoff frequency (e.g., 5 kHz) of the high-pass filter 404 sets a lower bound that defines an effective passband (e.g., from 5 kHz to 25 kHz). In some embodiments, the rectifier 406 is a half-wave rectifier, e.g., with a gain of -5V/V. In some embodiments, the rectifier 406 is an active circuit comprising one or more amplifiers. In some embodiments, the low-pass filter 408 is a second-order Butterworth filter, e.g., with a cutoff frequency of 5 Hz and a gain of -10V/V. In some embodiments, the low-pass filter 408 is an active circuit comprising one or more amplifiers.

Turning to Figure 4B, example waveforms 410, 412, and 414 correspond to the input signal "PDIN," the output signal "VHWR," and the output signal "VOUT," respectively, in analog circuitry 400. For example, the waveform 410 of the input signal "PDIN" is a sinusoidal wave, and has a DC input current of 0.5 µA and an RMS-to-DC ratio of 0.54. In another example, the transimpedance amplifier 402, the high-pass filter, and the rectifier 406 may be configured to generate the waveform 412 of the output signal "VHWR" to the low-pass filter 408 based on the waveform 410 of the input signal "PDIN," In another example, the low-pass filter 408 is configured to generate, based on the waveform 412 of the output signal "VHWR," the waveform 414 of the output signal "VOUT." The output signal "VOUT" may have a peak-to-peak amplitude of 1.05 V.

In some embodiments, a total power consumption of the analog IC 109 (e.g., analog circuitry 300 or analog circuitry 400) is on the order of 1.0 mW. For example, the total power consumption may be in the range of 0.75 mW to 1.50 mW, based on a configuration with three amplifiers biased at 5 V and a total input current (e.g., "PDIN") of 200 µA (e.g., in reference to Figures 3A and 4A). In some embodiments, the total current consumption comprises approximately 198.15 µA, including a quiescent current of approximately 60 µA per operational amplifier for three operational amplifiers totaling approximately 180 µA, plus approximately 6.0 µA for biasing resistors. In some embodiments, the total power consumption is calculated as 5 V multiplied by 198.15 µA, yielding approximately 0.99 mW. In some embodiments, one or more amplifiers of the analog IC 109 operate at 3.3 V, thereby rendering a lower power consumption in the range of 0.50 mW to 0.8 mW (e.g., 0.65 mW).

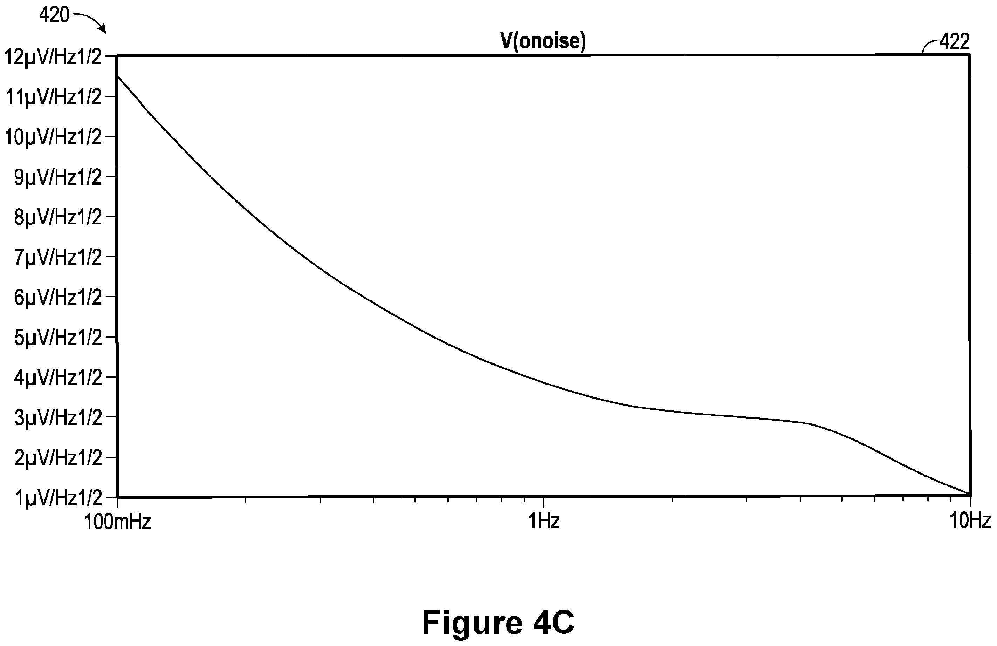

Figure 4C illustrates an example noise analysis 420 corresponding to the analog IC 109, in accordance with some embodiments. The noise analysis 420 includes an example noise spectrum 422 spanning a frequency range from 0.1 Hz to 10 Hz. In some embodiments, the analog IC 109 is shot-noise limited, e.g., noise floor of the analog IC 109 is dominated by shot noise. For example, an intrinsic shot noise (e.g., a fundamental limit) generated by the analog IC 109 may be 63.2 pA RMS at a photocurrent of 0.5 µA and 179 pA RMS at a photocurrent of 4.0 µA. In another example, the transimpedance amplifier 206 may generate an output noise of 45.2 µV RMS (e.g., equivalent to 45.2 pA RMS for a transimpedance gain of 1 MΩ) within a frequency range of 0.1 Hz to 25 Hz. The transimpedance amplifier 206 may generate an output noise of 9.11 µV RMS (e.g., corresponding to 182 fA RMS) within a frequency range of 0.1 Hz to 5 Hz. In yet another example, in real-time biometric data monitoring, a total noise generated by the analog IC 109 may be 10 nA RMS within a frequency range of 10 Hz to 100 kHz, 8.2 nA RMS within a frequency range of 5 Hz to 36 kHz, and/or 210 pA RMS within a frequency range of 0.02 Hz to 5 kHz.

Although Figures 4A-4C illustrate specific analog circuitry configurations for the analog IC, the present disclosure is not limited to these particular implementations. In some embodiments, the transimpedance amplifier has a gain other than 1 MΩ, such as gains in the range of 100 kΩ to 10 MΩ. In some embodiments, the transimpedance amplifier has a bandwidth other than 25 kHz, such as bandwidths in the range of 10 kHz to 100 kHz. In some embodiments, the high-pass filter has an order other than first-order, such as second-order, third-order, or higher-order filters providing steeper roll-off characteristics. In some embodiments, the high-pass filter has a cutoff frequency other than 5 kHz, such as cutoff frequencies in the range of 1 kHz to 15 kHz selected based on the frequency content of the blood flow signal. In some embodiments, the effective passband differs from the illustrated 5 kHz to 25 kHz range, with passbands selected based on the target tissue type, blood vessel characteristics, or motion artifact rejection requirements. In some embodiments, the rectifier comprises a full-wave rectifier rather than a half-wave rectifier, providing improved signal utilization and reduced ripple. In some embodiments, the rectifier has a gain other than -5 V/V, such as gains in the range of -1 V/V to -20 V/V. In some embodiments, the rectifier comprises diode types other than RB521CM-30, such as Schottky diodes, silicon diodes, or integrated rectifier circuits. In some embodiments, the low-pass filter has an order other than second-order Butterworth, such as first-order, third-order, or filters with Chebyshev or Bessel characteristics. In some embodiments, the low-pass filter has a cutoff frequency other than 5 Hz, such as cutoff frequencies in the range of 1 Hz to 100 Hz. In some embodiments, the low-pass filter has a gain other than -10 V/V, such as gains in the range of -1 V/V to -50 V/V. In some embodiments, the input signal has a DC input current other than 0.5 µA, such as DC input currents in the range of 0.1 µA to 10 µA. In some embodiments, the input signal has an RMS-to-DC ratio other than 0.54, such as ratios in the range of 0.1 to 1.0 depending on the modulation depth of the blood flow signal. In some embodiments, the output signal has a peak-to-peak amplitude other than 1.05 V, such as amplitudes in the range of 0.1 V to 5 V selected to match the input range of subsequent processing stages. In some embodiments, the total power consumption differs from the illustrated range of 0.75 mW to 1.50 mW, with power consumption optimized based on battery life requirements or thermal constraints. In some embodiments, the analog circuitry operates from supply voltages other than 5 V or 3.3 V, such as 1.8 V or other supply voltages compatible with the wearable device power system. In some embodiments, the noise performance differs from the illustrated shot-noise limited behavior, with noise optimization techniques applied based on the target signal-to-noise ratio. In some embodiments, the analog circuitry supports pulsed-mode operation with duty cycles ranging from 1% to 100%. In some embodiments, the ADC input capacitance differs from the illustrated 12 pF, with capacitance values selected based on the ADC architecture and sampling rate. Accordingly, the specific configurations illustrated in Figures 4A-4C are provided as examples and should not be construed as limiting the scope of the present disclosure, the invention being defined in the appended claims.

Figure 5A illustrates an example processing flow for using LDF for don/doff condition detection, in accordance with some embodiments. As described previously, the wearable device 102 projects, from the light emitter 104, an outputted optical signal 110 to a portion of a body 120 of a user 101, and captures, by the photodetector 106, a return optical signal 112 corresponding to the outputted optical signal 110. The processing flow depicted in Figure 5A shows how the return optical signal 112 may be processed through three parallel processing paths to determine a wear state of the wearable device 102.

Referring to Figure 5A, an input signal comprising a time-varying waveform is fed through three parallel processing paths, each representing a different preprocessing approach before classification. In a first processing path, the input signal passes through a lowpass filter, which produces a one-dimensional time series output having a length of n samples along a time axis. The lowpass filtered signal is then provided to a convolutional neural network (CNN), which generates a prediction output. The first processing path represents an implementation of a reflection strength approach where a DC or low-frequency content of the signal is preserved.

With continued reference to Figure 5A, in a second processing path, the input signal passes through a bandpass filter, which similarly produces a one-dimensional time series output having a length of n samples along the time axis. The bandpass filtered signal is then provided to a CNN, which generates a prediction output. The second processing path provides a comparison signal where a DC signal level is removed but dynamics of the signal are preserved. The bandpass filtering removes the DC component while retaining signal variations that may indicate the don/doff condition.

In a third processing path shown in Figure 5A, the input signal passes through a time-frequency transform to generate a two-dimensional representation. In some embodiments, the time-frequency transform comprises a short-time Fourier transform (STFT). The two-dimensional representation comprises a plurality of frequency bins over a plurality of time samples, creating a spectrogram-like output having dimensions of n samples along the time axis and m frequency bins along a frequency axis. The two-dimensional representation is then provided to a CNN, which generates a prediction output. The third processing path captures both a spectral power distribution arising from blood flow and a temporal pulsatility arising from a heartbeat. In some embodiments, processing the two-dimensional representation comprises providing the two-dimensional representation as input to a classifier configured to perform binary classification between a donned state and a doffed state.

In some embodiments, the wear state detection uses a shallow CNN to perform binary classification between donned and doffed states. The CNN may receive inputs from any of the three processing paths, with the architecture of an input layer adjusted depending on the signal dimensionality. In some embodiments, the CNN is used to probe information content of the signals, and in deployed implementations, lighter-weight classifiers may be used in place of the CNN, such as threshold-based classifiers, rule-based classifiers, or other computationally efficient models that may be implemented on the module itself. In some embodiments, the classifier is trained and validated using a leave-one-subject-out (LOSO) approach to mitigate overfitting, where performance is assessed on an unseen subject. For the first and second processing paths, the CNN receives one-dimensional time series inputs. For the third processing path, the CNN receives the two-dimensional representation as input.

In some embodiments, the wear state detection processes a two-dimensional input structure with frequency bins and time samples to capture both spectral power distribution and temporal pulsatility. The two-dimensional input structure enables the classifier to leverage both frequency-domain information indicative of blood flow characteristics and time-domain information indicative of heartbeat pulsatility. When only one time point is considered (n=1), the third processing path is equivalent to a flow spectrum technique that relies on spectral power distribution. When only one frequency bin is considered (m=1), the third processing path is equivalent to a heartbeat technique that relies on temporal pulsatility. The combination of multiple frequency bins and multiple time samples enables the classifier to capture both types of information simultaneously.

In some embodiments, the three processing paths enable comparison of different signal representations for determining the wear state of the wearable device 102. The time-frequency transform approach of the third processing path captures both spectral and temporal characteristics that may be used to distinguish between donned and doffed conditions based on a presence of perfused skin. Blood flow in perfused skin results in a broad power spectrum in the return optical signal 112, in contrast to reflections from other materials which have power concentrated in a narrower band near DC.

In some embodiments, a non-transitory computer-readable storage medium stores instructions that, when executed by one or more processors of the wearable device 102, cause the wearable device 102 to perform operations comprising: projecting, from the light emitter 104, the outputted optical signal 110 to the portion of the body 120 of the user 101; capturing, by the photodetector 106, the return optical signal 112 corresponding to the outputted optical signal 110; performing the time-frequency transform on the return optical signal 112 to generate the two-dimensional representation comprising the plurality of frequency bins over the plurality of time samples; processing the two-dimensional representation to capture both the spectral power distribution arising from blood flow and the temporal pulsatility arising from the heartbeat; and determining, based on the processed two-dimensional representation, the wear state of the wearable device 102.

Although Figure 5A illustrates a specific processing flow with three parallel paths for don/doff condition detection, the present disclosure is not limited to this particular implementation. In some embodiments, the processing flow includes fewer than three parallel paths, such as a single processing path or two parallel paths. In some embodiments, the processing flow includes more than three parallel paths, such as four, five, or more parallel paths providing additional signal representations. In some embodiments, the preprocessing approaches differ from those illustrated, such as wavelet transforms, mel-frequency cepstral coefficients, power spectral density estimation, or other spectral analysis techniques in place of or in addition to lowpass filtering, bandpass filtering, or short-time Fourier transforms. In some embodiments, the time-frequency transform comprises a continuous wavelet transform, a discrete wavelet transform, a Wigner-Ville distribution, or other time-frequency representations rather than a short-time Fourier transform. In some embodiments, the classifier comprises a support vector machine, a random forest, a decision tree, a logistic regression model, a recurrent neural network, a threshold-based classifier, or a rule-based classifier rather than a convolutional neural network. In some embodiments, the input dimensionality differs from the illustrated one-dimensional or two-dimensional representations, such as three-dimensional representations incorporating additional signal characteristics. In some embodiments, the number of frequency bins differs from the illustrated values, such as 2, 3, 4, 5, 6, 8, 10, or more frequency bins selected based on the desired frequency resolution. In some embodiments, the number of time samples differs from the illustrated values, such as window lengths of 2, 4, 8, 16, 32, 64, or more samples selected based on the desired temporal resolution and latency requirements. In some embodiments, results from multiple processing paths are combined using voting, averaging, weighted combination, or other fusion techniques rather than being evaluated independently. In some embodiments, the classifier is trained using cross-validation, hold-out validation, or other training methodologies rather than leave-one-subject-out validation. In some embodiments, the wear state classification comprises multi-class classification including partially donned states, transitioning states, or other intermediate states rather than binary classification between donned and doffed states. In some embodiments, the input signal comprises multiple LDF channels, multiple wavelengths, or signals from multiple sensors rather than a single LDF channel. Accordingly, the specific processing flow illustrated in Figure 5A is provided as an example and should not be construed as limiting the scope of the present disclosure, the invention being defined in the appended claims.

Figure 5B illustrates example receiver operating characteristic (ROC) curves comparing detection performance of different techniques for don/doff condition detection, in accordance with some embodiments. The ROC curves depicted in Figure 5B plot a true doff rate on a vertical axis against a false doff rate on a horizontal axis, with both axes ranging from 0 to 1. A diagonal dashed line extending from an origin to an upper right corner of the graph represents chance-level performance for reference.

Referring to Figure 5B, three ROC curves are displayed corresponding to different detection techniques. A first curve labeled "DC, Unnormalized" represents a reflection strength technique and demonstrates highest performance among the three techniques. The first curve rises steeply near the origin and maintains a true doff rate near 1.0 across most false doff rate values. The reflection strength technique performs best due to a large step change in signal based on whether the wearable device 102 is worn. In some embodiments, the don/doff detection using the reflection strength technique has a latency of less than 10 milliseconds.

With continued reference to Figure 5B, a second curve labeled "DC, Normalized" represents a bandpassed case where a DC level is removed but signal dynamics are preserved. The second curve shows moderate performance between the other two curves. A third curve labeled "ARV, Normalized" represents a heartbeat technique using average rectified value processing. The third curve shows performance that modestly outperforms the normalized DC case, particularly when tuned for low false doff rates. The heartbeat technique using flow signal information uniquely enabled by LDF may encode more useful information than a simple reflection signal when the DC level is removed. In some embodiments, the don/doff detection using the heartbeat technique has a latency of approximately 2 seconds corresponding to capture of one or more cardiac cycles.

In some embodiments, the wearable device 102 quantifies power of the return optical signal 112 within a plurality of frequency bands to generate a plurality of power measurements. For example, the wearable device 102 determines, based on the plurality of power measurements, whether the return optical signal 112 exhibits a spectral distribution characteristic of perfused skin. The wearable device 102 further determines, based on whether the return optical signal 112 exhibits the spectral distribution characteristic of perfused skin, a wear state of the wearable device 102 indicating whether the wearable device 102 is donned or doffed. In some embodiments, the spectral distribution characteristic of perfused skin comprises a 1/f frequency dependence. Blood flow in perfused skin results in a broad power spectrum with the 1/f frequency dependence, in contrast to reflections from other materials which have power concentrated in a narrower band near DC.

In some embodiments, determining whether the return optical signal 112 exhibits the spectral distribution characteristic of perfused skin comprises computing a power ratio between two or more frequency bands of the plurality of frequency bands. In some embodiments, determining the wear state comprises comparing the power ratio to a threshold to discriminate between perfused skin and other materials. The threshold-based comparison enables the wearable device 102 to distinguish between skin of the user 101 and other surfaces such as foam, fabric, or a hat.

In some embodiments, the don/doff detection using a flow spectrum technique has a latency of approximately 100 milliseconds due to filter adjustment time. The flow spectrum technique quantifies power across multiple frequency bands to detect the spectral distribution characteristic of perfused skin. The latency of approximately 100 milliseconds represents a balance between the faster reflection strength technique and the slower heartbeat technique, while providing improved specificity compared to the reflection strength technique alone.

Although Figure 5B illustrates specific ROC curves for three detection techniques, the present disclosure is not limited to these particular implementations. For example, additional detection techniques may be employed, such as combinations of the reflection strength, flow spectrum, and heartbeat techniques. In some embodiments, the ROC curves exhibit different characteristics based on the specific implementation, training data, or operating conditions. In some embodiments, the latency values differ from those illustrated based on the sampling rate, filter characteristics, or processing architecture. In some embodiments, the threshold for discriminating between perfused skin and other materials is adaptive based on environmental conditions, user characteristics, or historical data. Accordingly, the specific ROC curves illustrated in Figure 5B are provided as examples and should not be construed as limiting the scope of the present disclosure, the invention being defined in the appended claims.

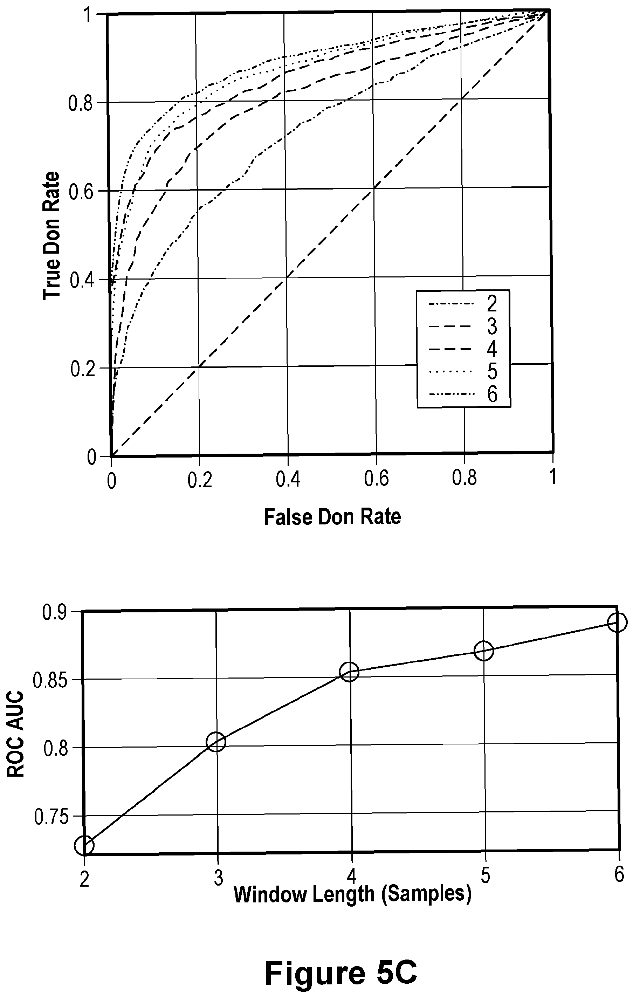

Figure 5C illustrates example ROC curves and corresponding area under the curve (AUC) values based on varying numbers of frequency bins, in accordance with some embodiments. The top portion of Figure 5C displays a graph showing ROC curves plotting a true don rate on a vertical axis against a false don rate on a horizontal axis. Five ROC curves are shown corresponding to different numbers of frequency bins, with the curves labeled 2, 3, 4, 5, and 6 in a legend. A dashed diagonal line represents performance of a random classifier. The ROC curves demonstrate that increasing the number of frequency bins improves classifier performance, with the curve corresponding to 6 frequency bins positioned closest to an upper left corner of the graph, indicating superior discrimination between donned and doffed states.

With continued reference to Figure 5C, the bottom portion of the figure displays a graph showing ROC AUC values on a vertical axis plotted against the number of frequency bins on a horizontal axis, ranging from 2 to 6. The curve shows that AUC increases from approximately 0.73 at 2 frequency bins to approximately 0.89 at 6 frequency bins. The improvement in AUC with additional frequency bins demonstrates the power of the flow spectrum technique, where a power distribution over frequency is adequately unique to have power to determine don/doff state. In some embodiments, quantifying power within each frequency band of the plurality of frequency bands comprises computing an average rectified value (ARV) for the frequency band. The ARV computation provides a measure of signal energy within each frequency band that may be used to characterize the spectral distribution of the return optical signal.

In some embodiments, determining whether the return optical signal exhibits the spectral distribution characteristic of perfused skin comprises fitting a curve to the plurality of power measurements and using one or more parameters of the fitted curve to classify the wear state. When more than two frequency bands are used, a curve may be fit to the power measurements from the plurality of frequency bands, and parameters of the fitted curve may be used to classify the wear state. For example, a slope parameter or an intercept parameter of the fitted curve may indicate whether the spectral distribution exhibits the 1/f frequency dependence characteristic of perfused skin. The improvement in classification performance with increasing numbers of frequency bins, as shown in Figure 5C, supports the use of curve fitting approaches that leverage multiple frequency measurements to characterize the spectral distribution.

In some embodiments, determining the wear state has a latency of approximately 100 milliseconds. The latency of approximately 100 milliseconds arises from filter adjustment time when implementing the flow spectrum technique. The flow spectrum technique provides a balance between the faster reflection strength technique and the slower heartbeat technique while providing improved specificity for distinguishing perfused skin from other materials.

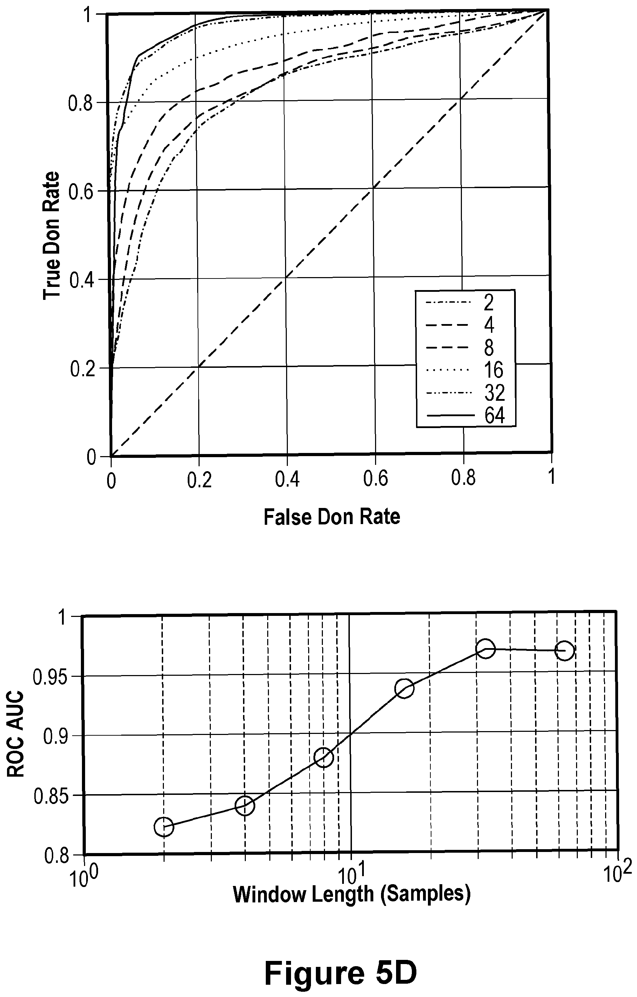

Figure 5D illustrates example ROC curves and ROC AUC analysis based on varying window lengths, in accordance with some embodiments. The top portion of Figure 5D displays a set of ROC curves plotting a true detection rate on a vertical axis against a false detection rate on a horizontal axis. Multiple curves are shown corresponding to different window lengths of 2, 4, 8, 16, 32, and 64 samples, with each curve represented by a different color. A diagonal dashed line represents chance-level performance. The curves demonstrate that longer window lengths achieve higher true detection rates for a given false detection rate, with the curves progressively approaching an upper left corner of the plot as window length increases.

With continued reference to Figure 5D, the bottom portion of the figure displays a graph plotting ROC AUC on a vertical axis against window length in samples on a horizontal axis, where the horizontal axis uses a logarithmic scale ranging from 10 to the power of 0 to 10 to the power of 2. The graph shows that ROC AUC increases from approximately 0.82 at a window length of approximately 1 sample to approximately 0.97 at window lengths of 32 and 64 samples. The curve exhibits a steep increase between window lengths of approximately 10 and 30 samples before plateauing at higher window lengths. The improvement in performance with longer windows is consistent with the heartbeat detection principle, as longer windows enable capture of one or more cardiac cycles that provide evidence of a heartbeat signal.

In some embodiments, a non-transitory computer-readable storage medium stores instructions that, when executed by one or more processors of the wearable device, cause the wearable device to perform operations comprising generating a plurality of time series from different frequency bins of the plurality of frequency bins. The wear state is determined based on the plurality of time series. By generating time series from different frequency bins, the wearable device captures both spectral power distribution information and temporal pulsatility information. The combination of multiple frequency bins and multiple time samples, as demonstrated by the sensitivity analyses in Figures 5C and 5D, enables improved classification accuracy for don/doff detection compared to approaches that consider only a single frequency bin or a single time sample.

Although Figures 5C and 5D illustrate specific sensitivity analyses for frequency bins and window length, the present disclosure is not limited to these particular implementations. In some embodiments, the number of frequency bins may range from 2 to 10 or more, with the specific number selected based on computational constraints, desired accuracy, or latency requirements. In some embodiments, the window length may range from 1 sample to 128 samples or more, with the specific length selected based on the sampling rate, desired latency, or cardiac cycle duration. In some embodiments, the relationship between the number of frequency bins or window length and classification performance may differ based on the classifier architecture, training data, or operating conditions. Accordingly, the specific sensitivity analyses illustrated in Figures 5C and 5D are provided as examples and should not be construed as limiting the scope of the present disclosure, the invention being defined in the appended claims.

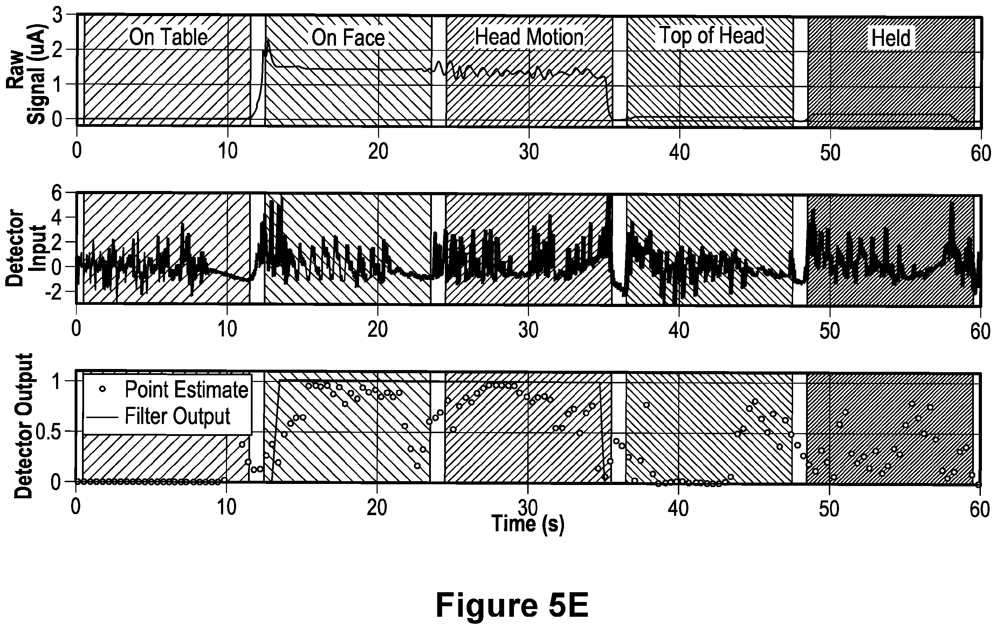

Figure 5E illustrates example detector curves for don/doff condition detection using LDF, in accordance with some embodiments. The figure comprises three vertically stacked graphs sharing a common time axis spanning from 0 to 60 seconds. The graphs are divided into five labeled regions corresponding to different wear states: On Table, On Face, Head Motion, Top of Head, and Held, with each region distinguished by different background shading.

Referring to Figure 5E, a top graph displays a raw signal measured in microamperes plotted against time. The raw signal remains near zero during an On Table period, rises sharply to approximately 2 microamperes when transitioning to an On Face state, maintains an elevated level with fluctuations during the On Face and Head Motion periods, drops to a lower level around 0.5 microamperes during a Top of Head period, and returns to near zero during a Held period. The raw signal demonstrates distinct amplitude characteristics for each wear state, with the On Face and Head Motion states exhibiting higher signal levels compared to the On Table, Top of Head, and Held states.

With continued reference to Figure 5E, a middle graph displays a detector input signal plotted against time. The detector input signal oscillates around zero during the On Table period, exhibits increased amplitude fluctuations ranging approximately between negative 2 and positive 6 during the On Face, Head Motion, Top of Head, and Held periods, with varying degrees of signal variation corresponding to the different wear states. The detector input signal captures dynamics of the return optical signal after preprocessing, providing input to a classifier for wear state determination.

A bottom graph in Figure 5E displays a detector output plotted against time, showing both point estimates represented as scattered dots and a filter output represented as a continuous line. The point estimates appear as individual data points scattered across the graph, while the filter output line provides a smoothed representation of the detector output. During the On Table period, both the point estimates and filter output remain near zero. The filter output rises toward 1 during the On Face period, maintains elevated values with some variation during the Head Motion period, and shows fluctuating behavior during the Top of Head and Held periods. The point estimates demonstrate variability around the filter output line throughout the different wear states.

In some embodiments, the don/doff detection distinguishes between true donned conditions and true doffed conditions. The true donned conditions include an on-face state and a head-motion state, where the wearable device 102 is positioned on a face of the user in a manner intended for use. The true doffed conditions include a top-of-head state, a held-by-bridge state, and a table position, where the wearable device 102 is not positioned for intended use. As shown in Figure 5E, the detector output exhibits higher values during the On Face and Head Motion periods compared to the On Table, Top of Head, and Held periods, demonstrating the ability of the detector to distinguish between donned and doffed conditions in real-time.

In some embodiments, the don/doff detection is evaluated using a data collection protocol comprising a plurality of wear states presented in a defined sequence. The protocol may include five states: a table state in which the wearable device 102 is placed on a surface, a worn-still state in which the wearable device 102 is worn on the face of the user while the user remains stationary, a worn-moving state in which the wearable device 102 is worn on the face of the user while the user is in motion (e.g., head motion), a top-of-head state in which the wearable device 102 is positioned on a top of the head of the user, and a held-by-bridge state in which the wearable device 102 is held by a bridge of the wearable device 102. Each state may have a defined duration, such as on the order of 10 seconds (e.g., 12 seconds). The sequence of states may be repeated a plurality of times (e.g., six times) during a data collection session. The worn-still state and the worn-moving state may correspond to true donned conditions, while the table state, the top-of-head state, and the held-by-bridge state may correspond to true doffed conditions. The doffed conditions may be selected to include scenarios that present challenges for conventional detection techniques, such as the top-of-head state which presents a risk of false don events for capacitive sensing approaches, and the held-by-bridge state which presents a risk of false don events for optical proximity sensing approaches due to skin being in close proximity to the sensor.

In some embodiments, the wear state is used to distinguish skin of the user from other materials including at least one of foam, fabric, or a hat. As described previously, blood flow in the portion of the body results in a broad power spectrum in the return optical signal, in contrast to reflections from other materials which have power concentrated in a narrower band near DC. The detector curves shown in Figure 5E demonstrate that the LDF-based detection can distinguish between the skin of the user during the On Face and Head Motion states and other surfaces encountered during the Top of Head and Held states.

In some embodiments, the wearable device 102 comprises an extended-reality headset, and determining the wear state reduces false don events when the extended-reality headset is placed on top of a hat or held by a temple arm. The Top of Head state shown in Figure 5E represents a scenario where a user places the extended-reality headset on top of a brim of a hat when not worn. The Held state represents a scenario where the extended-reality headset is held by a temple arm, which can cause false don events with conventional capacitive proximity sensing techniques. As shown in Figure 5E, the detector output during the Top of Head and Held periods remains lower than during the On Face and Head Motion periods, enabling the wearable device 102 to correctly identify these as doffed conditions and reduce false don events.

In some embodiments, the plurality of power measurements are determined in accordance with principles of LDF, where blood flow in the portion of the body results in a broad power spectrum in the return optical signal. The broad power spectrum arising from blood flow provides a spectral signature that distinguishes perfused skin from other materials. The detector curves in Figure 5E demonstrate that the LDF-based approach can leverage this spectral signature to accurately classify wear states across the five conditions tested.

In some embodiments, the method further comprises detecting a doff condition when the wearable device 102 is positioned on a top of a head of the user or held by a bridge of the wearable device 102. As shown in Figure 5E, the detector output during the Top of Head period and the Held period exhibits values that are distinguishable from the On Face and Head Motion periods. The Top of Head state corresponds to the wearable device 102 being positioned on the top of the head of the user, such as when placed on a hat brim. The Held state corresponds to the wearable device 102 being held by a bridge or temple arm of the wearable device 102. The detector correctly identifies both of these states as doff conditions, as indicated by the lower detector output values compared to the true donned conditions.

Although Figure 5E illustrates specific detector curves for five wear states, the present disclosure is not limited to these particular implementations. In some embodiments, additional wear states may be evaluated, such as partially donned states, transitioning states, or states where the wearable device 102 is worn on other portions of the body. In some embodiments, the detector output may be processed using different filtering approaches, such as Kalman filtering, exponential smoothing, or other temporal filtering techniques. In some embodiments, the threshold for distinguishing between donned and doffed conditions may be adaptive based on user characteristics, environmental conditions, or historical data. In some embodiments, the detector may output a confidence value rather than a binary classification, enabling downstream processing to make decisions based on the confidence level. Accordingly, the specific detector curves illustrated in Figure 5E are provided as examples and should not be construed as limiting the scope of the present disclosure, the invention being defined in the appended claims.

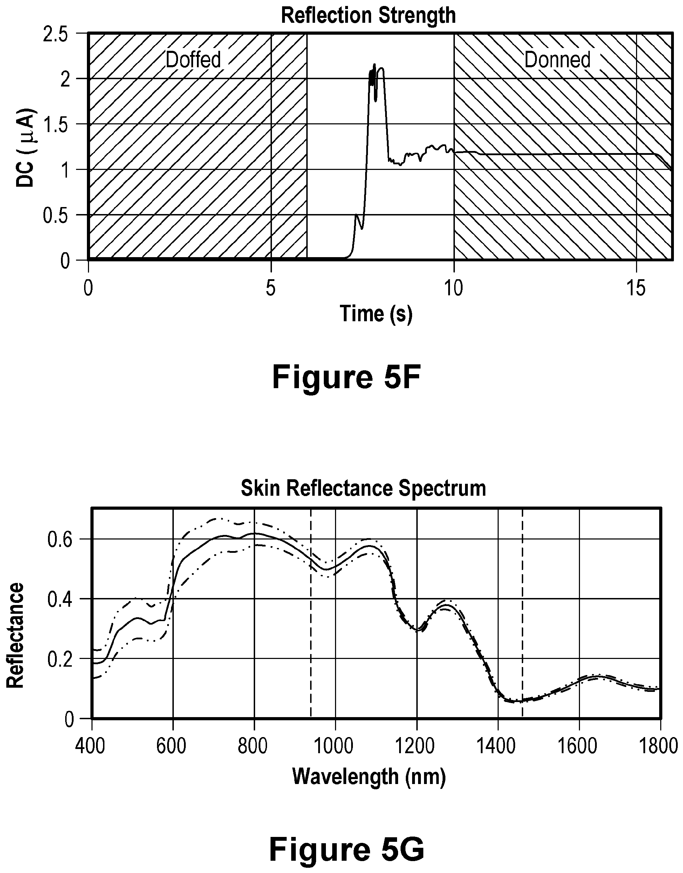

Figure 5F illustrates example reflection strength curves, in accordance with some embodiments. Reflection strength uses the strength of coupling between a source and a detector as a proxy for a distance to a target. The measurements depicted in Figure 5F plot a DC signal (measured between a range of 0 to 2.5 µA) over time (e.g., 0 to 15 seconds). During the doffed periods, the DC signal remains at a baseline level. When the wearable device 102 transitions from a doffed state to a donned state, the reflection strength exhibits a sharp spike, followed by fluctuations at an elevated level, demonstrating the change in reflection strength when the wearable device 102 transitions from the doffed state to the donned state on skin. While donned, the wearable device 102 maintains an elevated reflection strength level. In some embodiments, the don/doff detection using the reflection strength technique has a latency of less than 10 milliseconds.

Figure 5G illustrates example skin reflectance spectrums, in accordance with some embodiments. Skin reflectance refers to measuring the relative strength of reflections at different wavelengths using two or more sources, which can discriminate between skin and other surfaces. The reflection spectrum technique provides a method to improve specificity compared to the reflection strength technique alone. In some embodiments, the reflectance level is measured over a wavelength (e.g., from 400 to 1800 nm). Figure 5G displays a graph showing multiple curves representing different skin reflectance measurements, which demonstrate a reflectance strength (e.g., from 0 to 0.6) of reflections. The curves show varying reflectance values across the visible and near-infrared spectrum, with features including a rise in reflectance from approximately 0.2 at 400 nm to peak values around 0.5 to 0.6 in the 600 to 800 nm range, followed by variations in the near-infrared region with decreases in reflectance around 1400 nm corresponding to water absorption bands. Two vertical dashed lines are positioned at wavelength regions of interest. The spectral characteristics demonstrate how different wavelengths can be used to distinguish skin from other materials for don/doff and liveness detection applications. In some embodiments, the reflection spectrum technique has a latency of less than 10 milliseconds.

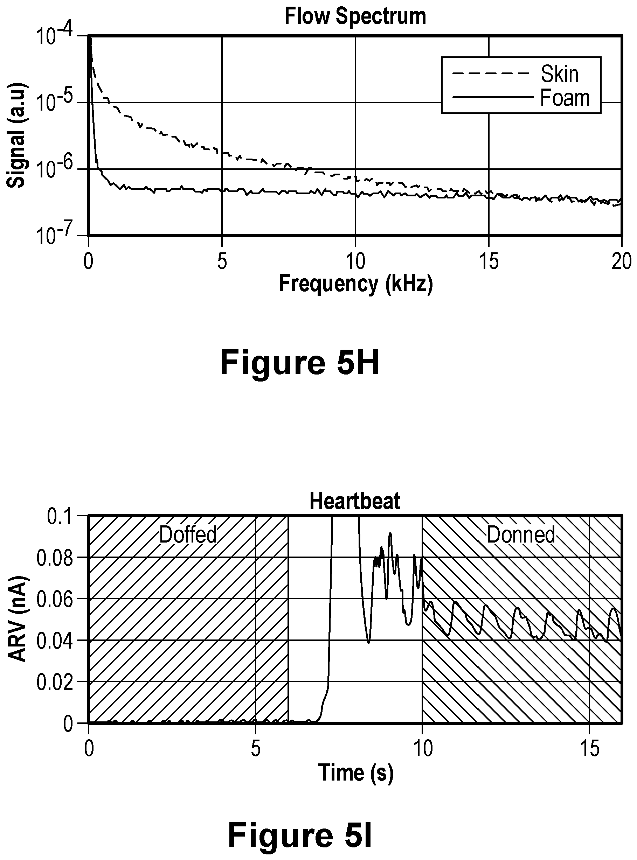

Figure 5H illustrates example flow spectrums, in accordance with some embodiments. The flow spectrum method is enabled by LDF. Blood flow results in a broad power spectrum in the LDF signal. In some embodiments, the LDF signal has a 1/f frequency dependence. In some embodiments, reflections from other materials have power concentrated in a narrower band (e.g., near DC). Figure 5H illustrates graphing the signal (e.g., from 10-7 to 10-4 a.u) and the frequency (from 0 to 20 kHz). The graph contains a solid line denoting the skin frequency based on the signal, and a dotted line denoting foam frequency based on the signal, demonstrating that the skin of the user can be distinguished from other materials. The presence of the broad spectrum with a characteristic frequency dependence is evidence that the system (e.g., the wearable device 102) is observing perfused skin, which does not require the blood flow to be pulsatile. In some embodiments, the flow spectrum technique has a latency of approximately 100 milliseconds due to filter adjustment time.

Figure 5I illustrates example heartbeat measurements, in accordance with some embodiments. The presence of a heartbeat in a processed LDF signal provides strong evidence that the sensor is targeting (e.g., placed on) the skin of a live person. Figure 5I displays a graph showing ARV in nanoamperes on the vertical axis over time in seconds on the horizontal axis, with doffed and donned regions indicated. During the doffed state, the ARV signal remains at a low value. Upon transition to the donned state, the signal shows clear periodic oscillations characteristic of a heartbeat waveform, with peaks and valleys demonstrating the presence of pulsatile blood flow when the wearable device 102 is worn on the skin. Measuring heartbeats requires sampling one or more cardiac cycles over several seconds and provides a high-confidence signal. However, in some embodiments, the heartbeat technique cannot discriminate between different areas on the body. In some embodiments, the heartbeat technique has a latency of approximately 2 seconds to capture one or more cardiac cycles.

Although Figures 5F to 5I illustrate specific measurements for various LDF sensing techniques, the present disclosure is not limited to these particular implementations. In some embodiments, the DC measurement may range from 0 to 2.5 mA or more, with the specific measurement number selected based on computational constraints, desired accuracy, or latency requirements. In some embodiments, the reflectance may range from 0 to 1 or more, with the specific values selected based on the sampling rate, wavelengths, or latency requirements. In some embodiments, the signal may range from 10⁻⁷ to 10⁻⁴ a.u or more, and frequency values for measuring flow spectrum may range from 0 to 20 kHz or more, with the specific values selected based on filters or desired latency. In some embodiments, the heartbeat measurement may range from 0 to 0.1 nA or more, based on the amount of time to measure, the amount of cardiac cycles to capture, or any latency requirements. Accordingly, the specific sensitivity analyses illustrated in Figures 5F to 5I are provided as examples and should not be construed as limiting the scope of the present disclosure, the invention being defined in the appended claims.

In some embodiments, the wearable device 102 comprises processing circuitry configured to process the return optical signal to detect a presence or absence of a heartbeat signal. The processing circuitry is further configured to determine, based on the presence or absence of the heartbeat signal, whether the wearable device 102 is targeting skin of a live person. The processing circuitry generates a liveness indication based on whether the wearable device 102 is targeting skin of the live person. The liveness indication may be used for a security or authentication function of the wearable device 102. The presence of a heartbeat in the processed LDF signal can provide strong evidence that the sensor is targeting the skin on a live person, enabling the wearable device 102 to distinguish between a live user and inanimate objects or spoofing attempts.

In some embodiments, detecting the presence or absence of the heartbeat signal has a latency of approximately 2 seconds corresponding to capture of one or more cardiac cycles. The latency of approximately 2 seconds arises from the need to sample for one or more cardiac cycles to reliably detect the heartbeat signal. While the heartbeat-based liveness detection is slower relative to other techniques such as reflection strength or flow spectrum approaches, the heartbeat-based approach provides a high confidence signal that the wearable device 102 is targeting skin of a live person.

In some embodiments, the processing circuitry is configured to generate a confidence output based on the heartbeat signal. The liveness indication is based on the confidence output. The confidence output may be generated by passing the LDF signal to a heart rate algorithm that provides a confidence level indicating the reliability of the detected heartbeat. The confidence level may be used directly as a wear indicator or liveness indicator. In some embodiments, the processing circuitry takes a history of confidence results and passes the history to an algorithm to generate a longer term confidence estimate. The longer term confidence estimate may be based on an assumption that confidence rises over time as more evidence of a heartbeat is accumulated.

In some embodiments, the processing circuitry is configured to extend a period between authentication events based on the liveness indication indicating that an authenticated user has continuous custody of the wearable device 102. When the liveness indication indicates that the wearable device 102 is targeting skin of a live person, the wearable device 102 may infer that the authenticated user has maintained continuous custody of the wearable device 102 since a previous authentication event. Based on this inference, the wearable device 102 may extend the period before requiring a subsequent authentication event. For example, if a user authenticates to the wearable device 102 and the liveness indication continuously indicates that the wearable device 102 is targeting skin of a live person, the wearable device 102 may extend the time before requiring re-authentication. This approach improves user experience by reducing the frequency of authentication prompts while maintaining security by verifying that the authenticated user has not removed the wearable device 102.

In some embodiments, the liveness detection functionality addresses security-sensitive applications on the wearable device 102. Even applications as ubiquitous as messaging have privacy and security implications. A high-confidence liveness indication enables the wearable device 102 to extend periods between authentication by allowing the wearable device 102 to be confident that the authenticated user has had continuous custody of the experience. The liveness detection may also be used to detect spoofing attempts, where an attacker attempts to bypass security measures by presenting a non-living target to the sensor.

In some embodiments, the wearable device 102 comprises smartglasses or an extended-reality headset. The liveness detection functionality may be implemented on smartglasses to improve security for applications such as messaging, payments, or access control. The liveness detection functionality may be implemented on an extended-reality headset to verify that the authenticated user is wearing the headset before granting access to sensitive content or functionality. The LDF-based liveness detection provides a non-contact approach that does not require additional sensors or user interaction beyond wearing the wearable device 102.

In some embodiments, the liveness detection may be combined with the don/doff detection described previously to provide a comprehensive wear state and liveness determination. The don/doff detection may provide a rapid initial determination of whether the wearable device 102 is worn, while the liveness detection provides a higher confidence determination that the wearable device 102 is targeting skin of a live person. The combination of don/doff detection and liveness detection enables the wearable device 102 to make security decisions based on both the wear state and the liveness state.

Figure 6A illustrates a circuit diagram of an example driving circuit 600 for the light emitter 104, in accordance with some embodiments. In some embodiments, the driving circuit 600 for the light emitter 104 includes short-circuit fault current (SFC) protection, e.g., to protect the light emitter 104 from an accidental short (e.g., caused by overbiasing, overheating, etc.). For example, a portion 602 (e.g., an anode) of the driving circuit 600 is configured to convert an input voltage "VIN" 606 to a driving current 608 of light emitter 104. The portion 602 of the driving circuit 600 is immune to a fault caused by the anode. In another example, a portion 604 (e.g., a cathode) of the driving circuit 600 is configured to limits the driving current 608 to 10 mA in case of a fault caused by the cathode or the input voltage "VIN" 606.

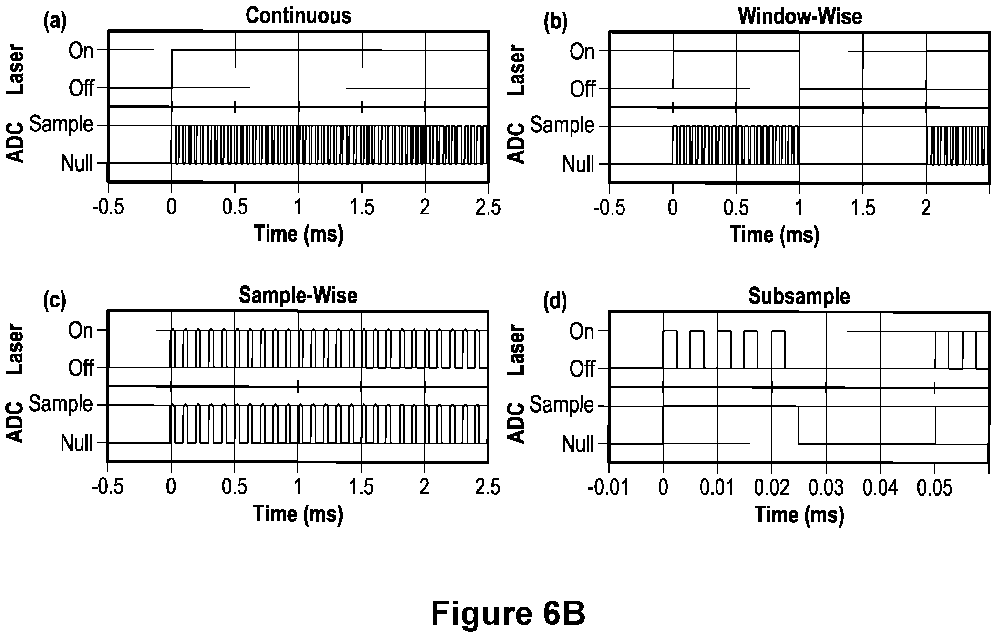

Figure 6B illustrates example timing diagrams for pulsed-mode laser Doppler flowmetry, in accordance with some embodiments. As described previously, the wearable device 102 comprises a light emitter, a photodetector, and processing circuitry. The light emitter projects an outputted optical signal to a portion of a body of a user. The light emitter operates in a pulsed-mode comprising a plurality of laser pulses separated by off periods. Figure 6B depicts four sub-diagrams labeled (a), (b), (c), and (d), each showing a different operation scheme for controlling the light emitter and sampling from the photodetector. Each sub-diagram displays two signal traces: a Laser signal showing On and Off states, and an ADC signal showing Sample and Null states, both plotted against Time in milliseconds.

Referring to Figure 6B, sub-diagram (a) is titled "Continuous" and depicts a continuous mode operation where the laser remains continuously on from approximately time 0 to time 2.5 ms, while an analog-to-digital converter (ADC) samples at a regular high-frequency rate throughout this period. In the continuous mode, the laser is run continuously and the photodetector is sampled at will. The continuous mode is simple to implement but may be non-ideal in consumer systems for a number of reasons, including laser tolerance requirements, optical safety, and coexistence with other optical systems.

With continued reference to Figure 6B, sub-diagram (b) is titled "Window-Wise" and depicts a window-wise pulse operation. In the window-wise pulse scheme, each laser pulse of the plurality of laser pulses includes multiple ADC samples captured during the laser pulse. The laser is turned on for a defined window period from approximately time 0 to time 1 ms, during which multiple ADC samples are collected. The laser then remains off while the ADC remains in a null state. The window-wise pulse approach offers a natural structure where each laser pulse corresponds to a processed sample. The wearable device 102 turns the laser on, collects a block of ADC samples, turns the laser off, and computes a single processed sample from that block of ADC samples.

In some embodiments, a pulse duration of each laser pulse is on an order of 1 millisecond. The window-wise pulse scheme uses pulses that are relatively long in order to capture enough samples to extend a lower end of a frequency range down to a low kHz region. In this example, a minimum pulse duration for window-wise pulsing is 500 microseconds, and a minimum dark time between laser pulses is 125 microseconds. The minimum dark time provides a period during which ambient light measurements may be captured for ambient light cancellation, as described elsewhere herein. The window-wise pulse approach offers a balance of performance and simplicity, with relatively relaxed timing requirements since the pulses are relatively long and infrequent, and the system (e.g., the wearable device 102) can permit significant settling time for a transmit side of the system.

With continued reference to Figure 6B, sub-diagram (c) is titled "Sample-Wise" and depicts a sample-wise pulse operation. In the sample-wise pulse scheme, each laser pulse of the plurality of laser pulses corresponds to a single ADC sample. The laser pulses and ADC samples occur in a one-to-one correspondence, with the laser cycling on and off at the same rate as an ADC sampling rate across a time period from approximately 0 to 2.5 ms. In the sample-wise scheme, there is no relation between the laser pulses and processed samples, and processed samples can be computed from any arbitrary block of ADC samples. The sample-wise approach results in much shorter laser pulses compared to the window-wise approach. The shorter pulse length may be more typical for pulsed laser systems in consumer products. However, the sample-wise approach implies stricter requirements on settling time and pulse-to-pulse stability of the laser, with pulse-to-pulse stability becoming a consideration.

Sub-diagram (d) in Figure 6B is titled "Subsample" and depicts a subsample pulse operation shown on a tighter time scale from approximately -0.01 to 0.06 ms. In the subsample pulse scheme, each ADC sample corresponds to multiple laser pulses of the plurality of laser pulses. The laser cycles on and off at a higher frequency than the ADC sampling rate, with multiple laser pulses occurring during each ADC sample period. The subsample pulse scheme permits very short pulses. However, inherent in the subsample approach is integration of dark noise in a receive signal, which may degrade signal-to-noise ratio. The subsample approach may also be prone to jitter in timing of the light emitter, where small variations in pulse duration would result in changes in pulse power. The subsample scheme is equivalent to the sample-wise approach but with sampling occurring through dark periods. The subsample scheme may be used if very short pulses are required for some reason and detector circuitry cannot achieve adequate timing precision.

In some embodiments, the window-wise pulse scheme is implemented in an integrated sensor module for product applications. The window-wise pulse scheme provides a balance between the continuous mode, which may present challenges for laser tolerance and optical safety, and the sample-wise or subsample schemes, which may impose stricter timing requirements. For example, by using pulses on the order of 1 millisecond with a minimum pulse duration of 500 microseconds and a minimum dark time of 125 microseconds, the window-wise pulse scheme enables the wearable device 102 to capture sufficient ADC samples within each pulse to achieve adequate frequency resolution while providing off periods for ambient light cancellation and coexistence with other optical systems.

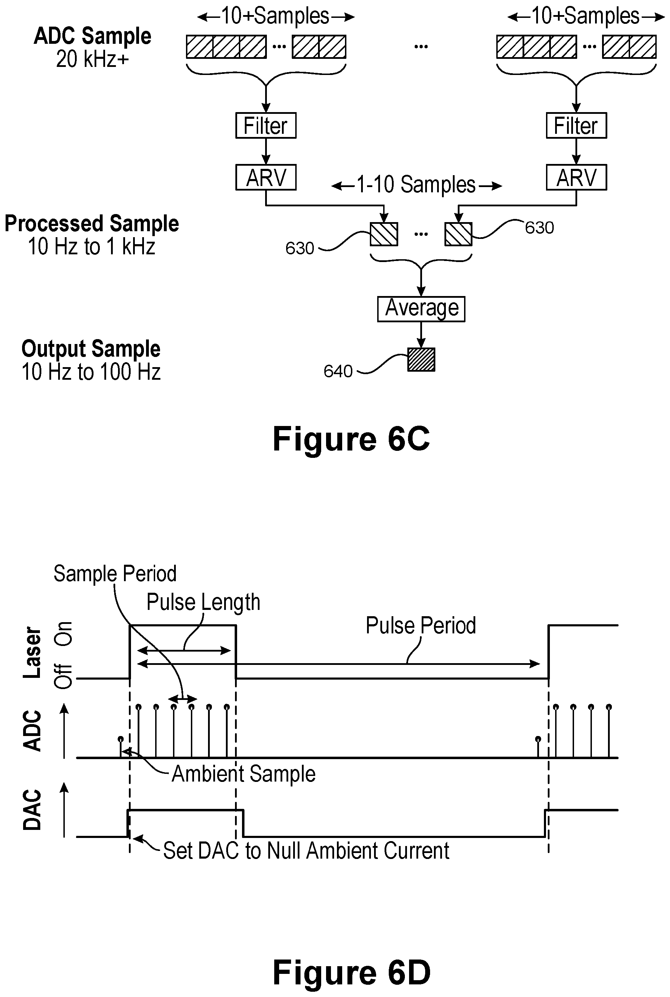

Figure 6C illustrates an example sample processing scheme showing a hierarchical relationship between different sample types used in LDF signal processing, in accordance with some embodiments. As described previously, the wearable device 102 captures, by the photodetector, a return optical signal corresponding to the outputted optical signal. The sample processing scheme depicted in Figure 6C shows how the return optical signal is processed through multiple stages to extract heart information of the user.

Referring to Figure 6C, the sample processing scheme comprises three hierarchical levels of samples: ADC samples, processed samples, and output samples. At a top level of the hierarchy, ADC samples are captured by sampling, by an ADC, the return optical signal at an ADC sampling rate to generate a plurality of ADC samples. In some embodiments, the ADC sampling rate is 20 kHz or higher. The ADC sampling rate of 20 kHz or higher enables the wearable device 102 to capture frequency content in the return optical signal that corresponds to blood flow information. For example, a frequency band of interest for blood flow measurement is approximately 5 kHz to 25 kHz, and the ADC sampling rate of 20 kHz or higher satisfies the Nyquist criterion for capturing this frequency content.

With continued reference to Figure 6C, the sample processing scheme shows two parallel processing paths, each receiving blocks of ADC samples. Each block comprises 10 or more ADC samples. The wearable device 102 processes a block of the plurality of ADC samples to generate a processed sample. Processing the block of ADC samples comprises filtering the block of ADC samples to isolate a frequency band of interest and performing energy quantification on the filtered ADC samples. As shown in Figure 6C, each block of ADC samples passes through a Filter stage that isolates the frequency band of interest. The frequency band of interest may be approximately 5 kHz to 25 kHz, corresponding to frequencies that encode blood flow velocity information. Following the Filter stage, each processing path includes an ARV stage that performs energy quantification on the filtered samples. In some embodiments, performing energy quantification comprises computing an ARV of the filtered ADC samples. The ARV computation quantifies power within the frequency band of interest, providing a measure of blood flow that varies with the cardiac cycle.

As further shown in Figure 6C, the output of each ARV stage produces a processed sample 630. The processed samples 630 are generated at a rate between 10 Hz and 1 kHz. In generating the processed sample 630, a consideration is the number of ADC samples to process, as this sets a frequency resolution of the filtering. The processed samples 630 encode flow information but may be computed at a rate higher than required to be output. In some embodiments, a minimum output data rate is 25 Hz for the processed flow signal. The processed samples 630 may be computed at a rate higher than required for output and then averaged or aggregated to generate output samples.

With continued reference to Figure 6C, the processed samples 630 from the parallel paths are combined through an Average stage. The wearable device 102 may generate an output sample 640 based on one or more processed samples 630. As shown in Figure 6C, 1 to 10 processed samples 630 are aggregated through the Average stage to produce the output sample 640. The output sample 640 is generated at an output sample rate corresponding to a physiological signal of interest. In some embodiments, the output sample rate is between 10 Hz and 100 Hz for capturing heartbeat information. For example, a heart rate of 60 beats per minute corresponds to a fundamental frequency of 1 Hz, and an output sample rate of 10 Hz to 100 Hz provides sufficient temporal resolution to capture variations in the heartbeat signal including higher harmonics and beat-to-beat variability.

The hierarchical sample processing scheme enables the wearable device 102 to reduce high-rate ADC data to a lower-rate output signal suitable for heart rate detection while preserving relevant frequency content through filtering and energy quantification operations. The wearable device 102 determines, based on the output sample 640, heart information of the user. The output sample 640, generated at the output sample rate between 10 Hz and 100 Hz, encodes the pulsatile blood flow information from which heart rate and other heart information may be extracted. By processing blocks of ADC samples through filtering and ARV computation to generate processed samples 630, and then aggregating the processed samples 630 through averaging to generate output samples 640, the sample processing scheme reduces computational burden on downstream processing while maintaining signal fidelity for heart information determination.

Although Figure 6C illustrates a specific hierarchical sample processing scheme with three levels of samples and two parallel processing paths, the present disclosure is not limited to this particular implementation. In some embodiments, the hierarchical structure comprises fewer than three levels or more than three levels of sample processing. In some embodiments, the ADC sampling rate is other than 20 kHz, such as sampling rates in the range of 10 kHz to 200 kHz selected based on the frequency content of the blood flow signal. In some embodiments, the number of ADC samples per block is other than 10, such as blocks of 5, 20, 50, 100, or more ADC samples selected based on the desired frequency resolution. In some embodiments, the filtering stage comprises bandpass filters, high-pass filters, notch filters, or combinations thereof rather than a single filter stage. In some embodiments, the energy quantification comprises root-mean-square (RMS) computation, variance computation, power spectral density estimation, or other statistical measures rather than ARV computation. In some embodiments, the processed sample rate is other than 10 Hz to 1 kHz, such as rates in the range of 1 Hz to 10 kHz selected based on the physiological signal of interest. In some embodiments, the output sample rate is other than 10 Hz to 100 Hz, such as rates in the range of 1 Hz to 1 kHz selected based on the target application. In some embodiments, the aggregation of processed samples 630 to generate output samples 640 comprises median filtering, weighted averaging, decimation, or other downsampling techniques rather than simple averaging. In some embodiments, the number of processed samples 630 aggregated per output sample 640 is other than 1 to 10, such as 1 to 100 or more processed samples 630 per output sample 640. In some embodiments, the number of parallel processing paths is other than two, such as a single processing path, three paths, four paths, or more paths corresponding to different frequency bands or different photodetectors. In some embodiments, the frequency band of interest is other than 5 kHz to 25 kHz, such as frequency bands in the range of 1 kHz to 50 kHz selected based on the target tissue type or blood vessel characteristics. In some embodiments, the blocks of ADC samples overlap with one another, providing improved temporal resolution at the cost of increased computation. In some embodiments, the sample processing scheme is applied to physiological signals other than heartbeat, such as respiration, blood pressure variability, or other periodic physiological phenomena. In some embodiments, the sample processing is performed using streaming or real-time processing architectures rather than block-based processing. Accordingly, the specific sample processing scheme illustrated in Figure 6C is provided as an example and should not be construed as limiting the scope of the present disclosure, the invention being defined in the appended claims.

Figure 6D illustrates example timing diagrams for pulsed-mode laser Doppler flowmetry showing ambient light cancellation, in accordance with some embodiments. As described previously, the wearable device 102 comprises a light emitter configured to project an outputted optical signal to a portion of a body of a user, wherein the light emitter is configured to operate in a pulsed-mode comprising a plurality of laser pulses separated by off periods. The wearable device 102 further comprises a photodetector configured to capture a return optical signal corresponding to the outputted optical signal, and an ADC configured to sample the return optical signal to generate ADC samples. The wearable device 102 may further comprise processing circuitry configured to process a block of the ADC samples to generate a processed sample (e.g., processed sample 630), generate an output sample (e.g., output sample 640) based on one or more processed samples, and determine, based on the output sample, heart information of the user.

Referring to Figure 6D, the timing diagram depicts three vertically aligned waveforms showing a relationship between laser operation, ADC sampling, and digital-to-analog converter (DAC) output over time. A top waveform shows a laser state transitioning between off and on states. Annotations in the top waveform indicate a pulse length corresponding to a duration when the laser is on, a sample period indicating an interval between individual samples, and a pulse period spanning from a start of one laser pulse to a start of a next laser pulse. The pulse length, sample period, and pulse period define temporal characteristics of the pulsed-mode operation.

With continued reference to Figure 6D, a middle waveform shows ADC sampling activity. Multiple sample points represented as vertical lines with dots at their tops occur during a laser on period. An ambient sample is captured during a laser off period before a pulse begins. The ambient sample provides a measurement of ambient light conditions when the light emitter is not active. By capturing the ambient sample during the off period, the wearable device 102 obtains a baseline measurement that represents contributions from ambient light sources without interference from the outputted optical signal.

A bottom waveform in Figure 6D shows a DAC output level. The DAC output transitions to a higher level after the ambient sample is captured. An annotation indicates that the DAC is set to null ambient current based on the ambient sample measurement. The wearable device 102 is configured to perform ambient light cancellation by sampling the return optical signal during an off period of the pulsed-mode to measure an ambient light level and adjusting a DAC to null an ambient signal based on the measured ambient light level. By adjusting the DAC based on the ambient sample, the wearable device 102 compensates for ambient light contributions before the laser pulse begins, enabling the ADC samples captured during the laser on period to represent primarily the return optical signal corresponding to the outputted optical signal rather than ambient light interference.

In some embodiments, the ambient light cancellation follows a sequence of operations for each pulse period. In a first step, while the light emitter is off during an off period, the ADC samples the return optical signal one or more times to measure the ambient light level. This ambient sample captures contributions from ambient light sources without interference from the outputted optical signal. In a second step, based on the measured ambient light level, the DAC is set to null the ambient signal. The DAC output is adjusted so that the ambient current contribution is substantially cancelled before the laser pulse begins. In a third step, the light emitter is turned on to emit a laser pulse, and the ADC samples the return optical signal during the laser pulse to capture a flow signal. Because the DAC has been adjusted to null the ambient contribution, the ADC samples captured during the laser on period represent primarily the return optical signal corresponding to the outputted optical signal rather than ambient light interference. This sequence repeats for each pulse period, enabling the wearable device 102 to continuously adapt to changing ambient light conditions.

In some embodiments, while the LDF signal itself is largely immune to effects of ambient light due to a focus on higher frequency components, ambient light can present a challenge to circuitry by creating large offsets that may saturate early stages of the signal processing chain or require large dynamic range. Active ambient light cancellation can be effective in addressing these challenges. The pulsed-mode operation provides a natural framework for implementing active ambient light cancellation, as the off periods between laser pulses provide opportunities to measure ambient light levels without interference from the outputted optical signal.

In some embodiments, the wearable device 102 is configured to determine a proximity estimate based on a difference between samples collected while the light emitter is on and an ambient sample collected while the light emitter is off. While ambient light cancellation uses only the ambient sample captured during the laser off period, proximity sensing uses a delta between the laser off and laser on states to measure reflection strength. The proximity estimate is computed by determining a difference between the samples collected during the laser on period and the ambient sample collected during the laser off period, and correcting for the DAC offset. The proximity estimate provides a measure of how close the portion of the body is to the wearable device 102 based on the strength of the return optical signal. In some embodiments, the wearable device 102 provides a proximity estimate for every laser pulse. In some embodiments, the wearable device 102 provides a proximity estimate every several pulses.

In some embodiments, the light emitter comprises a VCSEL. As described previously, the VCSEL may have a nominal emission wavelength in a near-infrared spectrum, such as approximately 850 nm or approximately 940 nm. The VCSEL may have a coherence length greater than a typical distance from the wearable device 102 to the skin, on the order of 2 cm. The pulsed-mode operation of the VCSEL enables the ambient light cancellation and proximity sensing functionality described with reference to Figure 6D.

In some embodiments, the wearable device 102 comprises an extended-reality headset. The ambient light cancellation functionality enables the extended-reality headset to perform LDF-based heart rate monitoring in varying ambient light conditions, including indoor environments with artificial lighting and outdoor environments with sunlight. The proximity sensing functionality enables the extended-reality headset to detect when the headset is positioned near the face of the user, which may be used for don/doff detection as described elsewhere herein.

In some embodiments, the pulsed-mode provides optical safety benefits by operating the light emitter farther from a lasing threshold compared to continuous-wave operation. Commodity grade VCSELs may have wide tolerances related to lasing and optical efficiency. For example, a threshold current of a VCSEL may range from 1 mA to 5 mA, with a slope efficiency ranging from 0.2 mW/mA to 0.65 mW/mA. In some embodiments, the VCSEL has a maximum allowable current of 8 mA. As a result, if operating without optical feedback and without calibration, the optical output at the maximum allowable current may be as low as 0.6 mW or as high as 4.55 mW, illustrating the tolerance challenge particularly when operating at low output powers. Operating in pulsed mode and varying a duty cycle provides an effective way to control average power over a wide range. The reduction in uncertainty that comes from operating farther from the lasing threshold can reduce margin requirements for optical safety.

In some embodiments, the pulsed-mode operation enables improved coexistence with other optical systems of the wearable device 102. One potential challenge faced by LDF is contention with other optical systems in the same product. For example, many eye tracking methods utilize an array of infrared illuminators arranged around an eye. There is a risk of optical coupling between the systems, where light from the LDF system might be observed by an eye tracking system and degrade performance, and light from eye tracking illuminators might be received by the LDF photodetector. Running in pulsed mode allows the wearable device 102 to time multiplex the systems to eliminate contention. The wearable device 102 may be configured to time multiplex the LDF system with eye tracking illuminators, e.g., turning off eye tracking illuminators during LDF laser pulses. The LDF laser is turned on in periods between samples of the eye tracking system, while the eye tracking illuminators are turned off. This time multiplexing enables both the LDF system and the eye tracking system to operate without optical interference from one another.

Although Figure 6D illustrates a specific timing diagram for ambient light cancellation and proximity sensing, the present disclosure is not limited to this particular implementation. In some embodiments, the number of ambient samples captured per pulse period is other than one, such as two, three, or more ambient samples that are averaged to reduce noise in the ambient light measurement. In some embodiments, the DAC adjustment is performed using techniques other than direct nulling, such as proportional adjustment, filtered adjustment, or predictive adjustment based on historical ambient light measurements. In some embodiments, the proximity estimate is computed using techniques other than simple differencing, such as ratio computation, normalized differencing, or model-based estimation. In some embodiments, the time multiplexing with other optical systems is performed using techniques other than alternating pulses, such as frequency division multiplexing, code division multiplexing, or adaptive scheduling based on system priorities. In some embodiments, the ambient light cancellation and proximity sensing functionality is implemented in hardware, firmware, software, or combinations thereof. Accordingly, the specific timing diagram illustrated in Figure 6D is provided as an example and should not be construed as limiting the scope of the present disclosure, the invention being defined in the appended claims.

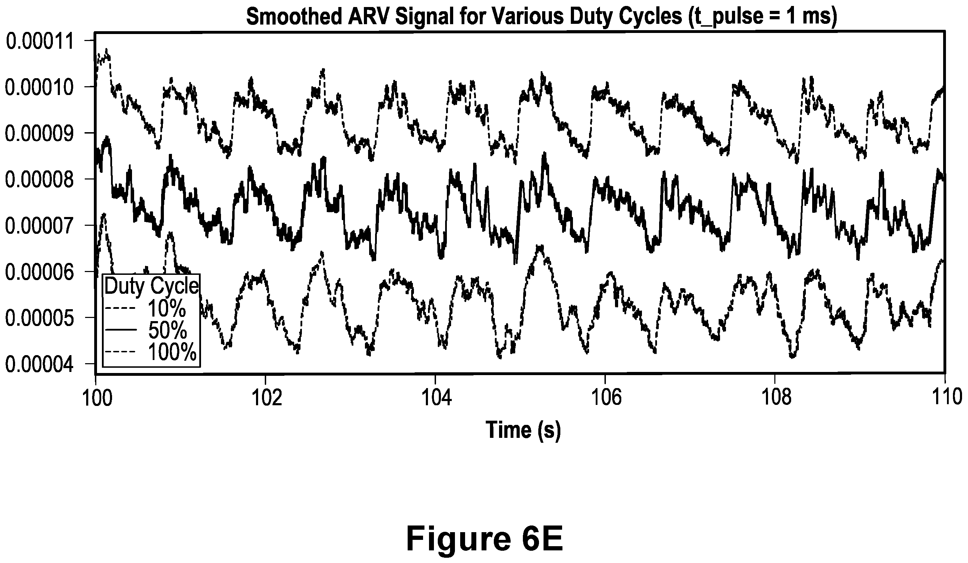

Figure 6E illustrates example time-series output waveforms showing heartbeat signals for different duty cycles of pulsed-mode LDF operation, in accordance with some embodiments. As described previously, the light emitter operates in a pulsed mode comprising a plurality of laser pulses separated by off periods. A duty cycle of the pulsed mode is controlled to adjust an average optical power of the outputted optical signal. Figure 6E depicts a graph showing smoothed ARV signals for duty cycles of 10%, 50%, and 100% with a pulse duration of 1 millisecond.

Referring to Figure 6E, a vertical axis represents signal amplitude ranging from approximately 0.00004 to 0.00011, while a horizontal axis represents time in seconds ranging from 100 to 110 seconds. Three waveforms are displayed corresponding to different duty cycle percentages as indicated in a legend. A waveform corresponding to a 100% duty cycle is shown in green and oscillates in a higher amplitude range between approximately 0.00009 and 0.00011. The 100% duty cycle case is equivalent to continuous mode operation, where the laser remains continuously on and the photodetector is sampled at will. At 100% duty cycle, the waveform exhibits a clear heartbeat pattern with sharp rises on pulse arrival followed by gradual decay, demonstrating well-defined cardiac pulse morphology.

With continued reference to Figure 6E, a waveform corresponding to a 50% duty cycle is shown in orange and oscillates in a middle amplitude range between approximately 0.00007 and 0.00009. The 50% duty cycle waveform maintains a strong fundamental frequency corresponding to the heartbeat while exhibiting some reduction in shape definition compared to the 100% duty cycle waveform. The 50% duty cycle represents a window-wise pulse operation where the laser is on for half of each pulse period, providing a balance between signal quality and reduced average optical power.

As further shown in Figure 6E, a waveform corresponding to a 10% duty cycle is shown in blue and oscillates in a lower amplitude range between approximately 0.00004 and 0.00007. The 10% duty cycle waveform shows increased low frequency noise compared to the higher duty cycle waveforms while still maintaining a detectable fundamental heartbeat frequency. Each waveform exhibits periodic oscillations characteristic of heartbeat signals, with the 100% duty cycle waveform showing sharper definition of the cardiac pulse shape compared to the lower duty cycle waveforms. The graph demonstrates that as duty cycle decreases, signal amplitude decreases and low frequency noise increases, while the fundamental heartbeat frequency remains detectable across all duty cycle conditions.

In some embodiments, the duty cycle is variable within a range from 1.25% to 80%. The variable duty cycle range enables the wearable device 102 to control average optical power over a broad dynamic range while maintaining consistent instantaneous laser power. For example, if a window-wise pulse scheme is implemented with a minimum pulse duration of 500 microseconds, a minimum dark time of 125 microseconds, and a minimum output data rate of 25 Hz, the duty cycle can be varied from a minimum of 1.25% up to a maximum of 80%. This corresponds to a dynamic range of 36 dB for the same instantaneous laser power. The ability to vary duty cycle over this range enables the wearable device 102 to adapt to different operating conditions, user characteristics, and optical safety requirements while maintaining heart rate detection capability.

In some embodiments, the duty cycle has a minimum value of 10%. The minimum duty cycle of 10% represents a lower bound that provides acceptable signal quality for heart rate detection while achieving substantial reduction in average optical power compared to continuous mode operation. As shown in Figure 6E, the 10% duty cycle waveform maintains a detectable fundamental heartbeat frequency despite the increased low frequency noise and reduced signal amplitude. The minimum duty cycle of 10% may be selected based on a tradeoff between signal quality requirements and power reduction objectives. For applications requiring higher signal quality or more precise cardiac pulse morphology analysis, higher duty cycles may be selected. For applications prioritizing power reduction or optical safety, lower duty cycles approaching the minimum value of 10% may be selected while still maintaining heart rate detection capability.

The tradeoffs between duty cycle, average optical power, and signal quality are illustrated by the waveforms in Figure 6E. Reducing the duty cycle from 100% to 50% to 10% progressively decreases the average optical power of the outputted optical signal while also decreasing signal amplitude and increasing low frequency noise in the processed signal. However, the fundamental heartbeat frequency remains detectable across all three duty cycle conditions shown, demonstrating that pulsed-mode operation with reduced duty cycles can maintain heart rate detection capability while achieving benefits including reduced average optical power, relaxed laser tolerance requirements, and improved optical safety margins. The selection of duty cycle for a particular application may be based on balancing these tradeoffs according to the requirements of the wearable device 102 and the target use case.

Although Figure 6E illustrates specific waveforms for duty cycles of 10%, 50%, and 100%, the present disclosure is not limited to these particular duty cycle values. In some embodiments, the duty cycle is set to values other than 10%, 50%, or 100%, such as duty cycles of 5%, 20%, 30%, 40%, 60%, 70%, or other values within the variable range from 1.25% to 80%. In some embodiments, the duty cycle is dynamically adjusted during operation based on signal quality metrics, ambient light conditions, battery state, or other operating parameters. In some embodiments, the relationship between duty cycle and signal quality differs from that illustrated based on the specific implementation, optical configuration, or tissue characteristics. In some embodiments, the minimum duty cycle is other than 10%, such as minimum duty cycles of 5%, 15%, 20%, or other values selected based on signal quality requirements. Accordingly, the specific waveforms illustrated in Figure 6E are provided as examples and should not be construed as limiting the scope of the present disclosure, the invention being defined in the appended claims.

In some embodiments, a non-transitory computer-readable storage medium stores instructions that, when executed by one or more processors of a wearable device (e.g., the wearable device 102) comprising a light emitter and a photodetector, cause the wearable device to perform operations for biometric data monitoring with optical coexistence. As described previously, the wearable device projects, from the light emitter, an outputted optical signal to a portion of a body of a user, wherein the light emitter operates in a pulsed mode comprising a plurality of laser pulses separated by off periods. The wearable device captures, by the photodetector, a return optical signal corresponding to the outputted optical signal. The wearable device samples the return optical signal at an ADC sampling rate to generate a plurality of ADC samples. The wearable device processes a block of the plurality of ADC samples to generate a processed sample (e.g., processed sample 630). The wearable device generates an output sample (e.g., output sample 640) based on one or more processed samples. The wearable device determines, based on the output sample, heart information of the user. The heart information comprises heart rate information determined in accordance with principles of LDF.

In some embodiments, the operations further comprise time multiplexing operation of the light emitter with one or more other optical systems of the wearable device to reduce optical contention. The wearable device may include multiple optical systems that operate in overlapping wavelength bands, such as near-infrared bands. Without coordination between these optical systems, light from one system may be detected by sensors of another system, causing interference that degrades performance of one or both systems. Time multiplexing addresses this challenge by coordinating the timing of optical emissions from different systems such that the systems do not emit light simultaneously. By time multiplexing the operation of the light emitter with the one or more other optical systems, the wearable device reduces optical contention and enables multiple optical systems to coexist within the same device without mutual interference.

In some embodiments, the one or more other optical systems comprise an eye tracking system. Eye tracking systems may utilize an array of infrared illuminators arranged around an eye of the user to illuminate the eye for imaging by one or more cameras. The infrared illuminators of the eye tracking system may emit light in wavelength bands that overlap with the wavelength band of the light emitter used for LDF. Without time multiplexing, light from the LDF light emitter might be observed by cameras of the eye tracking system and degrade eye tracking performance. Conversely, light from the eye tracking illuminators might be received by the photodetector of the LDF system and interfere with blood flow measurements. Time multiplexing the LDF system with the eye tracking system eliminates these sources of optical contention.

In some embodiments, the light emitter is turned on during periods between samples of the eye tracking system. The eye tracking system captures images of the eye at a sampling rate determined by the eye tracking application requirements. Between successive image captures by the eye tracking system, there are periods during which the eye tracking cameras are not actively sampling. The light emitter of the LDF system is turned on during these periods between samples of the eye tracking system. By timing the laser pulses of the LDF system to occur during periods when the eye tracking system is not capturing images, the wearable device prevents light from the LDF light emitter from appearing in eye tracking images. This coordination enables the LDF system to operate without degrading eye tracking performance.

In some embodiments, the operations further comprise turning off illuminators of the one or more other optical systems during the plurality of laser pulses. When the light emitter of the LDF system is turned on to emit a laser pulse, the illuminators of the eye tracking system or other optical systems are turned off. By turning off the illuminators of the one or more other optical systems during the plurality of laser pulses, the wearable device prevents light from the other optical systems from being received by the photodetector of the LDF system during active measurement periods. This bidirectional coordination ensures that neither the LDF system nor the other optical systems interfere with one another. The LDF system operates during periods when the eye tracking illuminators are off, and the eye tracking system operates during periods when the LDF light emitter is off.

In some embodiments, the time multiplexing is implemented by a controller or scheduler that coordinates timing of the light emitter and the illuminators of the one or more other optical systems. The controller may receive timing information from the eye tracking system indicating when image captures will occur, and may schedule laser pulses of the LDF system to occur during intervals between the image captures. Alternatively, the controller may provide timing information to the eye tracking system indicating when laser pulses will occur, and the eye tracking system may schedule image captures to occur during intervals between the laser pulses. The coordination may be implemented through hardware timing circuits, firmware scheduling, software coordination, or combinations thereof.

In some embodiments, the pulsed-mode operation of the light emitter facilitates time multiplexing with the one or more other optical systems. As described previously, the light emitter operates in a pulsed mode comprising a plurality of laser pulses separated by off periods. The off periods between laser pulses provide natural intervals during which the one or more other optical systems may operate without interference from the LDF light emitter. Similarly, the pulsed nature of the LDF operation enables the laser pulses to be scheduled during intervals when the one or more other optical systems are not actively emitting or sampling. Continuous-wave operation of the light emitter would not provide these natural intervals for time multiplexing, making coexistence with other optical systems more challenging.

In some embodiments, the time multiplexing enables the wearable device to perform LDF-based heart rate monitoring while simultaneously supporting eye tracking functionality. Extended-reality headsets may use eye tracking for gaze-based interaction, foveated rendering, or other applications. By time multiplexing the LDF system with the eye tracking system, the wearable device can provide both heart rate monitoring and eye tracking without requiring the user to choose between these functionalities or accept degraded performance in either system. The time multiplexing approach enables the wearable device to support multiple optical sensing modalities within a compact form factor (e.g., a high-compact form factor) where physical separation of optical systems may not be feasible.

Although the time multiplexing is described with reference to an eye tracking system, the present disclosure is not limited to this particular other optical system. In some embodiments, the one or more other optical systems comprise depth sensing systems, gesture recognition systems, facial tracking systems, proximity sensing systems, or other optical systems that may be present in the wearable device. In some embodiments, the time multiplexing coordinates the LDF system with multiple other optical systems simultaneously, scheduling laser pulses during intervals when none of the other optical systems are actively emitting or sampling. In some embodiments, the time multiplexing is adaptive, adjusting the timing of laser pulses based on the current operating state of the one or more other optical systems. Accordingly, the specific time multiplexing approach described herein is provided as an example and should not be construed as limiting the scope of the present disclosure, the invention being defined in the appended claims.

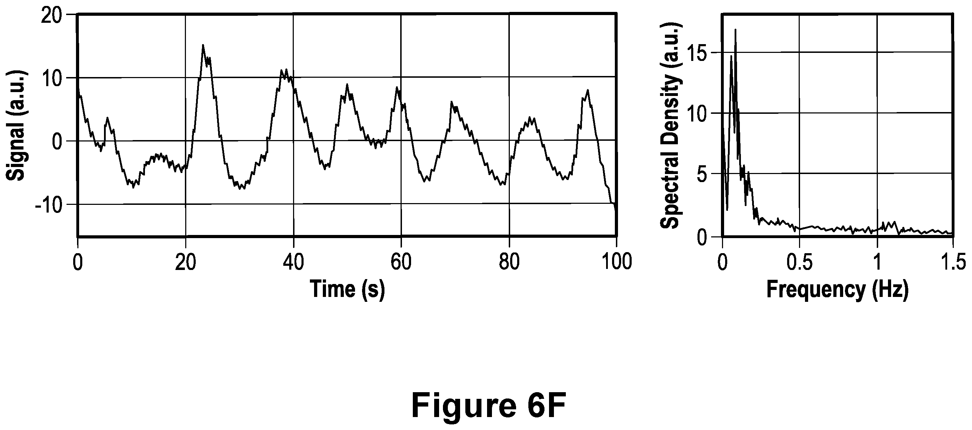

Figure 6F illustrates an example wrist-based PPG signal, in accordance with some embodiments. The figure comprises two graphs displayed side by side. A left graph shows a time-series signal plot with time in seconds on a horizontal axis ranging from 0 to 100 seconds and signal amplitude in arbitrary units on a vertical axis ranging from approximately -10 to 20. The signal waveform exhibits a quasi-periodic pattern with peaks occurring approximately every 15 to 20 seconds, showing characteristic modulation with sharp rises followed by gradual decays. A right graph shows a spectral density plot with frequency in Hz on a horizontal axis ranging from 0 to 1.5 Hz and spectral density in arbitrary units on a vertical axis ranging from 0 to approximately 17. The spectral density plot exhibits a prominent peak near 0 Hz with a dominant component at approximately 0.1 Hz. The dominant component at approximately 0.1 Hz corresponds to Mayer waves. The Mayer wave component in the PPG signal is so strong that any respiratory component appears buried beneath spectral broadening arising from instability in the Mayer wave fundamental. The spectral density decreases toward higher frequencies, approaching near-zero values above approximately 0.5 Hz.

With continued reference to Figure 6F, the PPG signal demonstrates characteristics that may limit respiration rate estimation. PPG measures distension of small blood vessels due to pressure changes. The PPG signal encodes vessel distension information. The dominant Mayer wave component at approximately 0.1 Hz can obscure respiratory modulation that may be present in a similar frequency range. As shown in the spectral density plot of Figure 6F, the Mayer wave component dominates the low-frequency spectrum, making identification of a distinct respiratory peak challenging. The strong Mayer wave component in PPG signals may limit the ability to accurately infer respiration rate from a PPG signal in all but the most calm scenarios.

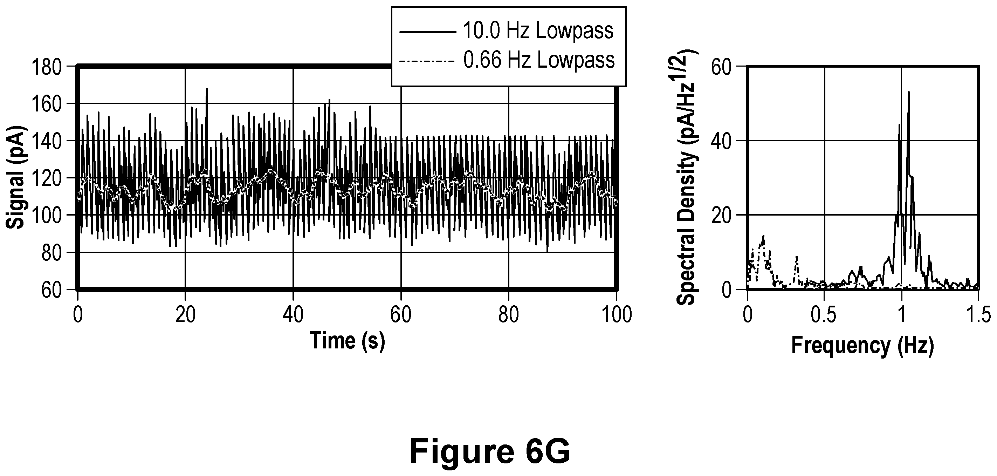

Figure 6G illustrates an example forehead-based laser LDF signal, in accordance with some embodiments. The figure comprises two graphs displayed side by side. A left graph shows a time-domain representation of a signal measured in picoamperes (pA) on a vertical axis versus time measured in seconds on a horizontal axis, spanning from 0 to 100 seconds. Two waveforms are displayed: a blue waveform representing a 10.0 Hz lowpass filtered signal that exhibits high-frequency oscillations ranging approximately between 60 pA and 180 pA, and a red waveform representing a 0.66 Hz lowpass filtered signal that shows a smoother trace oscillating approximately between 100 pA and 125 pA. A right graph shows a frequency-domain representation displaying spectral density measured in pA/Hz^(1/2) on a vertical axis versus frequency measured in Hz on a horizontal axis, spanning from 0 to 1.5 Hz. The blue curve corresponding to the 10.0 Hz lowpass signal shows a prominent peak at approximately 1 Hz with a spectral density reaching approximately 60 pA/Hz^(1/2). The red curve corresponding to the 0.66 Hz lowpass signal shows lower frequency content with peaks below 0.5 Hz and spectral density values reaching approximately 15 pA/Hz^(1/2).

Referring to Figure 6G, the LDF signal demonstrates a relatively modest Mayer wave component compared to the PPG signal shown in Figure 6F. The frequency-domain representation in Figure 6G shows a broad peak around 0.1 Hz representing Mayer wave modulation. However, the respiration frequency band exhibits a distinct peak around 0.3 Hz corresponding to respiration, separate from the broad peak around 0.1 Hz representing Mayer waves. The distinct peak at approximately 0.3 Hz enables improved resolution of the respiration rate compared to the PPG signal where the Mayer wave component dominates. The LDF signal shows a relatively modest Mayer wave component compared to PPG, enabling better resolution of the respiratory component.

As described previously, the wearable device projects, from the light emitter, an outputted optical signal to a portion of a body of a user, and captures, by the photodetector, a return optical signal corresponding to the outputted optical signal. The wearable device collects a time series flow signal based on the return optical signal. The time series flow signal encodes a representation of blood flow. The time series flow signal is determined in accordance with principles of LDF. The time series flow signal encodes blood flow information. Blood flow information measured by LDF differs from vessel distension information measured by PPG. This difference in what the two techniques measure may contribute to the different spectral characteristics observed in Figures 6F and 6G.

In some embodiments, the time series flow signal comprises a processed flow signal that encodes an abstracted representation of blood flow rate. The blood flow rate comprises at least one of a volumetric flow rate or a velocity. The processed flow signal may be generated by quantifying power within a frequency band of interest, as described previously with reference to the ARV computation. The processed flow signal encodes an abstracted representation of blood flow that varies with physiological processes including respiration and cardiac activity.

In some embodiments, the time series flow signal is reported at a rate between 25 Hz and 200 Hz. The time series flow signal is reported at at least twice the rate of the underlying signal modulation. For example, a respiration rate of 30 breaths per minute corresponds to a fundamental frequency of 0.5 Hz, and a sampling rate of at least 1 Hz satisfies the Nyquist criterion for capturing the respiratory modulation. However, the time series flow signal may be reported at a rate between 25 Hz and 200 Hz to enable estimation of variation in beat-to-beat interval and to capture higher frequency components of the blood flow signal. The processed flow signal may be reported at 25 Hz to 200 Hz, with a minimum of at least twice the rate of the underlying signal modulation.

In some embodiments, the wearable device filters the time series flow signal to isolate a respiration frequency band. The respiration frequency band is in a range from 0.1 Hz to 0.5 Hz. The filtering may include basic filtering to isolate the frequency band of interest corresponding to respiratory modulation. As shown in Figure 6G, the respiration frequency band from 0.1 Hz to 0.5 Hz encompasses both the Mayer wave component around 0.1 Hz and the respiratory component around 0.3 Hz. The filtered time series flow signal exhibits a distinct peak at approximately 0.3 Hz corresponding to the respiration rate.

In some embodiments, the wearable device identifies, based on the filtered time series flow signal, a fundamental frequency within the respiration frequency band corresponding to a respiration rate of the user. As shown in Figure 6G, the distinct peak around 0.3 Hz in the spectral density plot corresponds to the respiration rate. The wearable device may apply an algorithm to identify the fundamental frequency within the respiration frequency band. The wearable device outputs the respiration rate of the user. The respiration rate may be reported and used for health monitoring applications.

The comparison between Figures 6F and 6G demonstrates that the time series flow signal determined in accordance with principles of LDF exhibits a more modest Mayer wave component compared to a PPG signal, enabling improved resolution of the respiration rate. The PPG signal in Figure 6F shows a dominant Mayer wave component at approximately 0.1 Hz that obscures respiratory modulation. In contrast, the LDF signal in Figure 6G shows a relatively modest Mayer wave component with a distinct peak around 0.3 Hz corresponding to respiration. This difference may arise from the difference in what PPG and LDF measure. The LDF signal may be less sensitive to vascular tone compared to PPG, resulting in a more modest Mayer wave component that enables improved resolution of the respiratory component.

In some embodiments, identifying the fundamental frequency within the respiration frequency band comprises applying a time domain algorithm comprising an autocorrelation approach. The autocorrelation approach computes a correlation of the filtered time series flow signal with a time-shifted version of itself. Peaks in the autocorrelation function correspond to periodicities in the filtered time series flow signal. The wearable device may identify a lag value corresponding to a peak in the autocorrelation function, and the fundamental frequency may be computed as an inverse of the lag value. For example, if the autocorrelation function exhibits a peak at a lag of 3 seconds, the fundamental frequency is computed as 1/3 Hz, corresponding to a respiration rate of approximately 20 breaths per minute. The autocorrelation approach operates in the time domain and may be computationally efficient for real-time respiration rate estimation. The autocorrelation approach may be robust to noise and amplitude variations in the filtered time series flow signal, as the autocorrelation function emphasizes periodic components regardless of absolute signal amplitude.

In some embodiments, identifying the fundamental frequency within the respiration frequency band comprises applying a frequency domain algorithm comprising a Fast Fourier Transform (FFT). The FFT transforms the filtered time series flow signal from the time domain to the frequency domain, producing a spectrum that represents signal power as a function of frequency. The wearable device identifies a peak in the spectrum within the respiration frequency band, and the frequency corresponding to the peak is identified as the fundamental frequency corresponding to the respiration rate. For example, if the spectrum exhibits a peak at 0.3 Hz within the respiration frequency band from 0.1 Hz to 0.5 Hz, the fundamental frequency is identified as 0.3 Hz, corresponding to a respiration rate of approximately 18 breaths per minute. The FFT approach provides a direct representation of frequency content in the filtered time series flow signal and may enable identification of multiple frequency components including harmonics of the fundamental respiration frequency. The FFT approach may also enable estimation of signal quality or confidence based on characteristics of the spectral peak, such as peak height relative to surrounding noise floor or peak width.

In some embodiments, the wearable device applies both the time domain algorithm comprising the autocorrelation approach and the frequency domain algorithm comprising the FFT to identify the fundamental frequency. Results from the two approaches are compared or combined to improve accuracy and robustness of the respiration rate estimation. If both approaches identify a similar fundamental frequency, confidence in the respiration rate estimate is increased. If the two approaches identify different fundamental frequencies, the wearable device selects one result based on signal quality metrics or flags the estimate as uncertain.

In some embodiments, learned models are used to identify the fundamental frequency within the respiration frequency band. The learned models are trained on labeled data comprising filtered time series flow signals and corresponding ground truth respiration rates. The learned models comprise neural networks, support vector machines, random forests, or other machine-learning architectures. The learned models receive the filtered time series flow signal as input and output an estimated respiration rate.

In some embodiments, respiratory modulation appears in the time series flow signal as at least one of a modulation of a baseline flow or a modulation of a flow pulsatility amplitude. Respiration modulates the cardiovascular system through variations in volume and pressure in a thoracic cavity with inhalation and exhalation. The modulation of a baseline flow refers to variations in a mean or DC level of the time series flow signal that correlate with the respiratory cycle. The modulation of a flow pulsatility amplitude refers to variations in an amplitude of cardiac pulsations in the time series flow signal that correlate with the respiratory cycle. Both types of modulation produce variations at the respiration frequency and are distinct from one another, though both are present simultaneously in some embodiments.

In some embodiments, the wearable device detects the modulation of baseline flow by applying a lowpass filter to the time series flow signal to extract the baseline component, and then analyzing the baseline component to identify periodic variations at the respiration frequency. In some embodiments, the wearable device detects the modulation of flow pulsatility amplitude by computing an envelope of the time series flow signal, such as by rectifying and lowpass filtering the signal or by applying a Hilbert transform, and then analyzing the envelope to identify periodic variations at the respiration frequency.

In some embodiments, respiration also results in a small variation in an interbeat interval due to respiratory sinus arrhythmia, where heart rate increases during inhalation and decreases during exhalation. However, the potential differentiated value of LDF for respiration rate estimation is in the baseline flow and flow pulsatility amplitude signals, where the more modest Mayer wave component in LDF compared to PPG enables improved resolution of the respiratory component.

As described previously, the light emitter and the photodetector are configured for non-contact sensing. The non-contact sensing configuration enables the wearable device to measure respiration rate without requiring direct skin contact, which is advantageous for form factors such as smartglasses where consistent skin contact is difficult to achieve.

In some embodiments, the wearable device comprises one or more additional sensors in addition to the light emitter and the photodetector. The wearable device comprises processing circuitry configured to collect the time series flow signal, obtain one or more additional signals from the one or more additional sensors, perform sensor fusion using the time series flow signal and the one or more additional signals, and estimate, based on the sensor fusion, a respiration rate of the user.

In some embodiments, the one or more additional sensors comprise an IMU configured to measure motion of the user. When the wearable device is worn on the head of the user, the IMU measures head motion that includes respiratory-related motion components. The processing circuitry extracts the respiratory-related motion components from the motion data and combines the extracted components with the time series flow signal through sensor fusion.

In some embodiments, the one or more additional sensors comprise a microphone configured to capture breath sounds of the user. Breath sounds exhibit periodic characteristics that correlate with the respiratory cycle. The processing circuitry extracts the breath sound components from the audio data and combines the extracted components with the time series flow signal through sensor fusion.

In some embodiments, the time series flow signal and the one or more additional signals exhibit complementarity. The one or more additional signals provide improved respiration estimation during strenuous activity when breath sounds are more pronounced, while the time series flow signal provides improved respiration estimation during rest when motion artifacts are minimal. The processing circuitry weights contributions from each sensing modality based on signal quality metrics, activity level, or other contextual information.

In some embodiments, the wearable device comprises smartglasses, an extended-reality headset, earbuds, or a wrist-wearable device. The smartglasses form factor positions the light emitter and the photodetector in proximity to the face of the user and provides a platform for integrating the IMU and the microphone. The earbuds form factor positions the sensors in proximity to an ear of the user. The wrist-wearable device form factor enables long-term monitoring during sleep and daily activities. Each form factor implements the respiratory modulation detection and respiration rate estimation functionality described herein.

In some embodiments, the portion of the body comprises at least one of a face, an ear, or a wrist of the user, depending on the wearable device form factor. The respiratory modulation detection and respiration rate estimation are performed regardless of which portion of the body is targeted, as the respiratory modulation of the cardiovascular system affects blood flow throughout the body.

In some embodiments, a non-transitory computer-readable storage medium stores instructions that, when executed by one or more processors of a wearable device, cause the wearable device to perform operations for detecting respiratory modulation and estimating respiration rate. The wearable device detects respiratory modulation in the time series flow signal, wherein the respiratory modulation comprises at least one of a baseline flow modulation or a flow pulsatility amplitude modulation, and estimates, based on the detected respiratory modulation, a respiration rate of the user. In some embodiments, the operations further comprise detecting an interbeat interval modulation in the time series flow signal, and the respiration rate is estimated based on the interbeat interval modulation in combination with the baseline flow modulation or the flow pulsatility amplitude modulation.

Although specific wearable device form factors and body portions are described, the present disclosure is not limited to these particular implementations. In some embodiments, the wearable device comprises form factors other than smartglasses, wrist-wearable devices, or earbuds, such as smart rings, smart patches, head-mounted displays, or other body-worn devices. In some embodiments, the portion of the body comprises regions other than the face, ear, or wrist, such as the neck, chest, temple, or other regions with sufficient blood perfusion for LDF sensing. In some embodiments, the respiratory modulation detection uses techniques other than those described, such as wavelet analysis, empirical mode decomposition, or other signal processing approaches. Accordingly, the specific implementations described herein are provided as examples and should not be construed as limiting the scope of the present disclosure, the invention being defined in the appended claims.

Figure 6H illustrates example motion artifacts in a PPG signal during various activities, in accordance with some embodiments. The figure comprises six graphs arranged in two rows and three columns, corresponding to three activity states: Rest, Walk, and Run. A top row displays PPG signals measured in arbitrary units over a 10-second time period for each activity state. A bottom row displays corresponding acceleration signals measured in units of gravitational acceleration (g) over the same 10-second time periods, with three axes labeled X, Y, and Z shown in different colors.

Referring to Figure 6H, during a Rest condition, the PPG signal exhibits a clean periodic waveform characteristic of heartbeat detection. The acceleration signals remain relatively flat and stable across all three axes during the Rest condition, indicating minimal motion. The clean periodic waveform during rest demonstrates that the PPG signal can capture heartbeat information when motion is absent. During a Walk condition, the PPG signal shows increased amplitude variations and some distortion compared to the Rest condition. The acceleration signals display periodic oscillations corresponding to a walking gait pattern. The periodic oscillations in the acceleration signals correlate with the distortion observed in the PPG signal, indicating that motion during walking introduces artifacts into the PPG measurement.

As further shown in Figure 6H, during a Run condition, the PPG signal exhibits substantial noise and irregular variations that obscure an underlying heartbeat pattern. The acceleration signals show large amplitude oscillations with peaks reaching approximately 4 to 6 g, reflecting a high-impact nature of running activity. The acceleration plots share a common time axis but have different vertical scales across the three activity conditions, with the Rest condition showing a smallest range and the Run condition showing a largest range. The substantial noise in the PPG signal during running demonstrates that high-impact activities can severely degrade the ability of PPG to capture heartbeat information.

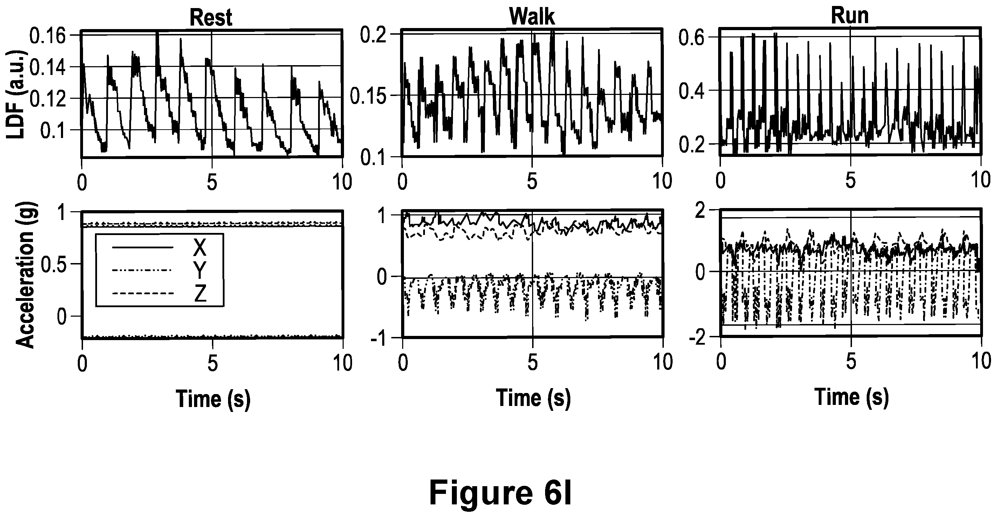

Figure 6I illustrates example motion artifacts in a LDF signal during various activities, in accordance with some embodiments. The figure comprises six graphs arranged in two rows of three columns, with each column corresponding to a different activity state: Rest, Walk, and Run. A top row displays LDF signals measured in arbitrary units over a ten-second time period, while a bottom row displays corresponding acceleration data measured in units of gravitational acceleration over the same ten-second time period. During a Rest condition, the LDF signal shows periodic oscillations between approximately 0.08 and 0.16 arbitrary units with a clear heartbeat pattern. The acceleration signals for X, Y, and Z axes remain relatively flat and stable near constant values during the Rest condition. The clear heartbeat pattern during rest demonstrates that the LDF signal can capture heartbeat information when motion is absent, similar to the PPG signal shown in Figure 6H.

With continued reference to Figure 6I, during a Walk condition, the LDF signal oscillates between approximately 0.1 and 0.2 arbitrary units with a similar periodic pattern to the Rest condition. The acceleration signals show periodic variations with a Y axis exhibiting oscillations between approximately negative one and positive one gravitational unit, and X and Z axes showing smaller variations near one gravitational unit. The LDF signal during walking maintains a periodic pattern similar to the Rest condition despite the presence of motion artifacts in the acceleration signals. This demonstrates that the LDF signal exhibits reduced motion sensitivity during walking compared to the PPG signal shown in Figure 6H, where the Walk condition showed increased amplitude variations and distortion.

As further shown in Figure 6I, during a Run condition, the LDF signal shows larger amplitude oscillations between approximately 0.2 and 0.6 arbitrary units with increased variability. The acceleration signals display larger periodic oscillations across all three axes, with a Y axis ranging between approximately negative two and positive two gravitational units, and X and Z axes showing correlated periodic variations. While the LDF signal during running shows increased variability compared to the Rest and Walk conditions, the LDF signal maintains more structure compared to the PPG signal during running shown in Figure 6H.

In some embodiments, the processed signal exhibits a thresholding behavior in which, below a motion threshold, the processed signal is robust to motion artifacts. As demonstrated by the comparison between Figures 6H and 6I, the LDF signal during the Walk condition maintains a periodic heartbeat pattern similar to the Rest condition, while the PPG signal during the Walk condition shows increased distortion. The thresholding behavior indicates that below a certain motion amplitude or velocity threshold, the LDF signal remains substantially unaffected by motion. Above the motion threshold, such as during running, motion artifacts become more pronounced in the LDF signal. However, even during running, the LDF signal shown in Figure 6I maintains more structure compared to the PPG signal shown in Figure 6H, where the heartbeat pattern is substantially obscured by noise.

In some embodiments, the thresholding behavior arises from a nonlinear relationship between motion and motion artifacts in the LDF signal. As described previously, the mechanism by which motion artifacts arise in a flow frequency band is highly nonlinear. To generate a heartbeat signal, the wearable device 102 quantifies power within a frequency band of interest. If motion artifacts fall outside this frequency band, the motion artifacts are rejected. A peak frequency of the motion artifact is linearly proportional to a peak velocity of motion of the wearable device 102 relative to the portion of the body. Thus, the wearable device 102 captures motion artifacts during violent moments of motion, such as foot contact during a running gait, while lower velocity motion during walking may produce artifacts at frequencies outside the frequency band of interest. This nonlinear relationship contributes to the thresholding behavior where the processed signal is robust to motion artifacts below the motion threshold.

The comparison between Figures 6H and 6I demonstrates advantages of LDF over PPG for heart rate monitoring during activities involving motion. During walking, the LDF signal maintains a clear heartbeat pattern while the PPG signal shows distortion. During running, both signals show increased artifacts, but the LDF signal maintains more structure. The thresholding behavior of the LDF signal enables the wearable device 102 to perform heart rate monitoring during moderate activity levels without requiring motion artifact compensation, simplifying signal processing and improving accuracy compared to PPG-based approaches.

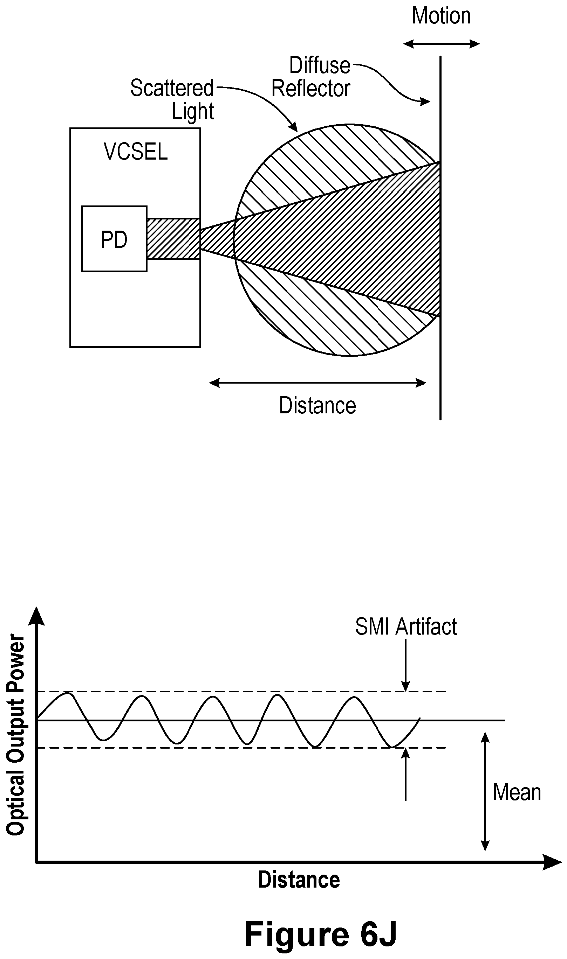

Figure 6J illustrates an SMI artifact mechanism in an LDF system, in accordance with some embodiments. As described previously, the wearable device 102 may extract, from a return optical signal, a low frequency component that encodes motion information corresponding to motion of the light emitter relative to the portion of the body. The wearable device 102 processes the return optical signal to generate a processed signal from a higher frequency component that encodes blood flow information.

Referring to Figure 6J, a top portion of the figure shows a schematic representation of an optical sensing configuration. A sensing module contains a VCSEL and a photodetector (PD) arranged adjacent to one another. The VCSEL projects an outputted optical signal toward a diffuse reflector, which represents the portion of the body of the user such as skin. The diffuse reflector is positioned at a distance from the sensing module and is capable of motion relative to the sensing module, as indicated by a bidirectional motion arrow. Scattered light from the diffuse reflector returns toward the sensing module, where a portion of the backscattered light couples back into a cavity of the VCSEL. This coupling of scattered light back into the laser cavity creates the self-mixing interferometry effect.

In some embodiments, the motion artifacts arise from SMI within the light emitter. Backscattered light couples into an optical cavity of the light emitter and modulates an output power of the light emitter. The relative phase of the backscattered light depends on the distance from the light emitter to the target. Depending on the phase of the backscattered light, varying degrees of constructive or destructive interference occur within the optical cavity. This interference results in a small variation in an efficiency of the light emitter and a corresponding small variation in the output optical power. The system (e.g., the wearable device 102) transits one full cycle of the interference effect for each half wavelength of motion relative to the target, as the light travels to the target and back.

With continued reference to Figure 6J, a bottom portion of the figure shows a graph depicting optical output power as a function of distance. The graph illustrates the SMI artifact, which manifests as a periodic modulation of the optical output power. A waveform oscillates around a mean value, with an amplitude of oscillation representing the SMI artifact magnitude. The periodic nature of the output power variation corresponds to changes in the relative phase of the backscattered light as the distance between the sensing module and the diffuse reflector changes.

In some embodiments, a relationship between the motion and the motion artifacts in the higher frequency component is nonlinear. A peak artifact frequency in the higher frequency component is proportional to a peak velocity of the motion. For a given velocity, the SMI effect results in a periodic output with a frequency shift Δf given by the laser Doppler vibrometer equation: $Δ f = \frac{2 v}{λ}$ where λ is a wavelength of the light emitter and v is a component of velocity along a beam path. The frequency shift equals 2v/λ, where v is velocity and λ is wavelength. This equation describes the Doppler shift of the reflected light and is equivalent to the equation for a laser Doppler vibrometer.

In some embodiments, the motion artifacts arise from a laser Doppler vibrometer (LDV) effect caused by a fixed reference arm in the wearable device 102. The fixed reference arm comprises at least one of a cover glass reflection, cavity scattering, or self-mixing interferometry within the light emitter. The presence of the fixed reference arm introduces sensitivity to motion between the wearable device 102 and the skin. In effect, the fixed reference arm makes the system (e.g., the wearable device 102) function as a laser Doppler vibrometer. For example, for a light emitter operating at 850 nm and a velocity of 5 mm/s, the laser Doppler vibrometer equation predicts peak interference frequencies of approximately 11.8 kHz, as the frequency shift equals 2 × 5 mm/s / 850 nm ≈ 11.8 kHz.

In some embodiments, the motion artifact frequency is proportional to an absolute value of the velocity. The wearable device 102 detects a value of the difference in frequency but not a direction of motion. This results in a doubling of a fundamental frequency of any periodic motion. For example, if the wearable device 102 is driven in a periodic motion profile with a zero-mean triangle wave velocity profile, the interference component varies in frequency proportional to the absolute value of the velocity. The SMI effect introduces motion artifacts into the LDF signal that can interfere with accurate blood flow measurements, particularly during high-velocity motion events such as running or other high-impact activities.

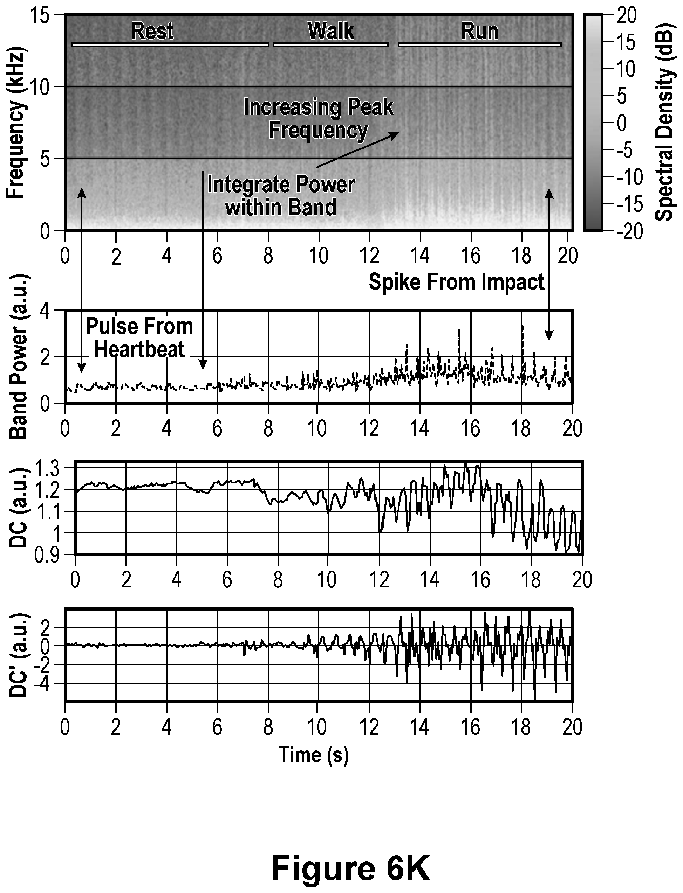

Figure 6K illustrates an example method of extracting a pulse signal from an LDF signal, in accordance with some embodiments. Figure 6K comprises four vertically stacked plots sharing a common time axis spanning from 0 to 20 seconds. The plots are divided into three activity regions labeled as Rest, Walk, and Run, with white horizontal lines demarcating these regions. The top plot displays a frequency spectrogram with frequency on a vertical axis ranging from 0 to 15 kHz and spectral density indicated by a color scale ranging from -20 dB to 20 dB. The spectrogram provides a time-frequency representation of the return optical signal, showing how frequency content varies over time across the three activity states. Annotations in the spectrogram indicate an increasing peak frequency trend during the Run period and a region where power is integrated within a band between approximately 5 kHz and 10 kHz. A spike from impact is annotated near an 18 second mark during the Run period, corresponding to a high-velocity motion event such as foot contact during a running gait.

With continued reference to Figure 6K, the spectrogram demonstrates that the low frequency component comprises frequencies below 25 Hz. The low frequency component is correlated with sensor-to-skin motion. As shown in the spectrogram, low frequency content near the bottom of the frequency axis varies with activity level, with increased low frequency energy during the Walk and Run periods compared to the Rest period. The low frequency component encodes motion information that may be used to compensate for motion artifacts in the processed signal.

As further shown in Figure 6K, the higher frequency component comprises frequencies above 1 kHz. The higher frequency component encodes blood flow velocity information. The spectrogram shows that frequency content in the range from approximately 1 kHz to 15 kHz varies with both blood flow and motion. During the Rest period, the higher frequency content exhibits a relatively stable pattern corresponding to blood flow. During the Walk and Run periods, the higher frequency content shows increased variability and the appearance of motion-induced artifacts, particularly during high-impact moments in the Run period.

A second plot in Figure 6K displays band power in arbitrary units on a vertical axis ranging from 0 to 4, showing a time series with annotations indicating pulses from heartbeat during the Rest period and a spike from impact during the Run period. The band power is computed by quantifying power of the return optical signal within a frequency band of interest. As shown in the spectrogram, the frequency band of interest spans approximately 5 kHz to 10 kHz. The wearable device 102 generates the processed signal by quantifying power within this frequency band to generate a heartbeat signal. During the Rest period, the band power exhibits periodic oscillations corresponding to the heartbeat. During the Run period, the band power shows a spike corresponding to the impact event annotated in the spectrogram.

In some embodiments, the processing circuitry is configured to reject motion artifacts that fall outside the frequency band. As described previously, the mechanism by which motion artifacts arise in the flow frequency band is highly nonlinear. If motion artifacts fall outside the frequency band of interest spanning approximately 5 kHz to 10 kHz, the motion artifacts are rejected and do not appear in the processed signal. The spectrogram in Figure 6K shows that during the Walk period, motion-induced frequency content may fall below the frequency band of interest, resulting in rejection of the motion artifacts and preservation of the heartbeat signal in the band power plot.

A third plot in Figure 6K displays a DC level in arbitrary units on a vertical axis ranging from approximately 0.9 to 1.3, showing baseline variations that increase in amplitude and frequency during the Walk and Run periods compared to the Rest period. The DC level corresponds to the low frequency component of the return optical signal. During the Rest period, the DC level remains relatively stable. During the Walk and Run periods, the DC level exhibits oscillations that correlate with the periodic motion of walking and running gaits. The DC level encodes motion information that may be used for compensating for motion artifacts in the processed signal.

A fourth plot in Figure 6K displays a derivative of the DC level in arbitrary units on a vertical axis ranging from approximately -4 to 2, showing oscillations that correlate with motion intensity across the three activity periods. The derivative of the DC level represents a rate of change of the low frequency component over time. In some embodiments, the motion artifacts comprise spiky artifacts during high-impact activities. The spiky artifacts correlate with peaks of an absolute value of a derivative of the low frequency component. As shown in Figure 6K, the derivative of the DC level exhibits sharp peaks during the Run period that correspond temporally to the spike from impact annotated in the spectrogram and the spike in the band power plot. The correlation between the derivative peaks and the motion artifacts provides a basis for using the low frequency component to compensate for motion artifacts in the processed signal.

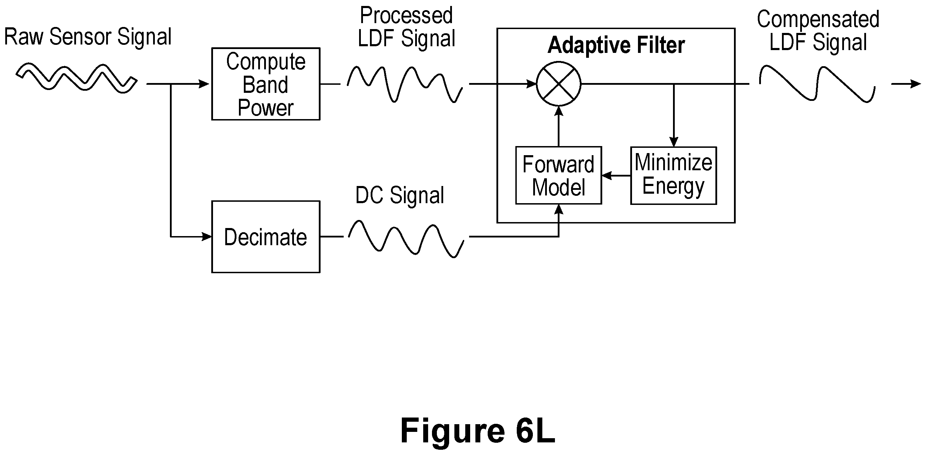

In some embodiments, the wearable device 102 compensates for motion artifacts in the processed signal using the low frequency component. As described previously with reference to Figure 6L, the wearable device 102 applies an adaptive filter that uses the low frequency component as a reference signal to estimate and remove motion artifacts from the processed signal. The DC level and derivative of the DC level shown in Figure 6K provide the motion information used by the adaptive filter. The correlation between the derivative peaks and the spiky artifacts during high-impact activities enables the adaptive filter to estimate when motion artifacts are present in the processed signal and to compensate for the artifacts accordingly.

In some embodiments, the wearable device 102 determines, based on the compensated processed signal, heart information of the user. After compensating for motion artifacts using the low frequency component, the compensated processed signal exhibits reduced motion artifact content compared to the uncompensated processed signal. The wearable device 102 extracts heart information from the compensated processed signal, such as heart rate, heart rate variability, or other cardiac metrics. The compensation using the low frequency component enables the wearable device 102 to determine heart information during activities involving motion, such as walking and running, where motion artifacts would otherwise degrade the accuracy of heart information determination.

The four plots in Figure 6K demonstrate the relationship between the low frequency component encoding motion information and the higher frequency component encoding blood flow information. The spectrogram shows the frequency distribution of the return optical signal over time, with the low frequency component below 25 Hz and the higher frequency component above 1 kHz. The band power plot shows the processed signal generated by quantifying power within the frequency band of interest from 5 kHz to 10 kHz. The DC level plot shows the low frequency component that encodes motion information correlated with sensor-to-skin motion. The derivative plot shows how peaks in the derivative of the low frequency component correlate with spiky motion artifacts during high-impact activities. Together, these plots illustrate how the wearable device 102 extracts multiple independent signals from the return optical signal and uses the low frequency component to compensate for motion artifacts in the processed signal, enabling determination of heart information during activities involving motion.

Figure 6L illustrates an example self-compensation approach for LDF signals, in accordance with some embodiments. As described previously, the wearable device 102 extracts, from the return optical signal, a low frequency component that encodes motion information corresponding to motion of the light emitter relative to the portion of the body, and processes the return optical signal to generate a processed signal from a higher frequency component that encodes blood flow information.

Figure 6L depicts a signal processing architecture configured to reduce motion artifacts in an LDF signal by using different frequency components of the signal itself. A raw sensor signal is split into two parallel processing paths. In a first path, the raw sensor signal is provided to a compute band power block that generates a processed LDF signal. The compute band power block quantifies power of the return optical signal within a frequency band to generate a heartbeat signal. As described previously, the frequency band of interest may span approximately 5 kHz to 10 kHz, corresponding to frequencies that encode blood flow velocity information. The processed LDF signal encodes blood flow information that varies with the cardiac cycle.

With continued reference to Figure 6L, in a second path, the raw sensor signal is provided to a decimate block that generates a DC signal representing low frequency baseline changes due to motion. The decimate block extracts a low frequency component from the return optical signal. The low frequency component encodes motion information corresponding to motion of the light emitter relative to the portion of the body. As described previously, the low frequency component may comprise frequencies below 25 Hz and is correlated with sensor-to-skin motion. The DC signal output from the decimate block provides a reference signal that encodes information about motion between the wearable device 102 and the skin of the user.

As further shown in Figure 6L, both the processed LDF signal and the DC signal are fed into an adaptive filter block. In some embodiments, compensating for motion artifacts comprises applying an adaptive filter that uses the low frequency component as a reference signal to estimate and remove the motion artifacts from the processed signal. The adaptive filter receives the processed LDF signal containing both blood flow information and motion artifacts, and receives the DC signal containing motion information. The adaptive filter uses the DC signal as the reference signal to identify and remove motion artifact components from the processed LDF signal.

Within the adaptive filter block shown in Figure 6L, the DC signal is provided to a forward model that estimates motion artifacts based on the low frequency motion information. In some embodiments, the adaptive filter comprises a forward model configured to estimate the motion artifacts based on the low frequency component. The forward model transforms the low frequency component into an estimated artifact signal that represents the expected motion artifact contribution to the processed LDF signal. The forward model accounts for the relationship between motion of the wearable device 102 relative to the skin and the resulting artifacts that appear in the higher frequency components encoding blood flow.

In some embodiments, the forward model computes a derivative of the low frequency component and applies a thresholding function to generate an estimated artifact signal. As described previously with reference to Figure 6K, the motion artifacts comprise spiky artifacts during high-impact activities, and the spiky artifacts correlate with peaks of an absolute value of a derivative of the low frequency component. The forward model can leverage this correlation by computing the derivative of the DC signal to identify rapid changes in the low frequency component that correspond to motion events. The thresholding function is applied to the derivative to generate the estimated artifact signal, where the thresholding function selects derivative values that exceed a threshold magnitude corresponding to motion events that produce artifacts in the processed LDF signal. The combination of derivative computation and thresholding provides an explicit form for the forward model that is interpretable and computationally efficient.

With continued reference to Figure 6L, an output of the forward model and the processed LDF signal are combined at a mixing element within the adaptive filter block. The mixing element computes a difference between the processed LDF signal and the estimated artifact signal from the forward model. The difference represents a residual signal that corresponds to the processed LDF signal with the estimated motion artifacts removed. The residual signal is provided to a minimize energy block that adjusts the forward model to minimize the energy of the residual signal.

In some embodiments, the adaptive filter minimizes energy of a residual signal corresponding to a difference between the processed signal and an estimated artifact. The minimize energy block computes the energy of the residual signal and adjusts parameters of the forward model to reduce the energy. The energy of the residual signal represents a measure of how well the estimated artifact signal matches the actual motion artifacts present in the processed LDF signal. When the estimated artifact signal accurately represents the motion artifacts, the residual signal contains primarily the blood flow information with minimal artifact content, resulting in lower energy. When the estimated artifact signal does not accurately represent the motion artifacts, the residual signal can contain both blood flow information and residual artifact content, resulting in higher energy. By adjusting the forward model to reduce the energy of the residual signal, the adaptive filter converges toward an estimated artifact signal that accurately represents the motion artifacts.

As further shown in Figure 6L, the residual signal with minimum energy corresponds to a compensated LDF signal that is output from the adaptive filter block. The compensated LDF signal represents the processed LDF signal with motion artifacts reduced or removed based on the motion information encoded in the DC signal. The wearable device 102 determines, based on the compensated LDF signal, heart information of the user. The compensated LDF signal exhibits reduced motion artifact content compared to the uncompensated processed LDF signal, enabling improved accuracy of heart information determination during activities involving motion.

In some embodiments, the wearable device 102 comprises a light emitter configured to project an outputted optical signal to a portion of a body of a user, a photodetector configured to capture a return optical signal corresponding to the outputted optical signal, and processing circuitry. The processing circuitry is configured to extract a low frequency component from the return optical signal, wherein the low frequency component encodes motion information. The processing circuitry is further configured to generate a processed signal by quantifying power of the return optical signal within a frequency band to generate a heartbeat signal. The processing circuitry is further configured to apply an adaptive filter that uses the low frequency component as a reference signal to estimate and remove motion artifacts from the processed signal. The processing circuitry is further configured to determine, based on the compensated processed signal, heart information of the user.

In some embodiments, the motion artifact compensation is performed by a trained model with no explicit forward model or objective function. Rather than using the explicit forward model comprising derivative computation and thresholding, a trained model receives the processed LDF signal and the DC signal as inputs and outputs the compensated LDF signal directly. The trained model learns the relationship between the low frequency component and the motion artifacts through training on labeled data, without requiring explicit specification of the forward model form or the energy minimization objective. The trained model may comprise a neural network, a support vector machine, or other machine-learning architecture. The trained model approach may capture more complex relationships between the low frequency component and the motion artifacts compared to the explicit forward model approach, potentially improving compensation performance in scenarios where the relationship is not well characterized by derivative computation and thresholding.

Although Figure 6L illustrates a specific self-compensation architecture with parallel processing paths and an adaptive filter, the present disclosure is not limited to this particular implementation. In some embodiments, the forward model comprises forms other than derivative computation and thresholding, such as polynomial functions, lookup tables, or parametric models of the motion artifact mechanism. In some embodiments, the energy minimization uses optimization algorithms other than those implied by the minimize energy block, such as gradient descent, least mean squares, recursive least squares, or Kalman filtering. In some embodiments, the adaptive filter architecture differs from that illustrated, such as architectures with multiple forward models, cascaded adaptive filters, or feedback connections. In some embodiments, the trained model approach uses architectures other than those described, such as recurrent neural networks, convolutional neural networks, or transformer architectures. Accordingly, the specific self-compensation architecture illustrated in Figure 6L is provided as an example and should not be construed as limiting the scope of the present disclosure, the invention being defined in the appended claims.

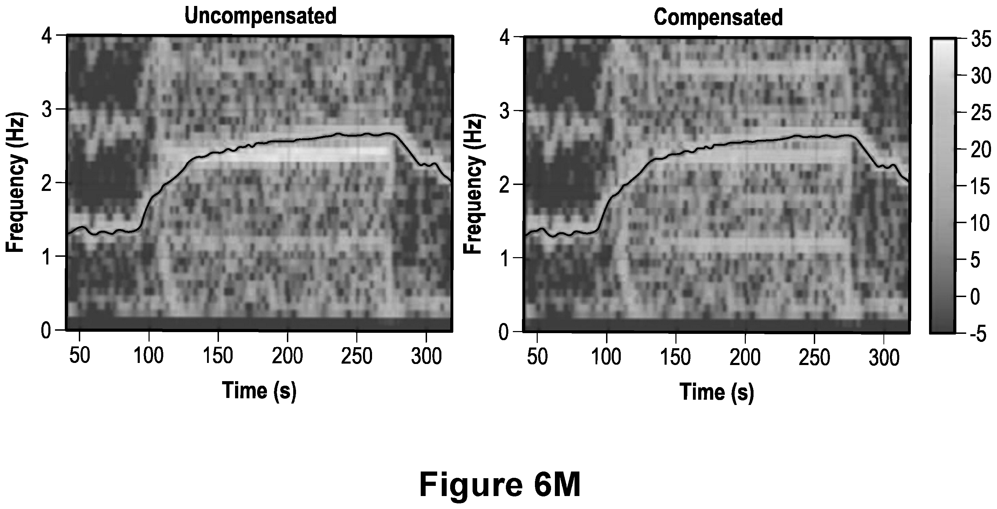

Figure 6M illustrates example spectrograms comparing uncompensated and compensated LDF signals during running, in accordance with some embodiments. As described previously, the wearable device 102 extracts, from the return optical signal, a low frequency component that encodes motion information corresponding to motion of the light emitter relative to the portion of the body, and processes the return optical signal to generate a processed signal from a higher frequency component that encodes blood flow information. The wearable device 102 compensates for motion artifacts in the processed signal using the low frequency component. Figure 6M demonstrates the effect of the self-compensation technique on reducing motion artifacts in the LDF signal during a high-impact activity.

Referring to Figure 6M, the figure includes two side-by-side spectrograms, with a left spectrogram labeled "Uncompensated" and a right spectrogram labeled "Compensated." Both spectrograms display frequency on a vertical axis ranging from 0 to 4 Hz and time on a horizontal axis ranging from approximately 50 to 300 seconds. A color scale on a right side of the figure indicates signal intensity values ranging from approximately -5 to 35. Each spectrogram includes a red curve that tracks a frequency component over time, showing an initial rise from approximately 1.5 Hz to approximately 2.5 Hz, followed by a plateau period, and then a decline back toward lower frequencies. The red curve represents a heartbeat frequency that varies over time as the user transitions through different activity levels.

With continued reference to Figure 6M, in the uncompensated spectrogram, a bright horizontal band appears at approximately 2.4 Hz during the plateau period. The bright horizontal band represents a motion artifact component corresponding to a motion fundamental frequency during running. The motion fundamental at approximately 2.4 Hz corresponds to a cadence of the running gait, where each foot strike produces a motion event that generates artifacts in the LDF signal. The motion fundamental appears as a bright horizontal band because the running cadence remains relatively constant during the plateau period, producing artifacts at a consistent frequency. The presence of the motion fundamental at approximately 2.4 Hz in the uncompensated spectrogram can make it difficult to resolve and track the heartbeat signal, particularly when the heartbeat frequency approaches the motion fundamental frequency.

As further shown in Figure 6M, in the compensated spectrogram, the motion artifact component at approximately 2.4 Hz is attenuated compared to the uncompensated version. The attenuation of the motion fundamental demonstrates the effect of the self-compensation technique on reducing motion artifacts in the LDF signal. By using the low frequency component as a reference signal to estimate and remove motion artifacts from the processed signal, the adaptive filter reduces the intensity of the motion fundamental in the compensated spectrogram. The attenuation of the motion fundamental makes it easier to resolve and track the heartbeat signal, as the heartbeat frequency component is no longer obscured by the bright horizontal band corresponding to the motion artifact.

In some embodiments, the compensated spectrogram shows additional horizontal bands at lower frequencies around 1.2 Hz that may represent harmonics introduced by the compensation process. The compensation process may introduce artifacts at harmonic frequencies of the motion fundamental, such as 0.5X and 1.5X harmonics where the fundamental is at 0.5X of the apparent motion frequency. While the introduction of harmonic artifacts is not ideal, the harmonic artifacts are at frequencies farther separated from the heartbeat frequency compared to the motion fundamental, making the harmonic artifacts more acceptable for heart rate tracking purposes. The tradeoff between attenuating the motion fundamental and introducing harmonic artifacts may be adjusted by tuning parameters of the forward model or the adaptive filter.

In some embodiments, the wearable device 102 does not require a separate accelerometer for motion artifact compensation. As described previously, the low frequency component and the processed signal are derived from a same optical signal captured by the photodetector. The self-compensation approach uses different frequency components of the return optical signal itself to perform motion artifact compensation, rather than relying on a separate motion sensor such as an accelerometer. The low frequency component encoding motion information and the higher frequency component encoding blood flow information are both extracted from the return optical signal captured by the photodetector. Because both components are derived from the same optical signal, the wearable device 102 can perform motion artifact compensation without requiring data from a separate motion sensor external to a module comprising the light emitter and the photodetector.

In some embodiments, a non-transitory computer-readable storage medium stores instructions that, when executed by one or more processors of a wearable device (e.g., the wearable device 102) comprising a light emitter and a photodetector, cause the wearable device to perform operations for motion artifact compensation. The wearable device projects, from the light emitter, an outputted optical signal to a portion of a body of a user. The wearable device captures, by the photodetector, a return optical signal corresponding to the outputted optical signal. The wearable device extracts multiple independent time series from the return optical signal by probing different portions of a frequency spectrum of the return optical signal. A first time series comprises a low frequency component encoding motion information and a second time series comprises a higher frequency component encoding blood flow information. The wearable device uses the first time series to compensate for motion artifacts in the second time series. The wearable device determines, based on the compensated second time series, heart information of the user.

In some embodiments, the light emitter comprises a single laser and the photodetector comprises a single photodiode operating at a single wavelength. The multiple independent time series are extracted from the return optical signal without requiring additional optical elements. As described previously, LDF differs from PPG in the ability to capture multiple independent signals from the exact same optical elements. In a simplest implementation, the wearable device has a single source comprising the laser and a single detector comprising the photodiode operating at only one wavelength. In this configuration, PPG would provide only a single time series. However, in the case of LDF, the wearable device extracts multiple independent time series by varying which part of a flow spectrum is probed. The low frequency component below 25 Hz encodes motion information correlated with sensor-to-skin motion, while the higher frequency component above 1 kHz encodes blood flow velocity information. The extraction of multiple independent time series from a single optical signal captured by a single photodiode enables the self-compensation approach without requiring additional optical elements such as multiple light emitters, multiple photodetectors, or multiple wavelengths.

In some embodiments, the operations are performed on a module comprising the light emitter and the photodetector. The heart information is determined without requiring data from a separate motion sensor external to the module. The self-compensation approach enables the module to perform motion artifact compensation using only signals derived from the return optical signal captured by the photodetector within the module. The module does not require an interface to an external accelerometer or other motion sensor, simplifying system architecture and reducing the number of components that must be integrated into the wearable device. The elimination of the separate accelerometer for motion artifact compensation reduces hardware complexity, lowers power consumption, and achieves a more compact form factor compared to approaches that rely on external motion sensors for compensation.

In some embodiments, the heart information comprises heart rate information determined in accordance with principles of LDF. As described previously, LDF measures flow of blood, where velocity is approximately encoded in a frequency distribution with volume given by signal power. The heart rate information is extracted from the compensated second time series, which represents the higher frequency component encoding blood flow information with motion artifacts reduced or removed. The self-compensation approach enables the wearable device to determine heart rate information during activities involving motion, such as running, where motion artifacts would otherwise degrade the accuracy of heart rate determination.

The comparison between the uncompensated and compensated spectrograms in Figure 6M demonstrates the effectiveness of the self-compensation technique for reducing motion artifacts during high-impact activities. The motion fundamental at approximately 2.4 Hz is strongly attenuated in the compensated spectrogram, making it easier to resolve and track the heartbeat signal represented by the red curve. The self-compensation approach achieves this attenuation without requiring a separate accelerometer, as both the motion information in the low frequency component and the blood flow information in the higher frequency component are derived from the same optical signal captured by the photodetector. The ability to perform motion artifact compensation using only signals from the LDF module simplifies system integration and enables embedding of the heart rate algorithm on the LDF module itself, as data from a separate sensor is no longer needed.

Figure 7 illustrates a flow diagram of a method of operating the wearable device 102 for biometric data monitoring, in accordance with some embodiments. Operations (e.g., steps) of the method 700 can be performed by one or more processors (e.g., central processing unit and/or MCU) of the wearable device 102. At least some of the operations shown in Figure 7 correspond to instructions stored in a computer memory or a non-transitory computer-readable storage medium (e.g., storage, RAM, and/or memory) of the wearable device 102. Operations of the method 700 can be performed by a single device (e.g., a head-wearable device) alone or in conjunction with one or more processors and/or hardware components of another communicatively coupled device (e.g., a handheld intermediary processing device (HIPD) 842) and/or instructions stored in memory or computer-readable medium of the other device communicatively coupled to of the wearable device 102. In some embodiments, the various operations of the methods described herein are interchangeable and/or optional, and respective operations of the methods are performed by any of the aforementioned devices, systems, or combination of devices and/or systems. For convenience, the method operations will be described below as being performed by wearable device 102, but should not be construed as limiting the performance of the operation to wearable device 102 in all embodiments.

(A1) The method 700 is performed at a wearable device (e.g., the wearable device 102) with a light emitter (e.g., light emitter 104, one or more of the plurality of light emitters 104'), a photodetector (e.g., photodetectors 106, one or more of the plurality of photodetectors 106'), an analog integrated circuit (IC) comprising a high-pass filter (e.g., high-pass filter 208, high-pass filter 304, or high-pass filter 404), a rectifier (e.g., rectifier 210, rectifier 306, rectifier 406), and a low-pass filter (e.g., low-pass filter 212, low-pass filter 308, or low-pass filter 408), and one or more processors. The method 700 includes projecting (702), from the light emitter, an outputted optical signal (e.g., outputted optical signal 110) to a portion of a body of a user. The method 700 further includes capturing (704), by the photodetector, a return optical signal (e.g., return optical signal 112) corresponding to the outputted optical signal. The method 700 further includes processing (706) the return optical signal in analog domain (e.g., using the analog IC 109). Execution of processing the return optical signal in analog domain includes generating (708) a filtered optical signal (e.g., filtered optical signal 238, filtered optical signal 238-1, filtered optical signal 238-2, or output signal "VHPF") by applying the high-pass filter (e.g., high-pass filter 208, high-pass filter 304, high-pass filter 404) to remove non-heart information from the return optical signal; generating (710) a rectified optical signal (e.g., rectified optical signal 240, rectified optical signal 240-1, rectified optical signal 240-2, or output signal "VHWR") by applying the rectifier (e.g., rectifier 210, rectifier 306, or rectifier 406) to rectify the filtered optical signal (e.g., filtered optical signal 238, filtered optical signal 238-1, filtered optical signal 238-2, or output signal "VHPF"); and generating (712) a time-averaged optical signal (e.g., time-averaged optical signal 242, time-averaged optical signal 242-1, time-averaged optical signal 242-2, or output signal "VOUT") by applying the low-pass filter (e.g., low-pass filter 212, low-pass filter 308, low-pass filter 408) to the rectified optical signal (e.g., rectified optical signal 240, rectified optical signal 240-1, rectified optical signal 240-2, output signal "VHWR"). The method 700 further includes determining (714), based on the time-averaged optical signal (e.g., time-averaged optical signal 242, time-averaged optical signal 242-1, time-averaged optical signal 242-2, or output signal "VOUT"), heart information of the user, e.g., using a system chip 116 (or a computing unit exterior to and communicatively coupled to the wearable device 102) to perform data analysis. The method 700 further includes causing (716) the heart information to be presented to the user. In some embodiments, the light emitter, the photodetector, and the analog IC are part of a sensing unit (e.g., sensing unit 108 or sensing unit 202). In some embodiments, the sensing unit (e.g., sensing unit 108, sensing unit 202) is part of a system chip. In some embodiments, the wearable device (e.g. wearable device 102) is configured to generate, based on the return optical signal, a filtered return optical signal prior to the return optical signal being captured by the photodetector. In some embodiments, the wearable device (e.g. wearable device 102) is configured to generate, based on the return optical signal or the filtered return optical signal, a detected optical signal (e.g., detected optical signal 234-1 or detected optical signal 234-2) by applying the photodetector to create a photocurrent for subsequent signal processing.

(A2) In some embodiments of A1, the photodetector is a first photodetector (e.g., a near photodiode). The wearable device (e.g. wearable device 102) further includes a second photodetector (e.g., a far photodiode). The first photodetector and the second photodetector are arranged to be at different distances (e.g., distance d1 and distance d2) from the portion of the body while the wearable device (e.g. wearable device 102) is being worn, e.g., in a dual-channel configuration 130B. The return optical signal comprises a first return optical signal detected by the first photodetector and a second return optical signal detected by the second photodetector. Execution of processing the return optical signal in the analog domain further comprises processing the first return optical signal in a first channel (e.g., signal processing channel 204-1) in the analog domain; and processing the second return optical signal in a second channel (e.g., signal processing channel 204-2) in the analog domain. In some embodiments, the wearable device (e.g. wearable device 102) is configured to operate in a single-channel configuration or a multi-channel configuration. In some embodiments, the first photodetector and the second photodetector are arranged at the same distance from the portion of the body. In some embodiments, the light emitter in the dual-channel configuration comprises a first light emitter and a second light emitter, and each light emitter is paired with one of the first photodetector and the second photodetector.

(A3) In some embodiments of any of A1-A2, projecting the outputted optical signal (e.g., at a sampling frequency), capturing, the return optical signal corresponding to the outputted optical signal, processing the return optical signal in analog domain, and determining the heart information of the user (e.g., using the system chip 116) are performed in accordance with principles of LDF, e.g., the return optical signal is scattered from the portion of the body of the user and exhibits a small shift compared with the sampling frequency. In some embodiments, the small shift comprises a Doppler shift induced by blood flow in the portion of the body. In some embodiments, the return optical signal comprises a Doppler-shifted component and an unshifted component that interfere at the photodetector to produce a beat frequency corresponding to blood flow velocity.

(A4) In some embodiments of any of A1-A3, the return optical signal comprises a plurality of flow-induced optical frequency shifts in a range from 1 kHz to 50 kHz. In some embodiments, the frequencies are in a range from 4 kHz to 25 kHz or 8 kHz to 25 kHz. In some embodiments, a target frequency for heart rate detection is 25 kHz.

(A5) In some embodiments of any of A1-A4, execution of processing the return optical signal comprises applying a transimpedance amplifier (e.g., transimpedance amplifier 206, transimpedance amplifier 302, or transimpedance amplifier 402) to amplify the return optical signal prior to generating the filtered optical signal (e.g., filtered optical signal 238, filtered optical signal 238-1, filtered optical signal 238-2, or output signal "VHPF"). In some embodiments, the transimpedance amplifier (e.g., transimpedance amplifier 206, transimpedance amplifier 302, or transimpedance amplifier 402) is configured to generate, based on return optical signal a converted optical signal (e.g., converted optical signal 236-1 or converted optical signal 236-2). In some embodiments, the transimpedance amplifier has a gain in a range of 1×105 V/A to 1×107 V/A. In some embodiments, the transimpedance amplifier has a bandwidth in the range of 8 kHz to 40 kHz.

(A6) In some embodiments of any of A1-A5, the high-pass filter (e.g., high-pass filter 304 or high-pass filter 404) has a cutoff frequency in a range from 1 kHz to 10 kHz. In some embodiments, the cutoff frequency is 5 kHz. In some embodiments, the cutoff frequency is selected based on the portion of the body of the user (e.g., based on a tissue type, a blood vessel size, and/or other characteristic of the portion of the body).

(A7) In some embodiments of any of A1-A6, the high-pass filter (e.g., high-pass filter 304) is a second-order high-pass filter. In some embodiments, the high-pass filter (e.g., high-pass filter 304) is an active circuit comprising one or more amplifiers. In some embodiments, the high-pass filter has an order greater than two. In some embodiments, the high-pass filter is a passive circuit comprising resistor components and capacitor components.

(A8) In some embodiments of any of A1-A7, the rectifier comprises (e.g., rectifier 306 or rectifier 406) a non-linear electrical component (e.g., diode "D1" and diode "D2" of the rectifier 306 or diode "D1" and diode "D2" of the rectifier 406). In some embodiments, the rectifier is a half-wave rectifier configured to pass only a positive or negative portion of the filtered optical signal. In some embodiments, the rectifier is a full-wave rectifier configured to convert both positive and negative portions of the filtered optical signal to a single polarity. In some embodiments, the rectifier operates at a voltage that is no greater than 1 V. In some embodiments, the rectifier has a bandwidth substantially similar to or greater than that of the filtered optical signal.

(A9) In some embodiments of any of A1-A8, the low-pass filter (e.g., low-pass filter 308 or low-pass filter 408) is a second-order low-pass filter. In some embodiments, the low-pass filter (e.g., low-pass filter 308 or low-pass filter 408) is an active circuit comprising one or more amplifiers. In some embodiments, the low-pass filter has an order greater than two. In some embodiments, the low-pass filter is a passive circuit comprising resistor components and capacitor components. In some embodiments, the low-pass filter has a cutoff frequency in the range of 1 Hz to 50 Hz. In some embodiments, the cutoff frequency of the low-pass filter is determined to be no greater than 200 Hz, which is sufficient to capture heart-induced vibrations.

(A10) In some embodiments of any of A1-A9, execution of processing the return optical signal in analog domain further comprises generating an amplified optical signal (e.g., amplified optical signal 244-1, amplified optical signal 244-2) by applying a programmable gain amplifier, to amplify the time-averaged optical signal (e.g., time-averaged optical signal 242, time-averaged optical signal 242-1, time-averaged optical signal 242-2, or output signal "VOUT"). In some embodiments, the programmable gain amplifier is configured such that the amplified optical signal is within an input voltage range of an analog-to-digital converter (ADC). In some embodiments, the programmable gain amplifier has a tunable gain that is adjustable based on signal strength of the time-averaged optical signal.

(A11) In some embodiments of any of A1-A10, the heart information is presented to the user via a graphical user interface on the wearable device (e.g., wearable device 102). In some embodiments, the graphical user interface corresponds to an application executing at the wearable device (e.g., wearable device 102). In some embodiments, the heart information is presented to the user 101 via a display that is communicatively coupled to the wearable device (e.g., wearable device 102). In some embodiments, the heart information is presented to the user 101 via a display of the wearable device (e.g., wearable device 102). In some embodiments, the heart information is presented to the user via an audio output of the wearable device (e.g. wearable device 102). In some embodiments, the heart information is presented to the user via a haptic output of the wearable device (e.g. wearable device 102). In some embodiments, the heart information is transmitted to a remote device for presentation to the user or a third party.

(A12) In some embodiments of any of A1-A11, the wearable device (e.g. wearable device 102) comprises an extended-reality headset (e.g., a MR headset or a pair of AR glasses as two examples in reference to Figures 8A-8C). In some embodiments, the wearable device (e.g. wearable device 102) comprises a wrist-wearable device. In some embodiments, the wearable device (e.g. wearable device 102) comprises earbuds. In some embodiments, the wearable device (e.g. wearable device 102) comprises a smart ring, a smart patch, or a smart textile-based garment.

(A13) In some embodiments of any of A1-A12, the light emitter (e.g., light emitter 104, light emitter 104-1, light emitter 104-2, etc.) and the photodetector (e.g., photodetector 106, photodetector 106-1, photodetector 106-2, etc.) are assembled on a same circuit board (e.g., a PCB). In some embodiments, the circuit board is part of the sensing unit (e.g., sensing unit 108 or sensing unit 202). In some embodiments, the light emitter and the photodetector are positioned adjacent to one another on the same circuit board. In some embodiments, the light emitter, the photodetector, and the analog IC are assembled on the same circuit board. In some embodiments, the light emitter and the photodetector are arranged on different circuit boards.

(A14) In some embodiments of any of A1-A13, the light emitter (e.g., light emitter 104, light emitter 104-1, light emitter 104-2, etc.) comprises a VCSEL. In some embodiments, the light emitter (e.g., light emitter 104, light emitter 104-1, light emitter 104-2, etc.) includes VCSELs arranged in an M1×M2 pixel array. In some embodiments, the light emitter comprises an edge-emitting laser (EEL). In some embodiments, the light emitter comprises a superluminescent diode (SLD). In some embodiments, the light emitter comprises a light-emitting diode (LED) or other partially coherent light source.

(A15) In some embodiments of any of A1-A14, the light emitter (e.g., light emitter 104, light emitter 104-1, light emitter 104-2, etc.) is configured to operate in a near-infrared band. In some embodiments, the light emitter (e.g., light emitter 104, light emitter 104-1, light emitter 104-2, etc.) is configured to emit light at a wavelength (e.g., nominal emission wavelength) of approximately 850 nm (e.g., within 10%, 5%, 2%, or 1% of 850 nm). In some embodiments, the light emitter (e.g., light emitter 104, light emitter 104-1, light emitter 104-2, etc.) is configured to emit light at a wavelength (e.g., nominal emission wavelength) of approximately 940 nm (e.g., within 10%, 5%, 2%, or 1% of 940 nm). In some embodiments, the light emitter is configured to emit light at a visible wavelength or a short-wave infrared (SWIR) wavelength. In some embodiments, the wavelength of the light emitter is selected based on tissue penetration depth, eye safety considerations, or compatibility with other optical systems of the wearable device (e.g. wearable device 102).

(A16) In some embodiments of any of A1-A15, the light emitter (or at least a subset of the plurality of light emitters 104' including light emitter 104-1 to 104-m) is positioned such that an emission axis of the light emitter is normal to a surface of the portion of the body while the wearable device (e.g. wearable device 102) is being worn by the user. In some embodiments, the emission axis of the light emitter is oriented at an oblique angle relative to the surface of the portion of the body. In some embodiments, the normal positioning enhances efficiency of optical signal transmission through the surface with minimized angular distortion and scattering effects. In some embodiments, implementing normal incidence simplifies optical calibration of the light emitter across different portions of a body of a single user and across different users.

(A17) In some embodiments of any of A1-A16, the light emitter (e.g., light emitter 104, light emitter 104-1, light emitter 104-2, etc.) comprises a laser component configured to emit light having a coherence length greater than a distance (e.g., distance d1, distance d2) between the photodetector (e.g., photodetector 106, photodetector 106-1, photodetector 106-2, etc.) and the portion of the body. In some embodiments, the distance between the photodetector and the portion of the body is in the range of 1 cm to 5 cm while the wearable device (e.g. wearable device 102) is worn by the user. In some embodiments, the light emitter is configured to have a narrow emission linewidth corresponding to the coherence length, thereby enhancing measurement precision in an interference-based optical system.

(A18) In some embodiments of any of A1-A17, a driving circuit (e.g., driving circuit 600) for the light emitter (e.g., light emitter 104, light emitter 104-1, light emitter 104-2, etc.) is configured to operate without a feedback loop. In some embodiments, the driving circuit operates without automatic power control (APC), which allows the non-contact measurement system 100 to operate with only minimal modifications to existing control logic. In some embodiments, the driving circuit operates with APC. In some embodiments, the driving circuit includes short-circuit fault current protection to protect the light emitter from accidental short conditions caused by overbiasing or overheating.

(A19) In some embodiments of any of A1-A18, the photodetector (e.g., photodetector 106, photodetector 106-1, photodetector 106-2, etc.) comprises a silicon (Si) photodiode. In some embodiments, the photodetector (e.g., photodetector 106, photodetector 106-1, photodetector 106-2, etc.) comprises a gallium arsenide (GaAs) photodiode. In some embodiments, the photodetector (e.g., photodetector 106) comprises a plurality of photodiodes (e.g., photodetector 106-1, photodetector 106-2, etc.) arranged in an N1×N2 pixel array. In some embodiments, the photodetector comprises an avalanche photodiode (APD) or a single-photon avalanche diode (SPAD). In some embodiments, the photodetector is configured with an infrared bandpass filter to filter the return optical signal prior to detection.

(A20) In some embodiments of any of A1-A19, the portion of the body comprises a face of the user. In some embodiments, the portion of the body comprises a forehead, a temple, or a cheek of the user. In some embodiments, the portion of the body comprises a wrist of the user. In some embodiments, the portion of the body comprises an ear of the user. In some embodiments, the portion of the body comprises any region with sufficient blood perfusion for detecting flow-induced optical frequency shifts.

(A21) In some embodiments of any of A1-A20, the heart information comprises heart rate information. In some embodiments, the heart information comprises heart rate information, heartbeat (e.g., arrhythmia) information, and/or other types of heart-related information (e.g., blood pressure information). In some embodiments, the heart information comprises heart rate variability information. In some embodiments, the heart information comprises microvasculature information including components at a beat frequency on the order of 10 kHz. In some embodiments, the heart information comprises a cardiac pulse waveform from which pulse morphology features are extracted.

(A22) In some embodiments of any of A1-A21, the heart information comprises microvasculature information. In some embodiments the microvasculature information includes components at a beat frequency on the order of 10 kHz. In some embodiments, the microvasculature information encodes blood flow velocity in superficial capillary beds and arterioles. In some embodiments, the microvasculature information is used to assess peripheral vascular health or detect changes in microcirculatory perfusion. In some embodiments, the microvasculature information is derived from a frequency distribution of the return optical signal, wherein signal power within the frequency distribution corresponds to blood volume and frequency corresponds to blood flow velocity. In some embodiments, the microvasculature information is used in combination with heart rate information to provide a more comprehensive assessment of cardiovascular function.

In another aspect, a computing system (e.g., comprising one or more devices) is configured to perform operations corresponding to any of the methods described herein (e.g., A1-A22). In another aspect, a non-transitory computer-readable storage medium stores instructions that, when executed by a computing system, cause the computing system to perform operations corresponding to any of the methods described herein (e.g., A1-A22).

The devices and systems described above are further detailed below, including wrist-wearable devices, headset devices, systems, and haptic feedback devices. Specific operations described above may occur as a result of specific hardware, such hardware is described in further detail below. The devices described below are not limiting and features on these devices can be removed or additional features can be added to these devices.

### Positional Disparity Correction

Various embodiments of this application include methods, devices, and systems described for correcting (e.g., compensating for) positional disparity induced by motion artifacts during LDF at a wearable device (e.g., any of the wearable devices described herein). Under nominal conditions, in the absence of blood flow, a light emitter emits light to a user's body and a back-scattered light in response to the emitted light exhibits no differential shift (e.g., flow-induced optical frequency shift). However, in some circumstances, user motion (e.g., during high-motion activities) may cause the back-scattered light to re-couple into the light emitter's optical cavity, which results in modulation shifts in amplitude and/or frequency of the emitted light due to the self-mixing effect. Accordingly, wearable devices described herein may perform a compensation process to adjust the emitted light, thereby suppressing the self-mixing effect induced by the user motion.

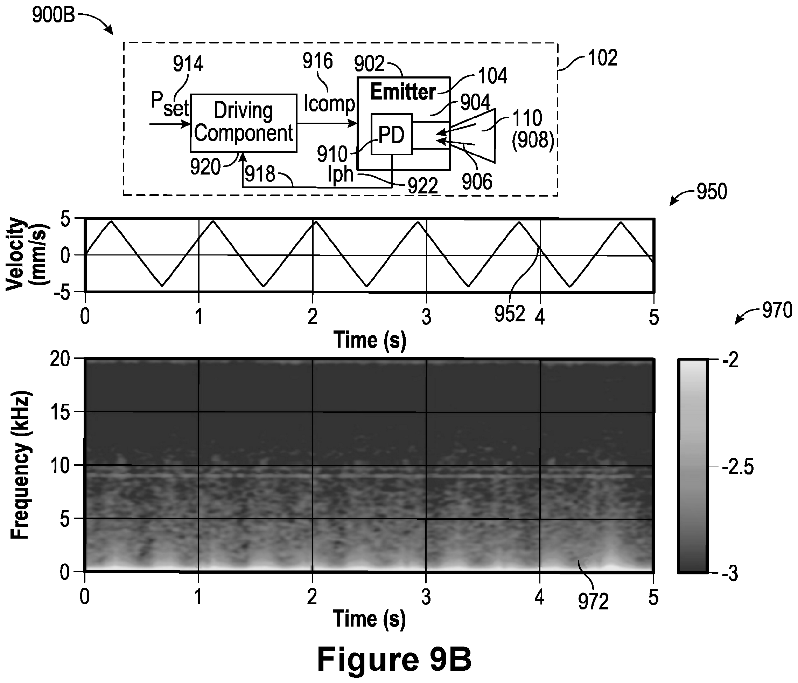

Figures 9A and 9B illustrate example configurations 900A and 900B for controlling the light emitter 104 of the wearable device 102, in accordance with some embodiments. In some embodiments, the configuration 900A is an open-loop configuration. In some embodiments, the configuration 900B is a closed-loop configuration, e.g., a closed-loop compensation for eliminating the self-mixing effect. In some embodiments, the wearable device 102 comprises an extended-reality headset or a wrist-wearable device.

In some embodiments, the wearable device 102 comprises a light emitting component 902 (e.g., a laser module) that is part of a sensing unit (e.g., sensing unit 108 or sensing unit 202). In some embodiments, the light emitting component 902 comprises the light emitter 104. In some embodiments, the light emitter 104 is contained (e.g., disposed) in a cavity 904 (e.g., an optical cavity). In some embodiments, the light emitter 104 is configured to, at a current moment, project (e.g., emit) the outputted optical signal 110 from the cavity 904 to a portion of the body 120 of the user 101 (e.g., in reference to Figure 1A). After the projection (e.g., emission), a portion of the outputted optical signal 110 is scattered and back-reflected from the portion of the body 120, forming a scattered light signal 906. The scattered light signal 906 is re-coupled into the cavity 904, and a re-coupled portion (e.g., the scattered light signal 906 or at least a portion of the scattered light signal 906) interferes with intracavity optical fields and induces modulation shifts in amplitude and/or frequency due to the self-mixing effect. For example, at a subsequent moment following the re-coupling, the light emitter 104 projects a modulated optical signal 908 that indicates changes in optical power and/or frequency caused by interference with the re-coupled portion. In some embodiments, the optical power of the modulated optical signal 908 at the subsequent moment is different from the optical power of the outputted optical signal 110 at the current moment. In some embodiments, the current moment and the subsequent moment occur nearly instantaneously, as the self-mixing effect takes place in real time within the cavity 904. In some embodiments, the cavity 904 is a laser cavity. In some embodiments, the cavity 904 functions as a fixed reference arm, e.g., with respect to the portion of the body 120 when the user who wears the wearable device 102 is in motion.

In some embodiments, the wearable device 102 comprises a photodetector 910 configured to capture (e.g., detect) the modulated optical signal 908 corresponding to the outputted optical signal 110. As discussed, the modulated optical signal 908 is generated by the light emitter 104 based on the re-coupled portion resulting from the scattered light signal 906. In some embodiments, the photodetector 910 is configured to generate a photocurrent 922 based on the detected optical power of the modulated optical signal 908.

In some embodiments, the wearable device 102 comprises a driving component 920 (e.g., illustrated in Figures 9A and 9B). In some embodiments, the driving component 920 comprises the driver 220 (e.g., the laser driver illustrated in Figure 2). In some embodiments, the driving component 920 is configured to supply a driving current to drive the light emitter 104 based on a set power 914 (e.g., a constant power "Pset"). In some embodiments, the set power 914 is provided to the driving component 920 from the wearable device 102. In some embodiments, the driving component 920 is part of a sensing unit (e.g., sensing unit 108 or sensing unit 202).

In some embodiments, the wearable device 102 comprises a feedback loop 918 (e.g., illustrated in Figure 9B) that electrically couples an output of the photodetector 910 to a respective input of the driving component 920, such that a compensation can be performed to eliminate self-mixing effect. In some embodiments, the respective input of the driving component 920 receives the generated photocurrent from the photodetector 910. In some embodiments, the driving component 920 is configured to supply the driving current to drive the light emitter 104 based on both the set power 914 and the photocurrent 922.

In some embodiments, the wearable device 102 implements the configuration 900A (e.g., an open-loop configuration), in which the light emitting component 902 operates in a constant current mode. In this configuration, the driving component 920 is configured to drive the light emitter 104 via a set current 912 (e.g., a constant current "Iset") in the absence of feedback. For example, when a movement 952 (e.g., a high motion activity similar to running with a frequency between 0.5 Hz and 1.0 Hz, as illustrated in plot 950) is induced to a target reflector (e.g., the portion of the body 120 of the user 101), the scattered light signal 906, e.g., a portion of the outputted optical signal 110, is back-reflected from the target reflector and re-coupled into the cavity 904. This re-coupling triggers a modulation shift in amplitude and/or frequency of the outputted optical signal 110 due to the self-mixing effect, thereby generating the modulated optical signal 908 with a corresponding change in optical power. Accordingly, the return optical signal 112 (e.g., in reference to Figures 1A-2), which corresponds to the modulated optical signal 908, may exhibit an interference pattern 962 (e.g., as illustrated in plot 960) resulting from the modulation shift. Stated another way, the output power of the light emitter 104 is modulated as a result of the self-mixing effect. Since there is no feedback in the configuration 900A, the driving component 920 does not receive information about any change in the optical power of the outputted optical signal 110. In this circumstance, the driving current of the light emitter 104 remains fixed at the set current 912, and the wearable device 102 does not adjust the driving current of the light emitter 104 in response to movement-induced variations in the outputted optical signal 110 (e.g., caused by the movement 952).

Turning to Figure 9B, in some embodiments, the wearable device 102 implements the configuration 900B (e.g., a closed-loop configuration for compensating the self-mixing effect), in which the light emitting component 902 operates under a constant power mode. In this configuration, the driving component 920 is configured to drive the light emitter 104 via a compensated current 916 (e.g., a compensated current "Icomp") with the feedback loop 918. In some embodiments, the driving component 920 is configured to determine, based on the modulated optical signal 908 that is captured by the photodetector 910, a positional disparity between the light emitter 104 and the portion of the body 120 of the user 101. The driving component 920 is further configured to, in accordance with a determination that the positional disparity satisfies disparity correction criteria, adjust the driving current of the light emitter 104 to generate the compensated current 916 that compensate movement-induced variations in the outputted optical signal 110 caused by the positional disparity. For example, when a movement 952 (e.g., a high motion activity similar to running with a frequency between 0.5 Hz and 1.0 Hz, as illustrated in plot 950) is induced to a target reflector (e.g., the portion of the body 120 of the user 101), the scattered light signal 906, e.g., a portion of the outputted optical signal 110, is back-reflected from the target reflector and re-coupled into the cavity 904. This re-coupling induces a modulation shift in amplitude and/or frequency of the outputted optical signal 110 as a result of the self-mixing effect, thereby generating the modulated optical signal 908 with a corresponding change in optical power (e.g., modulation shift) with respect to the outputted optical signal 110. Accordingly, the photodetector 910 captures the modulated optical signal 908 and generates a corresponding photocurrent (e.g., the photocurrent 922). The driving component 920 receives the corresponding photocurrent from the photodetector 910 through the feedback loop 918. In response, the driving component 920 adjusts the driving current of the light emitter 104 based on the set power 914 and the corresponding photocurrent and generates the compensated current 916 for compensating the corresponding change in optical power. In this circumstance, the interference pattern 962 (e.g., as illustrated in the plot 960) induced by the movement 952 is substantially reduced to an interference pattern 972 (e.g., as illustrated in plot 970).

In some embodiments, the driving component 920 comprises a controlling unit (e.g., a chip having a laser controller and/or a computing unit, a SoC, etc.) electrically coupled to the light emitter 104. In some embodiments, the controlling unit is configured to determine, based on the set power 914 and the photocurrent 922, a positional disparity between the light emitter 104 and the portion of the body 120 of the user 101. The controlling unit is further configured to compare the positional disparity to disparity correction criteria (e.g., a target set point). In some embodiments, the controlling unit comprises operational amplifier(s) and switch(es) (e.g., transistor(s)) controlled by the operational amplifier(s).

In some embodiments, the light emitter 104, the feedback loop 918, and the driving component 920 form a closed-loop control system. In some embodiments, bandwidth of the closed loop control system is set above the frequency of the self-mixing artifacts (e.g., corresponding to the interference patterns 962 and 972 in Figures 9A and 9B).

In some embodiments, the photodetector 910 is positioned (e.g., disposed) within the cavity 904 of the light emitter 104. For example, the photodetector 910 may be integrated into the cavity 904 of the light emitter 104, such that the photodetector 910 is part of a compact assembly for both light emission and light detection. In some circumstances, this integrated configuration can enhance sensitivity and responsiveness of the photodetector 910 for performing the compensation. In some embodiments, the photodetector 910 is positioned exterior to the cavity 904. This configuration may provide flexibility in designing and integrating the photodetector 910, as the light emitter 104 and photodetector 910 can be fabricated using separate manufacturing processes. For example, the photodetector 910 may be optimized for specific performance requirements including sensitivity (e.g., responsivity), wavelength-dependent absorption profile, and/or power consumption. In some embodiments, the photodetector 910 is positioned in proximity to the light emitter 104. For example, when the photodetector 910 is positioned within the cavity 904 of the light emitter 104, an absorption region of the photodetector 910 may be positioned in proximity to one or more optical cavity modes of the light emitter 104 for enhancing its interaction with resonant optical fields. In another example, when the photodetector 910 is positioned exterior to the cavity 904, close proximity between the photodetector 910 and the light emitter 104 may increase the portion of emitted light from the light emitter 104 that is captured by the photodetector 910.

In some embodiments, the positional disparity is induced by a motion (e.g., movement) of the user 101 who wears the wearable device 102. In some embodiments, the motion of the user 101 varies in real time and include low, medium, and/or high levels of motion, which result in different degrees of the positional disparity. In some embodiments, the wearable device 102 is configured to adjust the driving current (e.g., the compensated current 916) of the light emitter 104 to compensate for movement-induced variations in the outputted optical signal 110 caused by those different degrees of the positional disparity.

In some embodiments, the positional disparity between the light emitter 104 and the portion of the body 120 is determined by comparing the modulated optical signal 908 to one or more predetermined criteria, e.g., using the driving component 920 or other component of the wearable device 102. In some embodiments, the one or more predetermined criteria are predefined based on different degrees of the positional disparity in accordance with levels of motion of the user, e.g., low, medium, and/or high levels of motion. For example, the low or medium levels of motion may include walking and cycling. In another example, the high level of motion may include running. In some embodiments, the wearable device 102 is configured to adjust the driving current (e.g., the compensated current 916) of the light emitter 104 to compensate for movement-induced variations in the outputted optical signal 110 caused by those different degrees of the positional disparity.

In some embodiments, the wearable device 102 is configured to capture (e.g., detect), by the photodetector (e.g., photodetector 106, photodetector 106-1, photodetector 106-2, etc.), the return optical signal 112 corresponding to the outputted optical signal 110. The return optical signal 112 is used to determine the heart information of the user 101. In some embodiments, the photodetector (e.g., photodetector 106, photodetector 106-1, photodetector 106-2, etc.) used for determining (e.g., measuring) the heart information of the user 101 is positioned exterior to the cavity 904 of the light emitter 104. In some embodiments, the photodetector 910 (e.g., in reference to Figures 9A and 9B) is distinct from the photodetector (e.g., photodetector 106, photodetector 106-1, photodetector 106-2, etc.) used for determining (e.g., measuring) the heart information of the user 101. In some embodiments, the photodetector 910 (e.g., in reference to Figures 9A and 9B) is configured as the photodetector (e.g., photodetector 106, photodetector 106-1, photodetector 106-2, etc.) used for determining (e.g., measuring) the heart information of the user 101.

In some embodiments, the light emitter 104 comprises a VCSEL. In some embodiments, the light emitter 104 comprises VCSELs arranged in an M₁×M₂ pixel array. In some embodiments, the light emitter 104 comprises an edge-emitting laser (EEL). In some embodiments, the light emitter 104 comprises EELs arranged in an M₁×M₂ pixel array.

In some embodiments, the photodetector 910 (e.g., positioned either within or exterior to the cavity 904) comprises a Si photodiode. In some embodiments, the photodetector 910 (e.g., positioned either within or exterior to the cavity 904) comprises a gallium arsenide (GaAs) photodiode. In some embodiments, the photodetector 910 comprises a plurality of photodiodes arranged in an N1×N2 pixel array. In some embodiments, a driving circuit of the photodetector 910 is part of the driving component 920. In some embodiments, the photodetector 910 and its driving circuit are part of a sensing unit (e.g., sensing unit 108 or sensing unit 202).

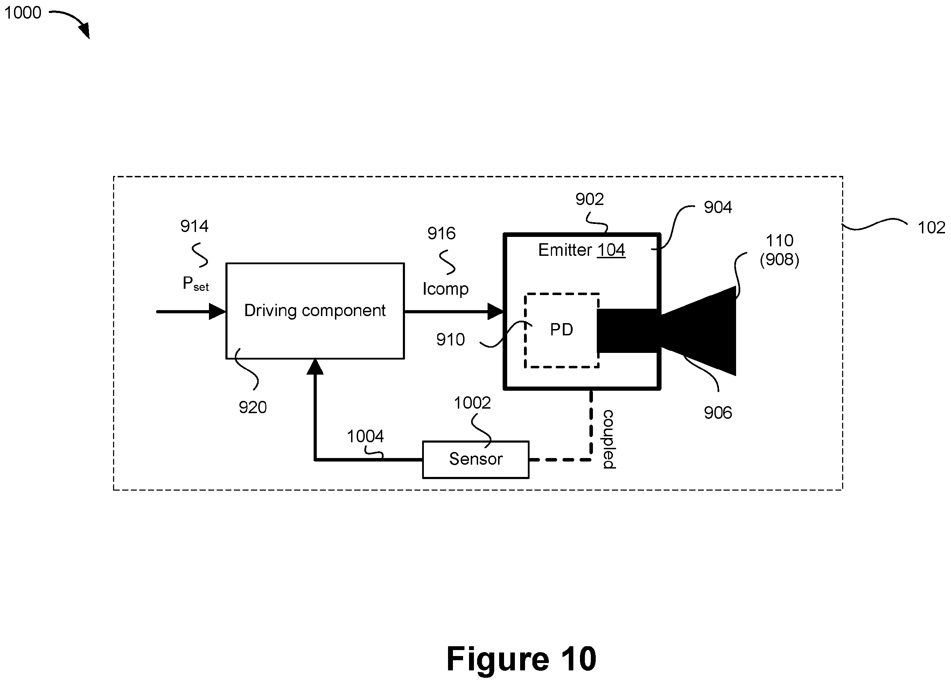

Figure 10 illustrates another example configuration 1000 for controlling the light emitter 104 of the wearable device 102, in accordance with some embodiments. In some embodiments, the configuration 1000 is a closed-loop configuration, e.g., a closed-loop compensation for eliminating the self-mixing effect. In some embodiments, the wearable device 102 comprises an extended-reality headset or a wrist-wearable device.

In some embodiments, the wearable device 102 implements the configuration 1000 (e.g., a closed-loop configuration), in which the positional disparity between the light emitter 104 and the portion of the body 120 is determined by monitoring a forward voltage of the light emitter 104. In some embodiments, the wearable device 102 comprises a sensor 1002 and a feedback loop 1004. The sensor 1002 is electrically coupled to the light emitter 104 and the feedback loop 1004, and the feedback loop 1004 is electrically coupled to the driving component 920. The sensor 1002 is configured to measure the forward voltage of the light emitter 104 and capture a corresponding change in the forward voltage induced by a motion of the user 101. For example, as discussed above, the portion of the outputted optical signal 110 that is re-coupled into the cavity 904 may induce a change in the optical power and/or the forward voltage of the light emitter 104 due to the self-mixing effect. In this circumstance, the sensor 1002 captures (e.g., monitors) the forward voltage of the light emitter 104, and the driving component 920 receives the captured forward voltage. In some embodiments, the driving component 920 is configured to determine, based on the captured forward voltage and/or the set power 914, the positional disparity between the light emitter 104 and the portion of the body 120. In some embodiments, the wearable device 102 that implements the configuration 1000 does not include the photodetector 910.

In some embodiments, the sensor 1002 is part of the feedback loop 1004. In some embodiments, the sensor 1002 is part of the light emitting component 902. For example, the sensor 1002 is integrated into the light emitting component 902. In some embodiments, the sensor 1002 comprises a shunt resistor. In some embodiments, the sensor 1002 comprises an integrated circuit, e.g., configured as a chip.

In some embodiments, the wearable device 102 comprises an amplification circuit to amplify the forward voltage of the light emitter 104 that is captured by the sensor 1002. For example, the amplification circuit may be part of the feedback loop 1004 or the driving component 920. In some embodiments, the wearable device 102 is configured to remove an DC offset of the forward voltage of the light emitter 104, e.g., at the feedback loop 1004 or the driving component 920.

In some embodiments, the wearable device 102 implements both the configuration 900B and the configuration 1000. Stated another way, the compensation is achieved by monitoring variations in both the output power and the forward voltage of the light emitter 104. In some embodiments, the light emitting component 902 having the light emitter 104, the photodetector 910, the sensor 1002, the driving component 920, or any combination thereof is arranged on a same circuit board. In some embodiments, the light emitter 104 and the photodetector 910 are positioned adjacent to one another on the same circuit board. In some embodiments, the light emitter 104 and the sensor 1002 are positioned adjacent to one another on the same circuit board. In some embodiments, the circuit board is implemented as a printed circuit board (PCB). In some embodiments, the circuit board is part of a sensing unit (e.g., sensing unit 108 or sensing unit 202). In some embodiments, the light emitting component 902 and the driving component 920 are arranged on different circuit boards.

Figure 11 illustrates a flow diagram of an example method of correcting positional disparity at a wearable device (e.g., the wearable device 102, in accordance with some embodiments. Operations (e.g., steps) of the method 1100 can be performed by one or more processors (e.g., central processing unit and/or MCU) of the wearable device 102. At least some of the operations shown in Figure 11 correspond to instructions stored in a computer memory or a non-transitory computer-readable storage medium (e.g., storage, RAM, and/or memory) of a wearable device (e.g., the wearable device 102). Operations of the method 1100 can be performed by a single device (e.g., a head-wearable device) alone or in conjunction with one or more processors and/or hardware components of another communicatively coupled device (e.g., a handheld intermediary processing device (HIPD) 842) and/or instructions stored in memory or computer-readable medium of the other device communicatively coupled to of the wearable device 102. In some embodiments, the various operations of the methods described herein are interchangeable and/or optional, and respective operations of the methods are performed by any of the aforementioned devices, systems, or combination of devices and/or systems. For convenience, the method operations will be described below as being performed by wearable device 102, but should not be construed as limiting the performance of the operation to wearable device 102 in all embodiments.

(B1) The method 1100 is performed at a wearable device (e.g., the wearable device 102) with a light emitter (e.g., light emitter 104, one or more of the plurality of light emitters 104'), a photodetector (e.g., photodetector 910), and one or more processors. The method 1100 includes projecting (1102), from the light emitter (e.g., light emitter 104, one or more of the plurality of light emitters 104'), an outputted optical signal (e.g., outputted optical signal 110) to a portion of a body 120 of a user 101. The method 1100 further includes capturing (1104), by the photodetector (e.g., photodetector 910), a modulated optical signal (e.g., modulated optical signal 908) corresponding to the outputted optical signal (e.g., outputted optical signal 110). The method 1100 further includes determining (1106), based on the modulated optical signal (e.g., modulated optical signal 908), a positional disparity between the light emitter (e.g., light emitter 104, one or more of the plurality of light emitters 104') and the portion of the body 120. The method 1100 further includes, in accordance with a determination that the positional disparity satisfies disparity correction criteria: adjusting (1108) a driving current of the light emitter (e.g., light emitter 104, one or more of the plurality of light emitters 104') to compensate variations in the outputted optical signal (e.g., outputted optical signal 110) caused by the positional disparity.

(B2) In some embodiments of B1, the light emitter (e.g., light emitter 104, one or more of the plurality of light emitters 104') is contained within a cavity (e.g., cavity 904). The modulated optical signal (e.g., modulated optical signal 908) is induced by a portion of the outputted optical signal (e.g., outputted optical signal 110) that is reflected back from the portion of the body 120 via the cavity (e.g., cavity 904). In some embodiments, the cavity is a laser cavity that functions as a fixed reference arm with respect to the portion of the body 120. In some embodiments, the modulated optical signal is induced by SMI within the cavity, wherein backscattered light re-couples into the cavity and interferes with intracavity optical fields. In some embodiments, the modulated optical signal encodes changes in optical power and frequency caused by the interference with the re-coupled portion.

(B3) In some embodiments of any of B1-B2, the photodetector (e.g., photodetector 910) is positioned within the cavity (e.g., cavity 904). In some embodiments, the photodetector (e.g., photodetector 910) is exterior to the cavity (e.g., cavity 904). In some embodiments, positioning the photodetector within the cavity enhances sensitivity and responsiveness of the photodetector for performing the compensation, as an absorption region of the photodetector is positioned in proximity to one or more optical cavity modes of the light emitter. In some embodiments, positioning the photodetector exterior to the cavity provides flexibility in designing and integrating the photodetector, as the light emitter and the photodetector are fabricated using separate manufacturing processes.

(B4) In some embodiments of any of B1-B3, the photodetector (e.g., photodetector 910) is positioned in proximity to the light emitter (e.g., light emitter 104, one or more of the plurality of light emitters 104'). In some embodiments, close proximity between the photodetector and the light emitter increases a portion of emitted light from the light emitter that is captured by the photodetector. In some embodiments, the photodetector and the light emitter are integrated into a single package or module. In some embodiments, the photodetector is positioned adjacent to the light emitter on a same substrate.

(B5) In some embodiments of any of B1-B4, a driving circuit for the light emitter (e.g., light emitter 104, one or more of the plurality of light emitters 104') is configured to operate with a feedback loop (e.g., feedback loop 918). In some embodiments, the feedback loop electrically couples an output of the photodetector to a respective input of a driving component, such that a compensation is performed to reduce or eliminate the self-mixing effect. In some embodiments, the light emitter, the feedback loop, and the driving component form a closed-loop control system. In some embodiments, a bandwidth of the closed-loop control system is set above a frequency of the self-mixing artifacts. In some embodiments, the driving circuit operates the light emitter in a constant power mode using the feedback loop.

(B6) In some embodiments of any of B1-B5, the positional disparity between the light emitter (e.g., light emitter 104, one or more of the plurality of light emitters 104') and the portion of the body 120 is determined by monitoring a forward voltage of the light emitter (e.g., light emitter 104, one or more of the plurality of light emitters 104'). In some embodiments, the wearable device (e.g., wearable device 102) comprises an amplification circuit to amplify the forward voltage of the light emitter (e.g., light emitter 104, one or more of the plurality of light emitters 104') that is captured by a sensor (e.g., sensor 1002). In some embodiments, the wearable device (e.g., wearable device 102) is configured to remove a DC offset of the forward voltage of the light emitter (e.g., light emitter 104, one or more of the plurality of light emitters 104'). In some embodiments, the sensor comprises a shunt resistor configured to measure the forward voltage. In some embodiments, the sensor comprises an integrated circuit configured as a chip. In some embodiments, the wearable device (e.g., wearable device 102) implements both forward voltage monitoring and output power monitoring via the photodetector to determine the positional disparity.

(B7) In some embodiments of any of B1-B6, the positional disparity is induced by movement of the user 101 wearing the wearable device (e.g., wearable device 102). In some embodiments, the motion of the user 101 varies in real time and include low, medium, and/or high levels of motion, which result in different degrees of the positional disparity. The wearable device (e.g., wearable device 102) is configured to adjust the driving current of the light emitter (e.g., light emitter 104, one or more of the plurality of light emitters 104') to compensate for variations in the outputted optical signal (e.g., outputted optical signal 110) caused by those different degrees of the positional disparity. In some embodiments, the low or medium levels of motion include walking and cycling. In some embodiments, the high level of motion includes running or other high-impact activities. In some embodiments, the wearable device (e.g., wearable device 102) determines the level of motion based on characteristics of the modulated optical signal, such as a frequency or amplitude of modulation shifts induced by the self-mixing effect.

(B8) In some embodiments of any of B1-B5 and B7, the positional disparity between the light emitter (e.g., light emitter 104, one or more of the plurality of light emitters 104') and the portion of the body 120 is determined by comparing the modulated optical signal (e.g., modulated optical signal 908) to one or more predetermined criteria. In some embodiments, the photodetector (e.g., photodetector 910) is distinct from the detector used for determining (e.g., measuring) the heart information of the user 101. In some embodiments, the one or more predetermined criteria are predefined based on different degrees of the positional disparity in accordance with levels of motion of the user 101, e.g., low, medium, and/or high levels of motion. For example, the low or medium levels of motion may include walking and cycling. In another example, the high level of motion may include running. The wearable device (e.g., wearable device 102) is configured to adjust the driving current of the light emitter (e.g., light emitter 104, one or more of the plurality of light emitters 104') to compensate for variations in the outputted optical signal (e.g., outputted optical signal 110) caused by those different degrees of the positional disparity. In some embodiments, the one or more predetermined criteria comprise threshold values for amplitude or frequency of the modulated optical signal. In some embodiments, the one or more predetermined criteria are calibrated during a calibration phase of the wearable device (e.g., wearable device 102). In some embodiments, the one or more predetermined criteria are dynamically updated based on historical motion data of the user 101.

(B9) In some embodiments of any of B1-B8, the method 1100 further includes detecting (1110) a return optical signal (e.g., return optical signal 112) corresponding to the outputted optical signal (e.g., outputted optical signal 110). The return optical signal (e.g., return optical signal 112) is used (1112) to determine heart information of the user 101. In some embodiments, the photodetector (e.g., photodetector 910) is distinct from the detector (e.g., photodetectors 106, one or more of the plurality of photodetectors 106') used for determining (e.g., measuring) the heart information of the user 101. In some embodiments, the return optical signal is processed in the analog domain using a signal processing channel comprising a high-pass filter, a rectifier, and a low-pass filter. In some embodiments, the return optical signal is processed in the digital domain. In some embodiments, the return optical signal is captured concurrently with the modulated optical signal, such that positional disparity correction and heart information determination are performed simultaneously.

(B10) In some embodiments of any of B1-B9, the heart information comprises heart rate information. In some embodiments, the heart information comprises heart rate information, heartbeat (e.g., arrhythmia) information, and/or other types of heart-related information (e.g., blood pressure information). In some embodiments, the heart information comprises heart rate variability information. In some embodiments, the heart information comprises a cardiac pulse waveform from which pulse morphology features are extracted. In some embodiments, the heart information comprises an indication of cardiac rhythm regularity or irregularity.

(B11) In some embodiments of any of B1-B10, the heart information comprises microvasculature information. In some embodiments the microvasculature information includes components at a beat frequency on the order of 10 kHz. In some embodiments, the microvasculature information encodes blood flow velocity in superficial capillary beds and arterioles. In some embodiments, the microvasculature information is derived from a frequency distribution of the return optical signal, wherein signal power within the frequency distribution corresponds to blood volume and frequency corresponds to blood flow velocity.

(B12) In some embodiments of any of B1-B11, the heart information is determined based on the return optical signal (e.g., return optical signal 112) detected by the photodetector (e.g., photodetector 910). In some embodiments, the photodetector (e.g., photodetector 910) serves a dual purpose of both capturing the modulated optical signal for positional disparity correction and detecting the return optical signal for heart information determination. In some embodiments, the heart information determination and the positional disparity correction share a common photodetector, reducing component count and simplifying the sensing unit.

(B13) In some embodiments of any of B1-B12, the photodetector is a first photodetector (e.g., photodetector 910). The wearable device (e.g., wearable device 102) further includes a second photodetector (e.g., photodetectors 106, one or more of the plurality of photodetectors 106') configured to detect the return optical signal (e.g., return optical signal 112). The heart information is determined based on the return optical signal (e.g., return optical signal 112) detected by the second photodetector (e.g., photodetectors 106, one or more of the plurality of photodetectors 106'). In some embodiments, the first photodetector is positioned within the cavity of the light emitter and the second photodetector is positioned exterior to the cavity. In some embodiments, the second photodetector is positioned at a distance from the light emitter selected to optimize capture of scattered light from the portion of the body 120. In some embodiments, the wearable device (e.g., wearable device 102) includes a plurality of second photodetectors arranged at different distances from the portion of the body 120 in a multi-channel configuration.

(B14) In some embodiments of any of B1-B13, the heart information is determined in accordance with principles of LDF, e.g., the return optical signal (e.g., return optical signal 112) is scattered from the portion of the body 120 of the user 101 and exhibits a small shift compared with the sampling frequency. In some embodiments, the small shift comprises a Doppler shift induced by blood flow in the portion of the body 120. In some embodiments, the return optical signal comprises a Doppler-shifted component and an unshifted component that interfere at the second photodetector to produce a beat frequency corresponding to blood flow velocity. In some embodiments, the positional disparity correction improves accuracy of the heart information determined in accordance with LDF principles by reducing self-mixing interferometry artifacts that would otherwise be misinterpreted as blood flow signals.

(B15) In some embodiments of any of B1-B14, the return optical signal (e.g., return optical signal 112) comprises a plurality of flow-induced optical frequency shifts in a range from 1 kHz to 50 kHz. In some embodiments, the frequencies are in a range from approximately 4 kHz to 25 kHz or 8 kHz to 25 kHz. In some embodiments, a corner frequency for heart rate detection is approximately 25 kHz. In some embodiments, the frequency range of the flow-induced optical frequency shifts is selected based on a depth of blood vessels targeted for measurement. In some embodiments, the plurality of flow-induced optical frequency shifts encode blood flow velocities in superficial microvasculature of the portion of the body 120.

(B16) In some embodiments of any of B1-B15, the heart information is presented to the user 101 via a graphical user interface on the wearable device (e.g., wearable device 102). In some embodiments, the graphical user interface corresponds to an application executing at the wearable device (e.g., wearable device 102). In some embodiments, the heart information is presented to the user 101 via a display that is communicatively coupled to the wearable device (e.g., wearable device 102). In some embodiments, the heart information is presented to the user 101 via a display of the wearable device (e.g., wearable device 102). In some embodiments, the heart information is presented to the user 101 via an audio output of the wearable device (e.g., wearable device 102). In some embodiments, the heart information is presented to the user 101 via a haptic output of the wearable device (e.g., wearable device 102). In some embodiments, the heart information is transmitted to a remote device or a cloud-based service for storage, further analysis, or presentation to a third party such as a healthcare provider.

(B17) In some embodiments of any of B1-B16, the wearable device (e.g., wearable device 102) comprises an extended-reality headset (e.g., a MR headset or a pair of AR glasses as two examples in reference to Figures 8A-8C). In some embodiments, the wearable device (e.g., wearable device 102) comprises a wrist-wearable device. In some embodiments, the wearable device (e.g., wearable device 102) comprises earbuds. In some embodiments, the wearable device (e.g., wearable device 102) comprises a smart ring, a smart patch, or a smart textile-based garment. In some embodiments, the positional disparity correction is performed regardless of the form factor of the wearable device (e.g., wearable device 102).

(B18) In some embodiments of any of B1-B17, the light emitter (e.g., light emitter 104, one or more of the plurality of light emitters 104') and the photodetector (e.g., photodetector 910) are assembled on a same circuit board. In some embodiments, the photodetector (e.g., photodetector 910) and the light emitter (e.g., light emitter 104, one or more of the plurality of light emitters 104') are positioned adjacent to one another on the same circuit board. In some embodiments, the light emitter (e.g., light emitter 104, one or more of the plurality of light emitters 104'), the photodetector (e.g., photodetector 910), and a driving circuit (e.g., driving component 920) are assembled on a same circuit board. In some embodiments, the circuit board is implemented as a printed circuit board (PCB). In some embodiments, the light emitter and the photodetector are arranged on different circuit boards. In some embodiments, the circuit board is part of a sensing unit (e.g., sensing unit 108 or sensing unit 202). In some embodiments, a sensor (e.g., sensor 1002) for monitoring a forward voltage of the light emitter is also assembled on the same circuit board.

(B19) In some embodiments of any of B1-B18, the light emitter (e.g., light emitter 104, one or more of the plurality of light emitters 104') comprises a VCSEL. In some embodiments, the light emitter (e.g., light emitter 104, one or more of the plurality of light emitters 104') includes VCSELs arranged in an M1×M2 pixel array. In some embodiments, the VCSEL has a threshold current in the range of 1 mA to 5 mA and a slope efficiency in the range of 0.2 mW/mA to 0.65 mW/mA. In some embodiments, the VCSEL is a commodity-grade VCSEL with wide manufacturing tolerances.

(B20) In some embodiments of any of B1-B19, the light emitter (e.g., light emitter 104, one or more of the plurality of light emitters 104') comprises an edge-emitting laser (EEL). In some embodiments, the light emitter (e.g., light emitter 104, one or more of the plurality of light emitters 104') comprises EELs arranged in an M1×M2 pixel array. In some embodiments, the light emitter comprises an SLD or other partially coherent light source. In some embodiments, the light emitter type is selected based on coherence length requirements, power consumption constraints, or form factor considerations of the wearable device (e.g., wearable device 102).

(B21) In some embodiments of any of B1-B20, the light emitter (e.g., light emitter 104, one or more of the plurality of light emitters 104') is configured to operate in a near-infrared band. In some embodiments, the light emitter (e.g., light emitter 104, one or more of the plurality of light emitters 104') is configured to emit light at a wavelength (e.g., nominal emission wavelength) of approximately 850 nm (e.g., within 10%, 5%, 2%, or 1% of 850 nm). In some embodiments, the light emitter (e.g., light emitter 104, one or more of the plurality of light emitters 104') is configured to emit light at a wavelength (e.g., nominal emission wavelength) of approximately 940 nm (e.g., within 10%, 5%, 2%, or 1% of 940 nm). In some embodiments, the light emitter is configured to emit light at a visible wavelength or a shortwave infrared (SWIR) wavelength. In some embodiments, the wavelength is selected to ensure eye safety for the user while providing sufficient tissue penetration for detecting blood flow in the portion of the body 120.

(B22) In some embodiments of any of B1-B21, the photodetector (e.g., photodetector 910) comprises a Si photodiode. In some embodiments, the photodetector (e.g., photodetector 910) comprises a GaAs photodiode. In some embodiments, the photodetector (e.g., photodetector 910) comprises a plurality of photodiodes arranged in an N1×N2 pixel array. In some embodiments, the photodetector comprises an APD or a SPAD. In some embodiments, the photodetector is selected based on responsivity at the emission wavelength of the light emitter, noise characteristics, or power consumption requirements. In some embodiments, a driving circuit of the photodetector is part of the driving component 920.

In another aspect, a computing system (e.g., comprising one or more devices) is configured to perform operations corresponding to any of the methods described herein (e.g., the methods 700, 1100, A1-A22, and B1-B22). In another aspect, a non-transitory computer-readable storage medium stores instructions that, when executed by a computing system, cause the computing system to perform operations corresponding to any of the methods described herein (e.g., the methods 700, 1100, A1-A22, and B1-B22).

The devices and systems described above are further detailed below, including wrist-wearable devices, headset devices, systems, and haptic feedback devices. Specific operations described above may occur as a result of specific hardware, such hardware is described in further detail below. The devices described below are not limiting and features on these devices can be removed or additional features can be added to these devices.

### Example Extended-Reality Systems

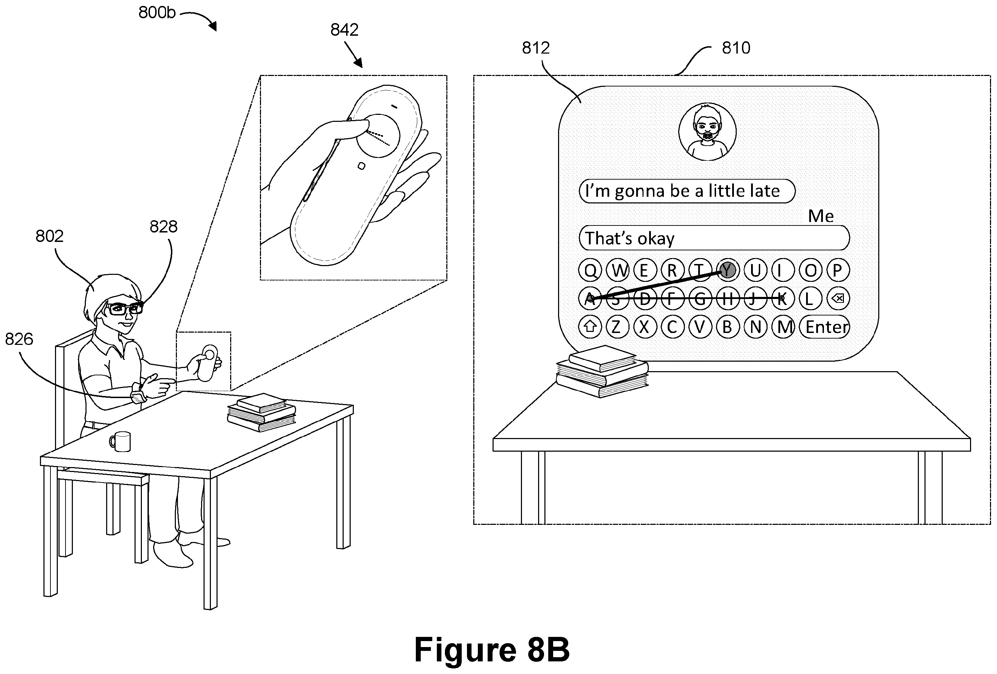

Figures 8A, 8B, 8C-1, and 8C-2, illustrate example XR systems that include AR and MR systems, in accordance with some embodiments. Figure 8A shows a first XR system 800a and first example user interactions using a wrist-wearable device 826, a head-wearable device (e.g., AR device 828), and/or a HIPD 842. Figure 8B shows a second XR system 800b and second example user interactions using a wrist-wearable device 826, AR device 828, and/or an HIPD 842. Figures 8C-1 and 8C-2 show a third MR system 800c and third example user interactions using a wrist-wearable device 826, a head-wearable device (e.g., an MR device such as a VR device), and/or an HIPD 842. As the skilled artisan will appreciate upon reading the descriptions provided herein, the above-example AR and MR systems (described in detail below) can perform various functions and/or operations.

The wrist-wearable device 826, the head-wearable devices, and/or the HIPD 842 can communicatively couple via a network 825 (e.g., cellular, near field, Wi-Fi, personal area network, wireless LAN). Additionally, the wrist-wearable device 826, the head-wearable device, and/or the HIPD 842 can also communicatively couple with one or more servers 830, computers 840 (e.g., laptops, computers), mobile devices 850 (e.g., smartphones, tablets), and/or other electronic devices via the network 825 (e.g., cellular, near field, Wi-Fi, personal area network, wireless LAN). Similarly, a smart textile-based garment, when used, can also communicatively couple with the wrist-wearable device 826, the head-wearable device(s), the HIPD 842, the one or more servers 830, the computers 840, the mobile devices 850, and/or other electronic devices via the network 825 to provide inputs.

Turning to Figure 8A, a user 802 is shown wearing the wrist-wearable device 826 and the AR device 828 and having the HIPD 842 on their desk. The wrist-wearable device 826, the AR device 828, and the HIPD 842 facilitate user interaction with an AR environment. In particular, as shown by the first XR system 800a, the wrist-wearable device 826, the AR device 828, and/or the HIPD 842 cause presentation of one or more avatars 804, digital representations of contacts 806, and virtual objects 808. As discussed below, the user 802 can interact with the one or more avatars 804, digital representations of the contacts 806, and virtual objects 808 via the wrist-wearable device 826, the AR device 828, and/or the HIPD 842. In addition, the user 802 is also able to directly view physical objects in the environment, such as a table 829 (e.g., a physical table), through transparent lens(es) and waveguide(s) of the AR device 828. Alternatively, an MR device could be used in place of the AR device 828 and a similar user experience can take place, but the user would not be directly viewing physical objects in the environment, such as the table 829, and would instead be presented with a virtual reconstruction of the table 829 produced from one or more sensors of the MR device (e.g., an outward facing camera capable of recording the surrounding environment).

The user 802 can use any of the wrist-wearable device 826, the AR device 828 (e.g., through physical inputs at the AR device and/or built-in motion tracking of a user's extremities), a smart-textile garment, externally mounted extremity tracking device, the HIPD 842 to provide user inputs, etc. For example, the user 802 can perform one or more hand gestures that are detected by the wrist-wearable device 826 (e.g., using one or more EMG sensors and/or IMUs built into the wrist-wearable device) and/or AR device 828 (e.g., using one or more image sensors (e.g., photodetectors) or cameras) to provide a user input. Alternatively, or additionally, the user 802 can provide a user input via one or more touch surfaces of the wrist-wearable device 826, the AR device 828, and/or the HIPD 842, and/or voice commands captured by a microphone of the wrist-wearable device 826, the AR device 828, and/or the HIPD 842. The wrist-wearable device 826, the AR device 828, and/or the HIPD 842 include an artificially intelligent digital assistant to help the user in providing a user input (e.g., completing a sequence of operations, suggesting different operations or commands, providing reminders, confirming a command). For example, the digital assistant can be invoked through an input occurring at the AR device 828 (e.g., via an input at a temple arm of the AR device 828). In some embodiments, the user 802 can provide a user input via one or more facial gestures and/or facial expressions. For example, cameras of the wrist-wearable device 826, the AR device 828, and/or the HIPD 842 can track the user 802's eyes for navigating a user interface.

The wrist-wearable device 826, the AR device 828, and/or the HIPD 842 can operate alone or in conjunction to allow the user 802 to interact with the AR environment. In some embodiments, the HIPD 842 is configured to operate as a central hub or control center for the wrist-wearable device 826, the AR device 828, and/or another communicatively coupled device. For example, the user 802 can provide an input to interact with the AR environment at any of the wrist-wearable device 826, the AR device 828, and/or the HIPD 842, and the HIPD 842 can identify one or more back-end and front-end tasks to cause the performance of the requested interaction and distribute instructions to cause the performance of the one or more back-end and front-end tasks at the wrist-wearable device 826, the AR device 828, and/or the HIPD 842. In some embodiments, a back-end task is a background-processing task that is not perceptible by the user (e.g., rendering content, decompression, compression, application-specific operations), and a front-end task is a user-facing task that is perceptible to the user (e.g., presenting information to the user, providing feedback to the user). The HIPD 842 can perform the back-end tasks and provide the wrist-wearable device 826 and/or the AR device 828 operational data corresponding to the performed back-end tasks such that the wrist-wearable device 826 and/or the AR device 828 can perform the front-end tasks. In this way, the HIPD 842, which has more computational resources and greater thermal headroom than the wrist-wearable device 826 and/or the AR device 828, performs computationally intensive tasks and reduces the computer resource utilization and/or power usage of the wrist-wearable device 826 and/or the AR device 828.

In the example shown by the first XR system 800a, the HIPD 842 identifies one or more back-end tasks and front-end tasks associated with a user request to initiate an AR video call with one or more other users (represented by the avatar 804 and the digital representation of the contact 806) and distributes instructions to cause the performance of the one or more back-end tasks and front-end tasks. In particular, the HIPD 842 performs back-end tasks for processing and/or rendering image data (and other data) associated with the AR video call and provides operational data associated with the performed back-end tasks to the AR device 828 such that the AR device 828 performs front-end tasks for presenting the AR video call (e.g., presenting the avatar 804 and the digital representation of the contact 806).

In some embodiments, the HIPD 842 can operate as a focal or anchor point for causing the presentation of information. This allows the user 802 to be generally aware of where information is presented. For example, as shown in the first XR system 800a, the avatar 804 and the digital representation of the contact 806 are presented above the HIPD 842. In particular, the HIPD 842 and the AR device 828 operate in conjunction to determine a location for presenting the avatar 804 and the digital representation of the contact 806. In some embodiments, information can be presented within a predetermined distance from the HIPD 842 (e.g., within five meters). For example, as shown in the first XR system 800a, virtual object 808 is presented on the desk some distance from the HIPD 842. Similar to the above example, the HIPD 842 and the AR device 828 can operate in conjunction to determine a location for presenting the virtual object 808. Alternatively, in some embodiments, presentation of information is not bound by the HIPD 842. More specifically, the avatar 804, the digital representation of the contact 806, and the virtual object 808 do not have to be presented within a predetermined distance of the HIPD 842. While an AR device 828 is described working with an HIPD, an MR headset can be interacted with in the same way as the AR device 828.

User inputs provided at the wrist-wearable device 826, the AR device 828, and/or the HIPD 842 are coordinated such that the user can use any device to initiate, continue, and/or complete an operation. For example, the user 802 can provide a user input to the AR device 828 to cause the AR device 828 to present the virtual object 808 and, while the virtual object 808 is presented by the AR device 828, the user 802 can provide one or more hand gestures via the wrist-wearable device 826 to interact and/or manipulate the virtual object 808. While an AR device 828 is described working with a wrist-wearable device 826, an MR headset can be interacted with in the same way as the AR device 828.

### Integration of Artificial Intelligence with XR Systems

Figure 8A illustrates an interaction in which an artificially intelligent virtual assistant can assist in requests made by a user 802. The Al virtual assistant can be used to complete open-ended requests made through natural language inputs by a user 802. For example, in Figure 8A the user 802 makes an audible request 844 to summarize the conversation and then share the summarized conversation with others in the meeting. In addition, the Al virtual assistant is configured to use sensors of the XR system (e.g., cameras of an XR headset, microphones, and various other sensors of any of the devices in the system) to provide contextual prompts to the user for initiating tasks.

Figure 8A also illustrates an example neural network 852 used in Artificial Intelligence applications. Uses of Artificial Intelligence (AI) are varied and encompass many different aspects of the devices and systems described herein. AI capabilities cover a diverse range of applications and deepen interactions between the user 802 and user devices (e.g., the AR device 828, an MR device 832, the HIPD 842, the wrist-wearable device 826). The Al discussed herein can be derived using many different training techniques. While the primary Al model example discussed herein is a neural network, other AI models can be used. Non-limiting examples of Al models include artificial neural networks (ANNs), deep neural networks (DNNs), convolution neural networks (CNNs), recurrent neural networks (RNNs), large language models (LLMs), long short-term memory networks, transformer models, decision trees, random forests, support vector machines, k-nearest neighbors, genetic algorithms, Markov models, Bayesian networks, fuzzy logic systems, and deep reinforcement learnings, etc. The AI models can be implemented at one or more of the user devices, and/or any other devices described herein. For devices and systems herein that employ multiple Al models, different models can be used depending on the task. For example, for a natural-language artificially intelligent virtual assistant, an LLM can be used and for the object detection of a physical environment, a DNN can be used instead.

In another example, an Al virtual assistant can include many different Al models and based on the user's request, multiple Al models may be employed (concurrently, sequentially or a combination thereof). For example, an LLM-based Al model can provide instructions for helping a user follow a recipe and the instructions can be based in part on another Al model that is derived from an ANN, a DNN, an RNN, etc. that is capable of discerning what part of the recipe the user is on (e.g., object and scene detection).

As Al training models evolve, the operations and experiences described herein could potentially be performed with different models other than those listed above, and a person skilled in the art would understand that the list above is non-limiting.

A user 802 can interact with an Al model through natural language inputs captured by a voice sensor, text inputs, or any other input modality that accepts natural language and/or a corresponding voice sensor module. In another instance, input is provided by tracking the eye gaze of a user 802 via a gaze tracker module. Additionally, the Al model can also receive inputs beyond those supplied by a user 802. For example, the Al can generate its response further based on environmental inputs (e.g., temperature data, image data, video data, ambient light data, audio data, GPS location data, inertial measurement (i.e., user motion) data, pattern recognition data, magnetometer data, depth data, pressure data, force data, neuromuscular data, heart rate data, temperature data, sleep data) captured in response to a user request by various types of sensors and/or their corresponding sensor modules. The sensors' data can be retrieved entirely from a single device (e.g., AR device 828) or from multiple devices that are in communication with each other (e.g., a system that includes at least two of an AR device 828, an MR device 832, the HIPD 842, the wrist-wearable device 826, etc.). The Al model can also access additional information (e.g., one or more servers 830, the computers 840, the mobile devices 850, and/or other electronic devices) via a network 825.

A non-limiting list of Al-enhanced functions includes but is not limited to image recognition, speech recognition (e.g., automatic speech recognition), text recognition (e.g., scene text recognition), pattern recognition, natural language processing and understanding, classification, regression, clustering, anomaly detection, sequence generation, content generation, and optimization. In some embodiments, Al-enhanced functions are fully or partially executed on cloud-computing platforms communicatively coupled to the user devices (e.g., the AR device 828, an MR device 832, the HIPD 842, the wrist-wearable device 826) via the one or more networks. The cloud-computing platforms provide scalable computing resources, distributed computing, managed Al services, interference acceleration, pre-trained models, APIs and/or other resources to support comprehensive computations required by the Al-enhanced function.

Example outputs stemming from the use of an Al model can include natural language responses, mathematical calculations, charts displaying information, audio, images, videos, texts, summaries of meetings, predictive operations based on environmental factors, classifications, pattern recognitions, recommendations, assessments, or other operations. In some embodiments, the generated outputs are stored on local memories of the user devices (e.g., the AR device 828, an MR device 832, the HIPD 842, the wrist-wearable device 826), storage options of the external devices (servers, computers, mobile devices, etc.), and/or storage options of the cloud-computing platforms.

The Al-based outputs can be presented across different modalities (e.g., audio-based, visual-based, haptic-based, and any combination thereof) and across different devices of the XR system described herein. Some visual-based outputs can include the displaying of information on XR augments of an XR headset, user interfaces displayed at a wrist-wearable device, laptop device, mobile device, etc. On devices with or without displays (e.g., HIPD 842), haptic feedback can provide information to the user 802. An Al model can also use the inputs described above to determine the appropriate modality and device(s) to present content to the user (e.g., a user walking on a busy road can be presented with an audio output instead of a visual output to avoid distracting the user 802).

### Example Augmented Reality Interaction

Figure 8B shows the user 802 wearing the wrist-wearable device 826 and the AR device 828 and holding the HIPD 842. In the second XR system 800b, the wrist-wearable device 826, the AR device 828, and/or the HIPD 842 are used to receive and/or provide one or more messages to a contact of the user 802. In particular, the wrist-wearable device 826, the AR device 828, and/or the HIPD 842 detect and coordinate one or more user inputs to initiate a messaging application and prepare a response to a received message via the messaging application.

In some embodiments, the user 802 initiates, via a user input, an application on the wrist-wearable device 826, the AR device 828, and/or the HIPD 842 that causes the application to initiate on at least one device. For example, in the second XR system 800b the user 802 performs a hand gesture associated with a command for initiating a messaging application (represented by messaging user interface 812); the wrist-wearable device 826 detects the hand gesture; and, based on a determination that the user 802 is wearing the AR device 828, causes the AR device 828 to present a messaging user interface 812 of the messaging application. The AR device 828 can present the messaging user interface 812 to the user 802 via its display (e.g., as shown by user 802's field of view 810). In some embodiments, the application is initiated and can be run on the device (e.g., the wrist-wearable device 826, the AR device 828, and/or the HIPD 842) that detects the user input to initiate the application, and the device provides another device operational data to cause the presentation of the messaging application. For example, the wrist-wearable device 826 can detect the user input to initiate a messaging application, initiate and run the messaging application, and provide operational data to the AR device 828 and/or the HIPD 842 to cause presentation of the messaging application. Alternatively, the application can be initiated and run at a device other than the device that detected the user input. For example, the wrist-wearable device 826 can detect the hand gesture associated with initiating the messaging application and cause the HIPD 842 to run the messaging application and coordinate the presentation of the messaging application.

Further, the user 802 can provide a user input provided at the wrist-wearable device 826, the AR device 828, and/or the HIPD 842 to continue and/or complete an operation initiated at another device. For example, after initiating the messaging application via the wrist-wearable device 826 and while the AR device 828 presents the messaging user interface 812, the user 802 can provide an input at the HIPD 842 to prepare a response (e.g., shown by the swipe gesture performed on the HIPD 842). The user 802's gestures performed on the HIPD 842 can be provided and/or displayed on another device. For example, the user 802's swipe gestures performed on the HIPD 842 are displayed on a virtual keyboard of the messaging user interface 812 displayed by the AR device 828.

In some embodiments, the wrist-wearable device 826, the AR device 828, the HIPD 842, and/or other communicatively coupled devices can present one or more notifications to the user 802. The notification can be an indication of a new message, an incoming call, an application update, a status update, etc. The user 802 can select the notification via the wrist-wearable device 826, the AR device 828, or the HIPD 842 and cause presentation of an application or operation associated with the notification on at least one device. For example, the user 802 can receive a notification that a message was received at the wrist-wearable device 826, the AR device 828, the HIPD 842, and/or other communicatively coupled device and provide a user input at the wrist-wearable device 826, the AR device 828, and/or the HIPD 842 to review the notification, and the device detecting the user input can cause an application associated with the notification to be initiated and/or presented at the wrist-wearable device 826, the AR device 828, and/or the HIPD 842.

While the above example describes coordinated inputs used to interact with a messaging application, the skilled artisan will appreciate upon reading the descriptions that user inputs can be coordinated to interact with any number of applications including, but not limited to, gaming applications, social media applications, camera applications, web-based applications, financial applications, etc. For example, the AR device 828 can present to the user 802 game application data and the HIPD 842 can use a controller to provide inputs to the game. Similarly, the user 802 can use the wrist-wearable device 826 to initiate a camera of the AR device 828, and the user can use the wrist-wearable device 826, the AR device 828, and/or the HIPD 842 to manipulate the image capture (e.g., zoom in or out, apply filters) and capture image data.

While an AR device 828 is shown being capable of certain functions, it is understood that an AR device can be an AR device with varying functionalities based on costs and market demands. For example, an AR device may include a single output modality such as an audio output modality. In another example, the AR device may include a low-fidelity display as one of the output modalities, where simple information (e.g., text and/or low-fidelity images/video) is capable of being presented to the user. In yet another example, the AR device can be configured with face-facing light emitting diodes (LEDs) configured to provide a user with information, e.g., an LED around the right-side lens can illuminate to notify the wearer to turn right while directions are being provided or an LED on the left-side can illuminate to notify the wearer to turn left while directions are being provided. In another embodiment, the AR device can include an outward-facing projector such that information (e.g., text information, media) may be displayed on the palm of a user's hand or other suitable surface (e.g., a table, whiteboard). In yet another embodiment, information may also be provided by locally dimming portions of a lens to emphasize portions of the environment in which the user's attention should be directed. Some AR devices can present AR augments either monocularly or binocularly (e.g., an AR augment can be presented at only a single display associated with a single lens as opposed presenting an AR augmented at both lenses to produce a binocular image). In some instances an AR device capable of presenting AR augments binocularly can optionally display AR augments monocularly as well (e.g., for power-saving purposes or other presentation considerations). These examples are non-exhaustive and features of one AR device described above can be combined with features of another AR device described above. While features and experiences of an AR device have been described generally in the preceding sections, it is understood that the described functionalities and experiences can be applied in a similar manner to an MR headset, which is described below in the proceeding sections.

### Example Mixed Reality Interaction

Turning to Figures 8C-1 and 8C-2, the user 802 is shown wearing the wrist-wearable device 826 and an MR device 832 (e.g., a device capable of providing either an entirely VR experience or an MR experience that displays object(s) from a physical environment at a display of the device) and holding the HIPD 842. In the third MR system 800c, the wrist-wearable device 826, the MR device 832, and/or the HIPD 842 are used to interact within an MR environment, such as a VR game or other MR/VR application. While the MR device 832 presents a representation of a VR game (e.g., first MR game environment 820) to the user 802, the wrist-wearable device 826, the MR device 832, and/or the HIPD 842 detect and coordinate one or more user inputs to allow the user 802 to interact with the VR game.

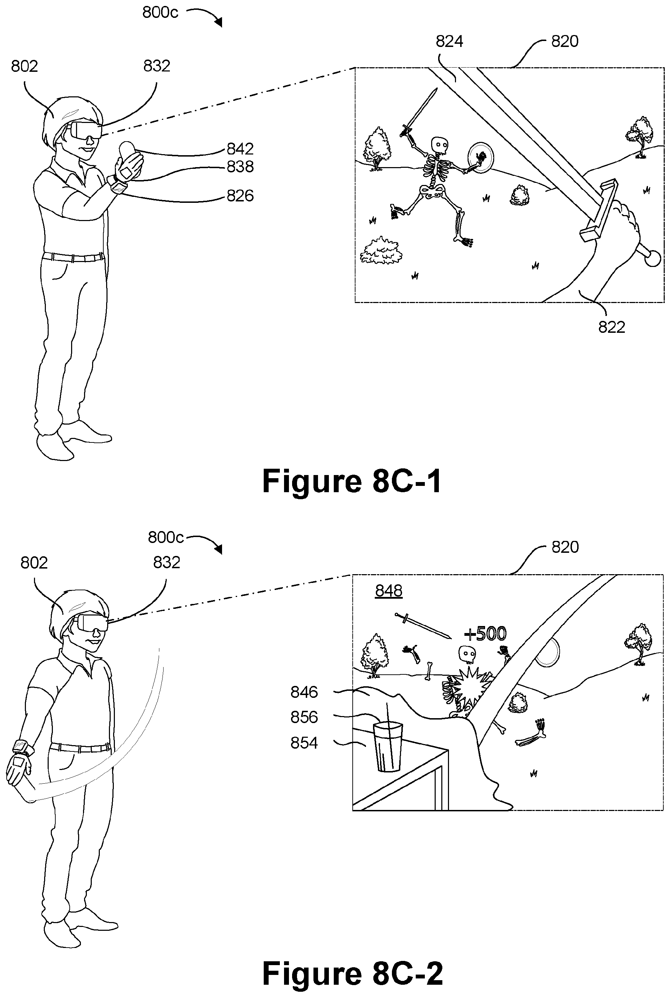

In some embodiments, the user 802 can provide a user input via the wrist-wearable device 826, the MR device 832, and/or the HIPD 842 that causes an action in a corresponding MR environment. For example, the user 802 in the third MR system 800c (shown in Figure 8C-1) raises the HIPD 842 to prepare for a swing in the first MR game environment 820. The MR device 832, responsive to the user 802 raising the HIPD 842, causes the MR representation of the user 822 to perform a similar action (e.g., raise a virtual object, such as a virtual sword 824). In some embodiments, each device uses respective sensor data and/or image data to detect the user input and provide an accurate representation of the user 802's motion. For example, image sensors (e.g., SLAM cameras or other cameras) of the HIPD 842 can be used to detect a position of the HIPD 842 relative to the user 802's body such that the virtual object can be positioned appropriately within the first MR game environment 820; sensor data from the wrist-wearable device 826 can be used to detect a velocity at which the user 802 raises the HIPD 842 such that the MR representation of the user 822 and the virtual sword 824 are synchronized with the user 802's movements; and image sensors of the MR device 832 can be used to represent the user 802's body, boundary conditions, or real-world objects within the first MR game environment 820.

In Figure 8C-2, the user 802 performs a downward swing while holding the HIPD 842. The user 802's downward swing is detected by the wrist-wearable device 826, the MR device 832, and/or the HIPD 842 and a corresponding action is performed in the first MR game environment 820. In some embodiments, the data captured by each device is used to improve the user's experience within the MR environment. For example, sensor data of the wrist-wearable device 826 can be used to determine a speed and/or force at which the downward swing is performed and image sensors of the HIPD 842 and/or the MR device 832 can be used to determine a location of the swing and how it should be represented in the first MR game environment 820, which, in turn, can be used as inputs for the MR environment (e.g., game mechanics, which can use detected speed, force, locations, and/or aspects of the user 802's actions to classify a user's inputs (e.g., user performs a light strike, hard strike, critical strike, glancing strike, miss) or calculate an output (e.g., amount of damage)).

Figure 8C-2 further illustrates that a portion of the physical environment is reconstructed and displayed at a display of the MR device 832 while the MR game environment 820 is being displayed. In this instance, a reconstruction of the physical environment 846 is displayed in place of a portion of the MR game environment 820 when object(s) in the physical environment are potentially in the path of the user (e.g., a collision with the user and an object in the physical environment are likely). Thus, this example MR game environment 820 includes (i) an immersive VR portion 848 (e.g., an environment that does not have a corollary counterpart in a nearby physical environment) and (ii) a reconstruction of the physical environment 846 (e.g., table 854 and cup 856). While the example shown here is an MR environment that shows a reconstruction of the physical environment to avoid collisions, other uses of reconstructions of the physical environment can be used, such as defining features of the virtual environment based on the surrounding physical environment (e.g., a virtual column can be placed based on an object in the surrounding physical environment (e.g., a tree)).

While the wrist-wearable device 826, the MR device 832, and/or the HIPD 842 are described as detecting user inputs, in some embodiments, user inputs are detected at a single device (with the single device being responsible for distributing signals to the other devices for performing the user input). For example, the HIPD 842 can operate an application for generating the first MR game environment 820 and provide the MR device 832 with corresponding data for causing the presentation of the first MR game environment 820, as well as detect the user 802's movements (while holding the HIPD 842) to cause the performance of corresponding actions within the first MR game environment 820. Additionally or alternatively, in some embodiments, operational data (e.g., sensor data, image data, application data, device data, and/or other data) of one or more devices is provided to a single device (e.g., the HIPD 842) to process the operational data and cause respective devices to perform an action associated with processed operational data.

In some embodiments, the user 802 can wear a wrist-wearable device 826, wear an MR device 832, wear smart textile-based garments 838 (e.g., wearable haptic gloves), and/or hold an HIPD 842 device. In this embodiment, the wrist-wearable device 826, the MR device 832, and/or the smart textile-based garments 838 are used to interact within an MR environment (e.g., any AR or MR system described above in reference to Figures 8A-8B). While the MR device 832 presents a representation of an MR game (e.g., second MR game environment 820) to the user 802, the wrist-wearable device 826, the MR device 832, and/or the smart textile-based garments 838 detect and coordinate one or more user inputs to allow the user 802 to interact with the MR environment.

In some embodiments, the user 802 can provide a user input via the wrist-wearable device 826, an HIPD 842, the MR device 832, and/or the smart textile-based garments 838 that causes an action in a corresponding MR environment. In some embodiments, each device uses respective sensor data and/or image data to detect the user input and provide an accurate representation of the user 802's motion. While four different input devices are shown (e.g., a wrist-wearable device 826, an MR device 832, an HIPD 842, and a smart textile-based garment 838) each one of these input devices entirely on its own can provide inputs for fully interacting with the MR environment. For example, the wrist-wearable device can provide sufficient inputs on its own for interacting with the MR environment. In some embodiments, if multiple input devices are used (e.g., a wrist-wearable device and the smart textile-based garment 838) sensor fusion can be utilized to ensure inputs are correct. While multiple input devices are described, it is understood that other input devices can be used in conjunction or on their own instead, such as but not limited to external motion-tracking cameras, other wearable devices fitted to different parts of a user, apparatuses that allow for a user to experience walking in an MR environment while remaining substantially stationary in the physical environment, etc.

As described above, the data captured by each device is used to improve the user's experience within the MR environment. Although not shown, the smart textile-based garments 838 can be used in conjunction with an MR device and/or an HIPD 842.

While some experiences are described as occurring on an AR device and other experiences are described as occurring on an MR device, one skilled in the art would appreciate that experiences can be ported over from an MR device to an AR device, and vice versa.

Some definitions of devices and components that can be included in some or all of the example devices discussed are defined here for ease of reference. A skilled artisan will appreciate that certain types of components described may be more suitable for a particular set of devices, and less suitable for a different set of devices. But subsequent reference to the components defined here should be considered to be encompassed by the definitions provided.

In accordance with some embodiments, example devices and systems, including electronic devices and systems are discussed. Such example devices and systems are not intended to be limiting, and one of skill in the art will understand that alternative devices and systems to the example devices and systems described herein may be used to perform the operations and construct the systems and devices that are described herein.

As described herein, an electronic device is a device that uses electrical energy to perform a specific function. It can be any physical object that contains electronic components such as transistors, resistors, capacitors, diodes, and integrated circuits. Examples of electronic devices include smartphones, laptops, digital cameras, televisions, gaming consoles, and music players, as well as the example electronic devices discussed herein. As described herein, an intermediary electronic device is a device that sits between two other electronic devices, and/or a subset of components of one or more electronic devices and facilitates communication, and/or data processing and/or data transfer between the respective electronic devices and/or electronic components.

The foregoing descriptions of Figures 8A-8C-2 provided above are intended to augment the description provided in reference to Figures 1A-7 and 9A-11. While terms in the following description may not be identical to terms used in the foregoing description, a person having ordinary skill in the art would understand these terms to have the same meaning.

Any data collection performed by the devices described herein and/or any devices configured to perform or cause the performance of the different embodiments described above in reference to any of the Figures, hereinafter the "devices," is done with user consent and in a manner that is consistent with all applicable privacy laws. Users are given options to allow the devices to collect data, as well as the option to limit or deny collection of data by the devices. A user is able to opt in or opt out of any data collection at any time. Further, users are given the option to request the removal of any collected data.

It will be understood that, although the terms "first," "second," etc. may be used herein to describe various elements, these elements should not be limited by these terms. These terms are only used to distinguish one element from another.

The terminology used herein is for the purpose of describing particular embodiments only and is not intended to be limiting of the claims. As used in the description of the embodiments and the appended claims, the singular forms "a," "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if" can be construed to mean "when" or "upon" or "in response to determining" or "in accordance with a determination" or "in response to detecting," that a stated condition precedent is true, depending on the context. Similarly, the phrase "if it is determined [that a stated condition precedent is true]" or "if [a stated condition precedent is true]" or "when [a stated condition precedent is true]" can be construed to mean "upon determining" or "in response to determining" or "in accordance with a determination" or "upon detecting" or "in response to detecting" that the stated condition precedent is true, depending on the context.

The foregoing description, for purpose of explanation, has been described with reference to specific embodiments. However, the illustrative discussions above are not intended to be exhaustive or to limit the claims to the precise forms disclosed. Many modifications and variations are possible in view of the above teachings, the invention being defined in the appended claims. The embodiments were chosen and described in order to best explain principles of operation and practical applications, to thereby enable others skilled in the art.

## Claims

1. A method comprising:
at a wearable device comprising a light emitter, a photodetector, and processing circuitry:
projecting, from the light emitter, an outputted optical signal to a portion of a body of a user, wherein the light emitter operates in a pulsed mode comprising a plurality of laser pulses separated by off periods;
capturing, by the photodetector, a return optical signal corresponding to the outputted optical signal;
sampling, by an analog-to-digital converter (ADC), the return optical signal at an ADC sampling rate to generate a plurality of ADC samples;
processing a block of the plurality of ADC samples to generate a processed sample, wherein processing the block of ADC samples comprises filtering the block of ADC samples to isolate a frequency band of interest and performing energy quantification on the filtered ADC samples;
generating an output sample based on one or more processed samples, wherein the output sample is generated at an output sample rate corresponding to a physiological signal of interest; and
determining, based on the output sample, heart information of the user.

2. The method of claim 1, wherein each laser pulse of the plurality of laser pulses comprises multiple ADC samples captured during the laser pulse.

3. The method of claim 1 or claim 2, wherein each laser pulse of the plurality of laser pulses corresponds to a single ADC sample.

4. The method of any preceding claim, wherein each ADC sample corresponds to multiple laser pulses of the plurality of laser pulses.

5. The method of any preceding claim, wherein a duty cycle of the pulsed mode is controlled to adjust an average optical power of the outputted optical signal;
optionally, wherein the duty cycle is variable within a range from 1.25% to 80%;
optionally, wherein the duty cycle has a minimum value of 10%.

6. The method of any preceding claim, and one or more of the following:
(i) wherein a pulse duration of each laser pulse is on an order of 1 millisecond;
(ii) ... wherein the ADC sampling rate is 20 kHz or higher;
(iii) ...wherein the output sample rate is between 10 Hz and 100 Hz for capturing heartbeat information;
(iv) wherein performing energy quantification comprises computing an average rectified value (ARV) of the filtered ADC samples.

7. A wearable device comprising:
a light emitter configured to project an outputted optical signal to a portion of a body of a user, wherein the light emitter is configured to operate in a pulsed mode comprising a plurality of laser pulses separated by off periods;
a photodetector configured to capture a return optical signal corresponding to the outputted optical signal;
an analog-to-digital converter (ADC) configured to sample the return optical signal to generate ADC samples; and
processing circuitry configured to:
process a block of the ADC samples to generate a processed sample,
wherein processing the block of ADC samples comprises filtering the block of ADC samples to isolate a frequency band of interest and performing energy quantification on the filtered ADC samples;
generate an output sample based on one or more processed samples,
wherein the output sample is generated at an output sample rate corresponding to a physiological signal of interest; and
determine, based on the output sample, heart information of the user.

8. The wearable device of claim 7, wherein each laser pulse of the plurality of laser pulses comprises multiple ADC samples captured during the laser pulse.

9. The wearable device of claim 7 or claim 8 or, wherein each laser pulse of the plurality of laser pulses corresponds to a single ADC sample.

10. The wearable device of any one of claims 7 to 9, wherein each ADC sample corresponds to multiple laser pulses of the plurality of laser pulses.

11. The wearable device of any one of claims 7 to 10, wherein a duty cycle of the pulsed mode is controlled to adjust an average optical power of the outputted optical signal.

12. A non-transitory computer-readable storage medium storing instructions that, when executed by one or more processors of a wearable device comprising a light emitter and a photodetector, cause the wearable device to perform operations comprising:
projecting, from the light emitter, an outputted optical signal to a portion of a body of a user, wherein the light emitter operates in a pulsed mode comprising a plurality of laser pulses separated by off periods;
capturing, by the photodetector, a return optical signal corresponding to the outputted optical signal;
sampling the return optical signal at an analog-to-digital (ADC) sampling rate to generate a plurality of ADC samples;
processing a block of the plurality of ADC samples to generate a processed sample, wherein processing the block of ADC samples comprises filtering the block of ADC samples to isolate a frequency band of interest and performing energy quantification on the filtered ADC samples;
generating an output sample based on one or more processed samples, wherein the output sample is generated at an output sample rate corresponding to a physiological signal of interest; and
determining, based on the output sample, heart information of the user.

13. The non-transitory computer-readable storage medium of claim 12, wherein each laser pulse of the plurality of laser pulses comprises multiple ADC samples captured during the laser pulse.

14. The non-transitory computer-readable storage medium of claim 12 or claim 13, wherein each laser pulse of the plurality of laser pulses corresponds to a single ADC sample.

15. The non-transitory computer-readable storage medium of any one of claims 12 to 14, wherein each ADC sample corresponds to multiple laser pulses of the plurality of laser pulses.
